(19) Europäisches Patentamt
European Patent Office
Office européen des brevets

(11) EP 4 549 430 A1

(12) **EUROPEAN PATENT APPLICATION**
published in accordance with Art. 153(4) EPC

(43) Date of publication:
**07.05.2025 Bulletin 2025/19**

(21) Application number: **23831579.0**

(22) Date of filing: **29.06.2023**

(51) International Patent Classification (IPC):
***C07D 209/80*** (2006.01) ***C07D 209/86*** (2006.01)
***C07D 405/14*** (2006.01) ***C07D 409/14*** (2006.01)
***C08F 20/36*** (2006.01) ***G02B 1/04*** (2006.01)
***G02B 1/11*** (2015.01) ***G02B 5/32*** (2006.01)
***G03H 1/02*** (2006.01) ***G11B 7/0065*** (2006.01)

(52) Cooperative Patent Classification (CPC):
**C07D 209/80; C07D 209/86; C07D 405/14; C07D 409/14; C08F 20/36; G02B 1/04; G02B 1/11; G02B 5/32; G03H 1/02; G11B 7/0065**

(86) International application number:
**PCT/JP2023/024169**

(87) International publication number:
**WO 2024/005140 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.06.2022 JP 2022106252**

(71) Applicant: **Mitsubishi Chemical Corporation**
**Tokyo 100-8251 (JP)**

(72) Inventors:
- **YAMASHITA, Shuji**
**Tokyo 100-8251 (JP)**
- **YABE, Akiko**
**Tokyo 100-8251 (JP)**
- **INOUE, Kazuma**
**Tokyo 100-8251 (JP)**
- **SATO, Ken**
**Tokyo 100-8251 (JP)**
- **Otsu, Yuuta**
**Tokyo 100-8251 (JP)**
- **ENDA, Jun**
**Tokyo 100-8251 (JP)**

(74) Representative: **Hoffmann Eitle**
**Patent- und Rechtsanwälte PartmbB**
**Arabellastraße 30**
**81925 München (DE)**

(54) **COMPOUND, POLYMERIZABLE COMPOSITION, POLYMER, HOLOGRAPHIC RECORDING MEDIUM, OPTICAL MATERIAL AND OPTICAL COMPONENT**

(57) A compound represented by the following formula (1):

EP 4 549 430 A1

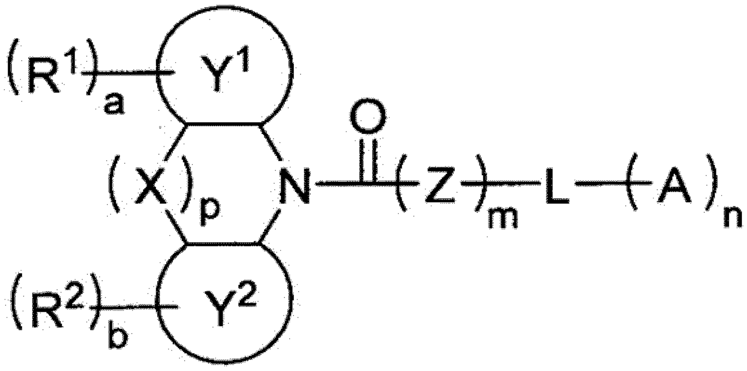

(1)

[In the formula, A represents a polymerizable group. n represents an integer of 1 to 3. L represents an optionally branched (n+1)-valent linking group. Z represents an oxygen atom, or a nitrogen atom optionally having a substituent. m represents an integer of 0 or 1. X represents a single bond, an oxygen atom, a sulfur atom, a carbon atom optionally having a substituent, or a nitrogen atom optionally having a substituent. p represents an integer of 0 to 2. When p is 2, the two Xs may be the same or different. $Y^1$ and $Y^2$ are each independently a benzene ring, a naphthalene ring, or a phenanthrene ring. When $Y^1$ or $Y^2$ is a benzene ring, a or b corresponding to $Y^1$ or $Y^2$ is an integer of 0 to 4. When $Y^1$ or $Y^2$ is a naphthalene ring, a or b corresponding to $Y^1$ or $Y^2$ is an integer of 0 to 6. When $Y^1$ or $Y^2$ is a phenanthrene ring, a or b corresponding to $Y^1$ or $Y^2$ is an integer from 0 to 8. $R^1$ and $R^2$ each independently represent an aromatic ring group optionally having a substituent. When a and b are 2 or more, a plurality of $R^1{}_S$ and $R^2$s may be the same or different from each other. In the formula, $Y^1$ and $Y^2$ are not both benzene rings and a and b are not both 0.]



(Cont. next page)

Fig.1

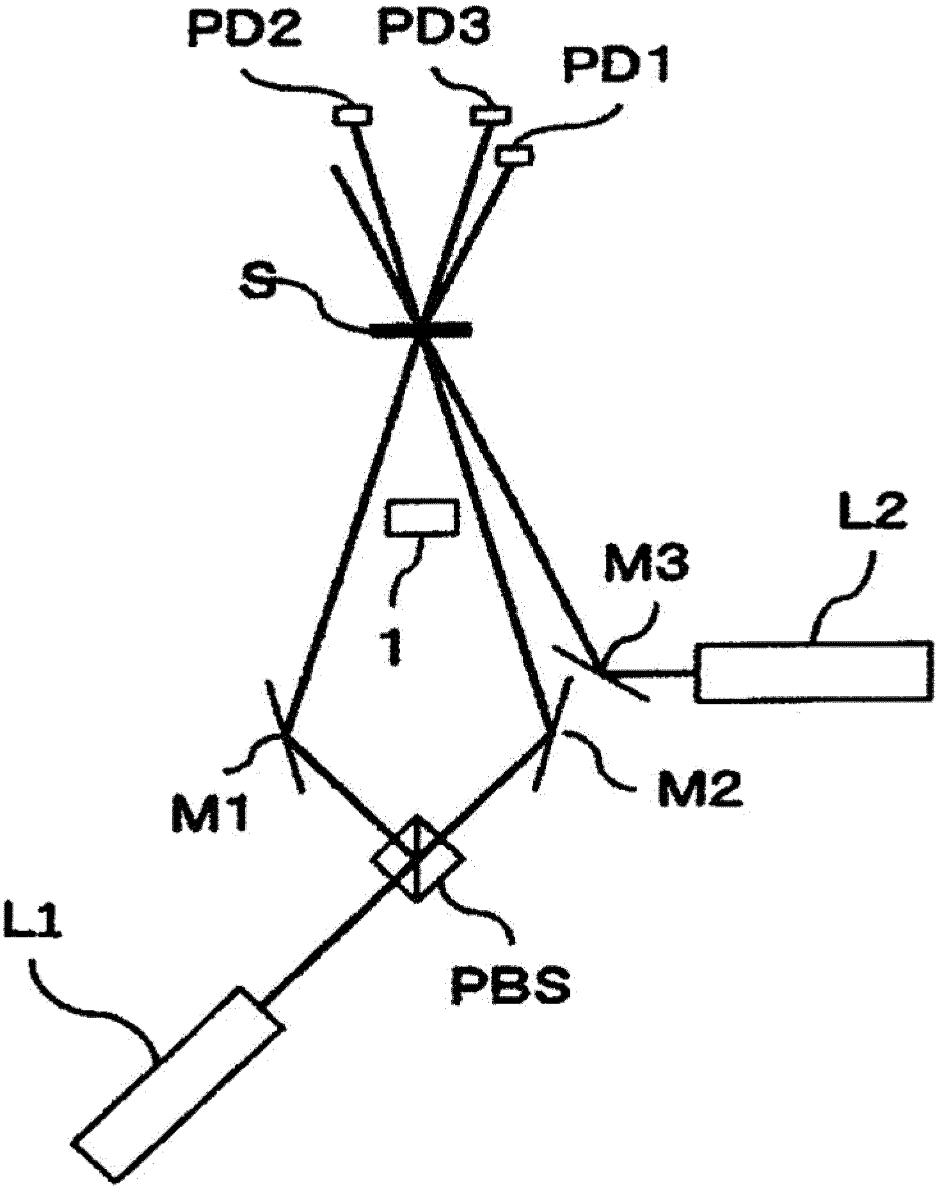

## Description

### Technical Field

The present invention relates to a compound having a high refractive index, high transparency and excellent chemical stability. The present invention also relates to a holographic recording medium, an optical material, and an optical component that use the polymerizable composition comprising the compound or a polymer thereof.

### Background Art

Glass has often been used for optical components. For example, as for optical lenses with the same focal length, a lens produced using a high-refractive index material can have a smaller thickness, and this is advantageous in that the weight of the lens can be reduced and that the design flexibility of an optical path increases. High-refractive index optical lenses are also effective in reducing size of optical imaging devices and increasing their resolution and angle of view.

In recent years, highly transparent plastics are receiving attention as optical materials alternative to glass. Plastic materials have advantages over glass in that they can be easily reduced in weight, that their mechanical strength can be easily improved, and that they can be easily shaped. With the development of peripheral technologies, there is an increasing demand for plastic optical materials with improved performance. For example, materials for optical lens applications are required to be easily polymerized (have easy polymerizability), have good curability, and chemical stability, especially thermal stability, and form polymerized products having a high refractive index.

Various resins have been developed for the purpose of increasing the refractive index.

For example, 9,9-bis[4-(2-acryloyloxyethoxy)phenyl]fluorene is frequently used as a high-refractive index acrylate. However, the refractive index of this acrylate is about 1.62, which is not sufficiently high (PTL 1).

One way to increase the refractive index is to introduce an aromatic ring, and introducing a sulfur atom into the molecule is also effective. For example, PTL 2 describes a diacrylate monomer having a pentaerythritol skeleton and including 1 to 2 naphthylthio groups per molecule. In this case, the refractive index is 1.62 to 1.65.

PTL 3 describes an acrylate compound having a glycerin skeleton and including two benzothiazole rings per molecule. In this case also, the refractive index is 1.63.

The above compounds are not sufficient for applications that require an ultrahigh refractive index higher than 1.65.

PTL 4 and PTL 5 describe ultrahigh-refractive index acrylate compounds including dibenzofuran or dibenzocarbazole and having a refractive index higher than 1.7. However, there is concern that this compound may discolor when heated in air, stored for long periods, or exposed to light, and it cannot be said to be a highly stable material.

### Citation List

#### Patent Literature

PTL 1: JPH6-220131A

PTL 2: JP2008-527413A

PTL 3: JP2005-133071A

PTL 4: WO2021/006011A1

PTL 5: WO2021/006012A1


### Summary of Invention

#### Technical Problem

It is an object of the present invention to provide a compound that has simultaneously a high refractive index, high transparency, and excellent chemical stability and is useful as an optical material or an optical component.

Solution to Problem

The present inventors have succeeded in improving the high refractive index, high transparency, and excellent chemical stability of polymerizable compositions and polymers, and have completed this technology.

The present invention is summarized as follows.

[1] A compound represented by the following formula (1):

[Chem. 1]

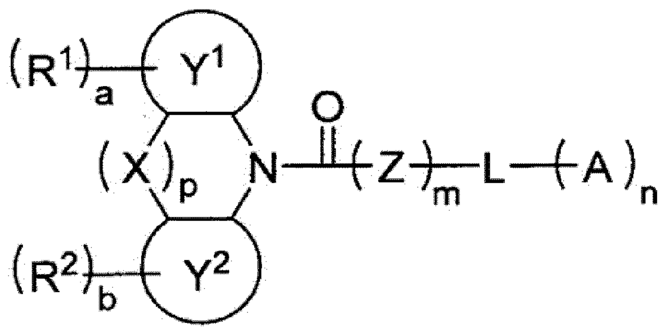

(1)

[In the formula, A represents a polymerizable group. n represents an integer of 1 to 3.

L represents an optionally branched (n+1)-valent linking group.

Z represents an oxygen atom, or a nitrogen atom optionally having a substituent. m represents an integer of 0 or 1.

X represents a single bond, an oxygen atom, a sulfur atom, a carbon atom optionally having a substituent, or a nitrogen atom optionally having a substituent. p represents an integer of 0 to 2. When p is 2, the two Xs may be the same or different.

$Y^1$ and $Y^2$ are each independently a benzene ring, a naphthalene ring, or a phenanthrene ring. When $Y^1$ or $Y^2$ is a benzene ring, a or b corresponding to $Y^1$ or $Y^2$ is an integer of 0 to 4. When $Y^1$ or $Y^2$ is a naphthalene ring, a or b corresponding to $Y^1$ or $Y^2$ is an integer of 0 to 6. When $Y^1$ or $Y^2$ is a phenanthrene ring, a or b corresponding to $Y^1$ or $Y^2$ is an integer from 0 to 8.

$R^1$ and $R^2$ each independently represent an aromatic ring group optionally having a substituent. When a and b are 2 or more, a plurality of $R^1$s and $R^2$s may be the same or different from each other.

In the formula, $Y^1$ and $Y^2$ are not both benzene rings and a and b are not both 0.]

[2] The compound according to [1], wherein p is 0 or 1.

[3] The compound according to [1] or [2], wherein A is an oxiranyl group, a vinyl group, an isopropenyl group, an allyl group, or a (meth)acryloyl group.

[4] The compound according to [3], wherein A is a (meth)acryloyl group.

[5] The compound according to any one of [1] to [4], wherein Z is a nitrogen atom optionally having a substituent.

[6] The compound according to any one of [1] to [5], wherein $R^1$ and/or $R^2$ are a condensed aromatic ring group or a monocyclic aromatic group substituted with an aromatic ring group.

[7] The compound according to any one of [1] to [6], wherein $Y^1$ and/or $Y^2$ are a benzene ring or a naphthalene ring.

[8] The compound according to [7], wherein $Y^1$ and $Y^2$ are benzene rings.

[9] A polymerizable composition comprising the compound according to any one of [1] to [8] and a polymerization initiator.

[10] A holographic recording medium comprising the polymerizable composition according to [9].

[11] A polymer obtained by polymerizing the polymerizable composition according to [9].

[12] An optical material comprising the polymer according to [11].

[13] An optical component comprising the polymer according to [11].

[14] A large-capacity memory comprising the holographic recording medium according to [10].

[15] An optical element obtained by recording a hologram in the holographic recording medium according to [10].

[16] An AR glass comprising the optical element according to [15].

Advantageous Effects of Invention

The present invention provides a high-refractive index compound having both high transparency and excellent chemical stability such as thermal stability and useful as an optical material.

The compound of the invention is particularly useful as a reactive compound used for hard coat layers of optical lenses and optical members, and for holographic recording mediums.

The use of the compound of the invention makes it possible to obtain an optical material and an optical component having high diffraction efficiency, high light transmittance, and excellent chemical stability.

Brief Description of Drawings

[Fig. 1] Fig. 1 is a schematic illustration showing the outline of the structure of a device used for holographic recording.

Description of Embodiments

Embodiments of the present invention will next be described specifically. The present invention is not limited to the following embodiments and can be modified variously within the scope of the invention.

In the present invention, "(meth)acrylate" is a generic term for acrylate and methacrylate. A "(meth)acryloyl group" is a generic term for an acryloyl group and a meth acryloyl group. The same applies to "(meth)acrylic".

In the present invention, the phrase "a group optionally having a substituent" means that the group may have one or more substituents.

1. About Compound of Present Invention

The compound of the present invention is represented by the following formula (1). Hereinafter, the compound represented by the following formula (1) may be referred to as "compound (1)".

[Chem. 2]

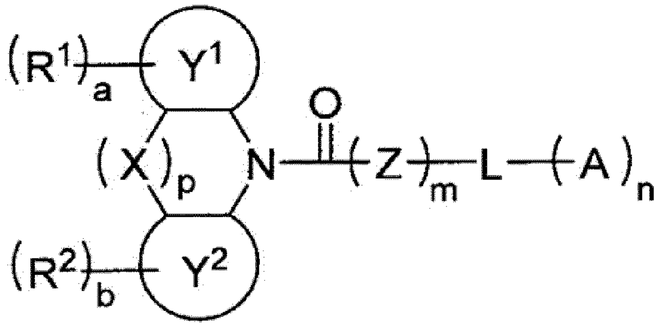

(1)

[In the formula, A represents a polymerizable group. n represents an integer of 1 to 3.

L represents an optionally branched (n+1)-valent linking group.

Z represents an oxygen atom, or a nitrogen atom optionally having a substituent. m represents an integer of 0 or 1.

X represents a single bond, an oxygen atom, a sulfur atom, a carbon atom optionally having a substituent, or a nitrogen atom optionally having a substituent. p represents an integer of 0 to 2. When p is 2, the two Xs may be the same or different.

$Y^1$ and $Y^2$ are each independently a benzene ring, a naphthalene ring, or a phenanthrene ring. When $Y^1$ or $Y^2$ is a benzene ring, a or b corresponding to $Y^1$ or $Y^2$ is an integer of 0 to 4. When $Y^1$ or $Y^2$ is a naphthalene ring, a or b corresponding to $Y^1$ or $Y^2$ is an integer of 0 to 6. When $Y^1$ or $Y^2$ is a phenanthrene ring, a or b corresponding to $Y^1$ or $Y^2$ is an integer from 0 to 8.

$R^1$ and $R^2$ each independently represent an aromatic ring group optionally having a substituent. When a and b are 2 or more, a plurality of $R^1$s and $R^2$s may be the same or different from each other.

In the formula, $Y^1$ and $Y^2$ are not both benzene rings and a and b are not both 0.]

1-1. About compound (1)

The compound (1) is characterized by a structure wherein a linking group having a polymerizable group and an aromatic amino group that exhibits a high refractive index are bonded via a carbonyl bond.

In optical materials, aromatic amine compounds such as carbazoles are sometimes used as radical polymerization initiators that generate radical species by light irradiation. However, in order to improve chemical stability against heating, light irradiation, and oxidation, which is the subject of the present invention, the generation and decomposition of reactive species from the aromatic amine structure, including radical species, is strictly prohibited.

In the compound (1), by bonding the carbonyl bond, which is an electron-withdrawing group, to an aromatic amino group that is rich in electrons and highly reactive, the so-called HOMO is lowered, thereby imparting chemical stability and suppressing decomposition and coloration due to heat, light irradiation, oxidation, and the like. In addition, by bonding a flexible linking group having a polymerizable group to an aromatic amine structure that exhibits a high refractive index and tend to become rigid, the solubility in various media and polymerizability of the compound (1) can be improved.

In the formula (1), when $Y^1$ and $Y^2$ are both benzene rings and a and b are both 0, the proportion of aromatic amino groups in the entire compound (1) is small, and the refractive index of the compound (1) decreases, making it insufficient for applications requiring an ultra-high refractive index exceeding 1.65. Therefore, in the formula (1), $Y^1$ and $Y^2$ are not both benzene rings and a and b are not both 0.

1-2. About A in the formula (1)

A is a polymerizable group, and no particular limitation is imposed on the structure of A. Examples of the polymerizable group include a (meth)acryloyl group, an allyl group, a vinyl group, an isopropenyl group, a vinyl-substituted phenyl group, an isopropenyl-substituted phenyl group, a vinyl-substituted naphthyl group, an isopropenyl-substituted naphthyl group, an oxiranyl group, a 2-methyloxiranyl group, and an oxetanyl group. A group suitable for the intended

polymerization method may be selected from these. In photopolymerization using a photopolymerization initiator, an oxiranyl group, a vinyl group, an allyl group, and a (meth)acryloyl group are preferable. Of these, a (meth)acryloyl group is particularly preferable because of its high reactivity.

1-3. About n in the formula (1)

n represents an integer of 1 to 3. n can be appropriately selected. For example, from the viewpoint of increasing the ease of polymerization of the compound of the formula (1), n may be 2 or 3.

In the compound of the formula (1), the smaller the number of polymerizable group As, the better, because the refractive index tends to increase. More preferably, the compound of the formula (1) is a monofunctional group having one polymerizable group A. Specifically, from the viewpoint of increasing the refractive index, n is preferably 1 or 2 and more preferably 1.

1-4. About L in the formula (1)

L represents an optionally branched (n+1)-valent linking group. L may contain an oxygen atom, a sulfur atom, or a nitrogen atom optionally having a substituent.

As the linking group constituting L, an aliphatic hydrocarbon group optionally having a substituent is preferred from the viewpoint of ease of synthesis and availability. The number of carbon atoms in the aliphatic hydrocarbon group (excluding the number of carbon atoms in a substituent) is preferably 1 to 8. When the number of carbon atoms in the aliphatic hydrocarbon group is 8 or less, the refractive index of the compound (1) is unlikely to decrease. In addition, since the molecular weight is small, the viscosity is small, and the processability tends to be improved. The aliphatic hydrocarbon group forming L may be any of a cyclic aliphatic hydrocarbon group and a chain aliphatic hydrocarbon group, and a combination of these structures may be used. From the viewpoint of reducing steric hindrance around the polymerizable group A, a chain aliphatic hydrocarbon group is preferable.

As the chain aliphatic hydrocarbon group constituting L, when n=1, an alkyl group having 1 to 8 carbon atoms may be mentioned. When n=2 or 3, L may be a combination of two or more alkyl groups having 1 to 8 carbon atoms.

L is preferably an aliphatic hydrocarbon group having an oxygen atom, a sulfur atom, or a nitrogen atom optionally having a substituent, from the viewpoint of imparting high solubility to various media to the compound (1). The aliphatic hydrocarbon group may have a substituent. The number of carbon atoms in the aliphatic hydrocarbon group (excluding the number of carbon atoms in a substituent) is preferably 1 to 8. When the number of carbon atoms in the aliphatic hydrocarbon group is 8 or less, the refractive index of the compound (1) is unlikely to decrease. In addition, since the molecular weight is small, the viscosity is small, and the processability tends to be improved. The aliphatic hydrocarbon group forming L may be any of a cyclic aliphatic hydrocarbon group and a chain aliphatic hydrocarbon group, and a combination of these structures may be used. From the viewpoint of reducing steric hindrance around the polymerizable group A, a chain aliphatic hydrocarbon group is preferable.

Examples of the chain aliphatic hydrocarbon group having an oxygen atom, a sulfur atom, or a nitrogen atom optionally having a substituent constituting L include the following.

When n=1, examples include an oxomethylene group, an oxoethylene group, an oxopropylene group, a 2-oxopropylene group, an oxobutylene group, an oxopentylene group, an oxohexylene group, an oxoheptylene group, an oxooctylene group, $-CH_2CH_2OCH_2CH_2-$, $-CH_2CH_2OCH_2CH_2O-$, $-CH_2CH_2OCH_2CH_2OCH_2CH_2-$, $-CH_2CH_2OCH_2CH_2OCH_2CH_2O-$, $-CH_2CH_2OCH_2CH_2NH-$, $-CH_2CH_2OCH_2CH_2OCH_2CH_2NH-$, $-CH_2CH_2SCH_2CH_2-$, $-CH_2CH_2SCH_2CH_2O-$, and the like.

L may be a combination of two or more of these groups.

Examples of L when n=2 or 3 include $-C(CH_3)(CH_2O)_2-$ and a linking group wherein any hydrogen atom in the above chain aliphatic hydrocarbon group is replaced with a bond to a polymerizable group. In this case, L may be bonded to the polymerizable group via a branched structure.

From the viewpoint of a high refractive index, L preferably contains a cyclic group, and the ring included in the cyclic group forming L may have a monocyclic structure or a fused ring structure. The number of rings included in L is preferably 1 to 4, more preferably 1 to 3, and still more preferably 1 to 2. Each ring included in L does not necessarily have aromaticity. However, to keep the size of L in the molecule small and maintain the high refractive index, L is preferably an aromatic hydrocarbon ring.

Examples of the aromatic hydrocarbon ring included in L include a benzene ring, an indene ring, a naphthalene ring, an azulene ring, a fluorene ring, an acenaphthylene ring, an anthracene ring, a phenanthrene ring, a pyrene ring, and the like.

The Linking group L may have a substituent. Examples of the optional substituent on L include halogen atoms (a chlorine atom, a bromine atom, and an iodine atom), a hydroxy group, a mercapto group, an alkyl group having 1 to 8 carbon atoms, an alkenyl group having 2 to 8 carbon atoms, an alkoxy group having 1 to 8 carbon atoms, a phenyl group, a

mesityl group, a tolyl group, a naphthyl group, a cyano group, an acetyloxy group, an alkylcarbonyloxy group having 2 to 9 carbon atoms, an alkoxycarbonyl group having 2 to 9 carbon atoms, a sulfamoyl group, an alkylsulfamoyl group having 2 to 9 carbon atoms, an alkylcarbonyl group having 2 to 9 carbon atoms, a phenethyl group, a hydroxyethyl group, an acetylamido group, a dialkylaminoethyl group to which alkyl groups having 1 to 4 carbon atoms are bonded, a trifluoromethyl group, an alkylthio group having 1 to 8 carbon atoms, an aromatic thio group having 6 to 10 carbon atoms, and a nitro group.

1-5. About $Y^1$, $Y^2$, a, and b in the formula (1)

$Y^1$ and $Y^2$ are each independently a benzene ring, a naphthalene ring, or a phenanthrene ring.
When $Y^1$ or $Y^2$ is a benzene ring, a or b corresponding to $Y^1$ or $Y^2$ is an integer from 0 to 4.
When $Y^1$ or $Y^2$ is a naphthalene ring, a or b corresponding to $Y^1$ or $Y^2$ is an integer from 0 to 6.
When $Y^1$ or $Y^2$ is a phenanthrene ring, a or b corresponding to $Y^1$ or $Y^2$ is an integer from 0 to 8.
From the viewpoints of ease of synthesis and availability of raw materials, $Y^1$ and $Y^2$ are each preferably independently a benzene ring or a naphthalene ring, and from the viewpoints of chemical stability and low coloring, more preferably a benzene ring.

1-6. About $R^1$ and $R^2$ in the formula (1)

$R^1$ and $R^2$ represent aromatic ring groups optionally having a substituent. The aromatic rings are broadly classified into an aromatic hydrocarbon ring and an aromatic heterocycle.
Examples of the aromatic hydrocarbon ring include groups such as a benzene ring, a naphthalene ring, an anthracene ring, a phenanthrene ring, a perylene ring, a tetracene ring, a pyrene ring, a benzpyrene ring, a chrysene ring, a biphenylene ring, a triphenylene ring, an acenaphthene ring, a fluoranthene ring, and a fluorene ring.
Examples of the aromatic heterocycle include: aromatic heterocycles including one heteroatom such as a furan ring, a benzofuran ring, a dibenzofuran ring, a naphthofuran ring, a benzonaphthofuran ring, a dinaphthofuran ring, a thiophene ring, a benzothiophene ring, a dibenzothiophene ring, a naphthothiophene ring, a benzonaphthothiophene ring, a dinaphthothiophene ring, a pyrrole ring, an indole ring, a carbazole ring, a benzo carbazole ring, a dibenzo carbazole ring, a pyridine ring, a quinoline ring, and an isoquinoline ring; aromatic heterocycles including two or more heteroatoms such as an imidazole ring, a triazole ring, a tetrazole ring, an oxazole ring, a thiazole ring, a pyridazine ring, a pyrimidine ring, a pyrazine ring, a triazine ring, and a thiadiazole ring; and, rings each including two or three fused rings including an aromatic heterocycle having two or more heteroatoms such as a benzoxazole ring, a thienooxazole ring, a thiazolooxazole ring, an oxazolooxazole ring, an oxazoloimidazole ring, an oxazolopyridine ring, an oxazolopyridazine ring, an oxazolopyrimidine ring, an oxazolopyrazine ring, a naphthooxazole ring, a quinolinooxazole ring, a dioxazolopyrazine ring, a phenoxazine ring, a benzothiazole ring, a furothiazole ring, a thienothiazole ring, a thiazolothiazole ring, a thiazoloimidazole ring, a thienothiadiazole ring, a thiazolothiadiazole ring, a thiazolopyridine ring, a thiazolopyridazine ring, a thiazolopyrimidine ring, a thiazolopyrazine ring, a naphthothiazole ring, a quinolinothiazole ring, a thianthrene ring, and a phenothiazine ring.
From the viewpoints of ease of synthesis and availability, the aromatic hydrocarbon rings included in $R^1$ and $R^2$ are preferably a benzene ring, a naphthalene ring, an anthracene ring, a phenanthrene ring, a pyrene ring, a biphenylene ring, or a fluorene ring. From the viewpoint of reducing fluorescence from the compound (1), the aromatic hydrocarbon rings included in $R^1$ and $R^2$ each independently is more preferably a benzene ring, a naphthalene ring, a biphenylene ring, or a fluorene ring.
The aromatic heterocycles included in $R^1$ and $R^2$ are preferably a sulfur-containing aromatic heterocycle because the refractive index of the compound (1) tends to increase. The sulfur-containing aromatic heterocycle has at least a sulfur atom as a heteroatom included in the aromatic heterocycle. The sulfur-containing aromatic heterocycle may have, in addition to the sulfur atom, an oxygen atom or a nitrogen atom as a heteroatom and may have an oxygen atom and a nitrogen atom. From the viewpoint of avoiding coloration and obtaining sufficient solubility, the number of heteroatoms included in the sulfur-containing aromatic heterocycle is preferably 1 to 3 and more preferably 1 to 2.
Examples of the sulfur-containing aromatic heterocycle include: aromatic heterocycles including one sulfur atom such as a thiophene ring, a benzothiophene ring, a dibenzothiophene ring, a benzonaphthothiophene ring, a dinaphthothiophene ring, a thiopyran ring, a naphthothiophene ring, a dinaphthothiophene ring, and a dibenzothiopyran ring; aromatic heterocycles including two or more sulfur atoms such as a thianthrene ring; and aromatic heterocycles including two or more types of heteroatoms such as a thiazole ring, an isothiazole ring, a benzothiazole ring, a naphthothiazole ring, a phenothiazine ring, a thiazoloimidazole ring, a thiazolopyridine ring, a thiazolopyridazine ring, a thiazolopyrimidine ring, a dioxazolopyrazine ring, a thiazolopyrazine ring, a thiazolooxazole ring, a dibenzobenzothiophene ring, a thienooxazole ring, a thienothiadiazole ring, a thiazolothiadiazole ring, and the like.
The sulfur-containing aromatic heterocycle may be a monocycle or may be a fused ring. From the viewpoint of

increasing the refractive index, the sulfur-containing aromatic heterocycle is preferably a fused ring. The number of rings forming the fused ring is preferably 2 to 8, more preferably 2 to 6, and still more preferably 2 to 5 in terms of availability of raw materials and ease of synthesis.

Among the above rings, a benzothiazole ring, a dibenzothiophene ring, a benzothiophene ring, a benzonaphthothiophene ring, a dinaphthothiophene ring, and a thianthrene ring are preferred in terms of low colorability and obtaining a high refractive index.

From the viewpoint of ease of synthesis, the aromatic heterocycles included in $R^1$ and $R^2$ may be a nitrogen-containing aromatic heterocycle. The nitrogen-containing aromatic heterocycle has at least a nitrogen atom as a heteroatom included in the aromatic heterocycle. The nitrogen-containing aromatic heterocycle may have, in addition to the nitrogen atom, an oxygen atom or a sulfur atom as a heteroatom and may have an oxygen atom and a sulfur atom. From the viewpoint of avoiding coloration, the number of heteroatoms included in the nitrogen-containing aromatic heterocycle is preferably 1 to 3 and more preferably 1 to 2.

Examples of the nitrogen-containing aromatic heterocycles include: aromatic heterocycles including one nitrogen atom such as pyrrole ring, indole ring, carbazole ring, benzocarbazole ring, dibenzocarbazole ring, pyridine ring, quinoline ring, isoquinoline ring, oxazole ring, thiazole ring, benzoxazole ring, naphthoxazole ring, benzothiazole ring, naphthothiazole ring, phenoxazine ring, phenothiazine ring, thienooxazole ring, thiazoloxazole ring, oxazoloxazole ring, furothiazole ring, thienothiazole ring, thiazolothiazole ring; and, aromatic heterocycles including two or more nitrogen atoms, such as imidazole ring, triazole ring, tetrazole ring, pyridazine ring, pyrimidine ring, pyrazine ring, triazine ring, thiadiazole ring, benzimidazole ring, oxazoloimidazole ring, oxazolopyridine ring, oxazolopyridazine ring, oxazolopyrimidine ring, oxazolopyrazine ring, quinolinoxazole ring, dioxazolopyrazine ring, thiazoloimidazole ring, thienothiadiazole ring, thiazolothiadiazole ring, thiazolopyridine ring, thiazolopyridazine ring, thiazolopyrimidine ring, thiazolopyrazine ring, and quinolinothiazole ring.

The nitrogen-containing aromatic heterocycle may be a monocycle or may be a fused ring. From the viewpoint of increasing the refractive index, the nitrogen-containing aromatic heterocycle is preferably a fused ring. The number of rings forming the fused ring is preferably 2 to 8, more preferably 2 to 6, and still more preferably 2 to 5 in terms of availability of raw materials and ease of synthesis.

In terms of low colorability and increasing the refractive index, the nitrogen-containing aromatic heterocycle is preferably a carbazole ring, a benzocarbazole ring, a dibenzocarbazole ring, a pyridine ring, a quinoline ring, an isoquinoline ring, a benzoxazole ring, a benzothiazole ring, a benzimidazole ring, or a thiadiazole ring; and is more preferably a carbazole ring, a benzocarbazole ring, a dibenzocarbazole ring, a benzoxazole ring, a benzothiazole ring, a benzimidazole ring, or a thiadiazole ring.

The aromatic heterocycles included in $R^1$ and $R^2$ may be an oxygen-containing aromatic heterocycle. In the case of an oxygen-containing aromatic heterocycle, the heat resistance and weather resistance of the polymer obtained using the compound (1) as a raw material tend to be improved. The oxygen-containing aromatic heterocycle has at least an oxygen atom as a heteroatom included in the aromatic heterocycle. The oxygen-containing aromatic heterocycle may have, in addition to the oxygen atom, a nitrogen atom or a sulfur atom as a heteroatom and may have a nitrogen atom and a sulfur atom. In terms of obtaining sufficient heat resistance, the number of oxygen atoms included in the oxygen-containing aromatic heterocycle is preferably 1 to 3 and more preferably 1 to 2.

Examples of the oxygen-containing aromatic heterocyclic rings include: aromatic heterocycles containing one oxygen atom such as furan ring, benzofuran ring, dibenzofuran ring, naphthofuran ring, benzonaphthofuran ring, dinaphthofuran ring, phenoxazine ring, oxazole ring, isoxazole ring, benzoxazole ring, benzoisoxazole ring, naphthoxazole ring, thienooxazole ring, thiazoloxazole ring, oxazoloimidazole ring, and furothiazole ring; and, aromatic heterocycles containing two or more oxygen atoms such as a dibenzodioxin ring, an oxazoloxazole ring, and a dioxazolopyrazine ring.

The oxygen-containing aromatic heterocycle may be a monocycle or may be a fused ring. From the viewpoint of increasing the refractive index, a fused ring is preferred. The number of rings forming the fused ring is preferably 2 to 8 and more preferably 2 to 6 and is particularly preferably 2 to 5 in terms of availability of raw materials and ease of synthesis.

In particular, in terms of low colorability and increasing the refractive index, the oxygen-containing aromatic heterocycle is preferably a dibenzofuran ring, a benzonaphthofuran ring, a dinaphthofuran ring, an oxazole ring, an isoxazole ring, a benzoxazole ring, a benzoisoxazole ring, or a naphthooxazole ring and more preferably a dibenzofuran ring, a benzonaphthofuran ring, a dinaphthofuran ring, or a benzoxazole ring.

The aromatic rings constituting these $R^1$ and $R^2$ may have a substituent. Examples of the substituent include halogen atoms such as chlorine, bromine, and iodine, an alkyl group having 1 to 8 carbon atoms, an alkenyl group having 2 to 8 carbon atoms, an alkynyl group having 2 to 8 carbon atoms, an alkoxyl group, a cyano group, an acetyloxy group, an alkylcarbonyloxy group having 2 to 9 carbon atoms, an alkoxycarbonyl group having 2 to 9 carbon atoms, a sulfamoyl group, an alkylsulfamoyl group having 2 to 9 carbon atoms, an alkylcarbonyl group having 2 to 9 carbon atoms, a phenethyl group, a hydroxyethyl group, an acetylamido group, a dialkylaminoethyl group to which an alkyl group having 1 to 4 carbon atoms is bonded, a trifluoromethyl group, an alkylthio group having 1 to 8 carbon atoms, an arylthio group having 6 to 10

carbon atoms, and a nitro group. Of these, an alkyl group having 1 to 8 carbon atoms, an alkoxyl group having 1 to 8 carbon atoms, an alkylthio group having 1 to 8 carbon atoms, an arylthio group having 6 to 10 carbon atoms, a cyano group, an acetyloxy group, an alkylcarboxyl group having 2 to 8 carbon atoms, a sulfamoyl group, an alkylsulfamoyl group having 2 to 9 carbon atoms, a nitro group are preferred.

From the viewpoint of increasing the refractive index of the compound (1), it is preferable that the above aromatic rings included in $R^1$ and $R^2$ further have, as a substituent, a group having an aromatic ring. The aromatic ring included in the substituent has the same meaning as the aromatic rings included in $R^1$ and $R^2$. The aromatic ring included in the substituent may be bonded directly to the aromatic groups included in $R^1$ and $R^2$ at any position, may be bonded to the aromatic group via an oxygen atom, a sulfur atom, or a nitrogen atom optionally having a substituent, or may be bonded to the aromatic group via any linking group. More preferably, the substituent is bonded directly to the aromatic groups included in a $R^1$ and $R^2$.

When the aromatic ring included in the substituent is a sulfur-containing aromatic heterocycle, the refractive index of the compound (1) tends to be higher. The definition of the sulfur-containing aromatic heterocycle is the same as the definition of that in $R^1$ and $R^2$. The sulfur-containing aromatic heterocycle is more preferably a fused ring and is particularly preferably a benzothiazole ring, a dibenzothiophene ring, a benzothiophene ring, a benzonaphthothiophene ring, a dinaphthothiophene ring, or a thianthrene ring.

No particular limitation is imposed on the number of aromatic rings included as substituents in $R^1$ and $R^2$. From the viewpoint of ease of synthesis and solubility, the number of aromatic rings is preferably 1 to 4 and more preferably 1 to 2.

From the viewpoint of obtaining simultaneously a high refractive index and high solubility in various mediums, the aromatic rings included in $R^1$ and $R^2$ are each independently preferably a fused aromatic ring optionally having a substituent or a monocyclic aromatic ring substituted with an aromatic ring group and more preferably a fused aromatic heterocycle optionally having a substituent or an aromatic hydrocarbon ring having an aromatic heterocycle as a substituent.

The aromatic rings constituting $R^1$ and $R^2$ may have two or more selected from the above mentioned sulfur-containing aromatic heterocycle, nitrogen-containing aromatic heterocycle, and oxygen-containing aromatic heterocycle.

1-7. About Z in the formula (1)

Z represents an oxygen atom or a nitrogen atom optionally having a substituent, that is, a nitrogen atom which has no substituent or a nitrogen atom which has a substituent.

When the nitrogen atom of Z has a substituent, examples of the substituent include an alkyl group having 1 to 8 carbon atoms, an alkenyl group having 2 to 8 carbon atoms, a phenyl group, a mesityl group, a tolyl group, a naphthyl group, an alkylcarbonyl group having 2 to 9 carbon atoms, a phenethyl group, a hydroxyethyl group, an acetylamide group, a trifluoromethyl group, an aromatic hydrocarbon group having 6 to 30 carbon atoms, a heterocyclic group having 2 to 20 carbon atoms, and the like.

From the viewpoint of solubility in various media, Z is preferably an oxygen atom. From the viewpoint of availability of raw materials, Z is preferably a nitrogen atom optionally having a substituent. In particular, from the viewpoint of ease of synthesis and expectation of a high refractive index in the polymer due to intermolecular hydrogen bonds, Z is more preferably a nitrogen atom which does not have a substituent.

1-8. About m in the formula (1)

m represents 0 or 1. This m can be appropriately selected. For example, from the viewpoint of improving the solubility of the compound (1) in various media, m=1 is preferred. On the other hand, from the viewpoint of chemical stability of the compound (1), m=0 is preferred.

1-9. About X in the formula (1)

X represents a single bond, an oxygen atom, a sulfur atom, a carbon atom optionally having a substituent, or a nitrogen atom optionally having a substituent.

X is preferably a single bond because it tends to achieve a high refractive index of the compound (1). When X=a single bond, the compound (1) is a carbazole compound, which is also preferred from the viewpoint of ease of synthesis.

Since the solubility of the compound (1) in various media tends to be improved, X is preferably an oxygen atom, a sulfur atom, a carbon atom optionally having a substituent, or a nitrogen atom optionally having a substituent. In particular, since a high refractive index tends to be achieved, a sulfur atom is more preferable as X. On the other hand, from the viewpoint of being able to adjust the physical properties for each medium used, a carbon atom optionally having a substituent or a nitrogen atom optionally having a substituent is more preferable.

Examples of the substituents that the carbon atom constituting X may have include an alkyl group having 1 to 8

carbon atoms, an alkenyl group having 2 to 8 carbon atoms, an alkoxy group having 1 to 8 carbon atoms, a phenyl group, a mesityl group, a tolyl group, a naphthyl group, an alkylcarbonyl group having 2 to 9 carbon atoms, a phenethyl group, a hydroxyethyl group, an acetylamide group, a trifluoromethyl group, an alkylthio group having 1 to 8 carbon atoms, an aromatic hydrocarbon group having 6 to 30 carbon atoms, a heterocyclic group having 2 to 20 carbon atoms, an aromatic ring thio group having 6 to 10 carbon atoms, a nitro group, and the like.

Examples of the substituents that the nitrogen atom constituting X may have include an alkyl group having 1 to 8 carbon atoms, an alkenyl group having 2 to 8 carbon atoms, a phenyl group, a mesityl group, a tolyl group, a naphthyl group, an alkylcarbonyl group having 2 to 9 carbon atoms, a phenethyl group, a hydroxyethyl group, an acetylamide group, a trifluoromethyl group, an aromatic hydrocarbon group having 6 to 30 carbon atoms, a heterocyclic group having 2 to 20 carbon atoms, and the like.

1-10. About p in the formula (1)

p represents an integer of 0 to 2. This p can be appropriately selected. For example, since the compound (1) tends to have a high refractive index, an integer of p=1 to 2 is preferred.

On the other hand, from the viewpoint of improving the solubility of the compound (1) in various media, p=0 is preferred.

From the viewpoints of high refractive index and solubility, p is preferably 0 or 1.

1-11. Preferred compound of the compound (1)

From the viewpoints of achieving a high refractive index and ease of synthesis, the compound (1) is preferably a compound represented by the following formula (2), wherein X in the formula (1) is a single bond.

[Chem. 3]

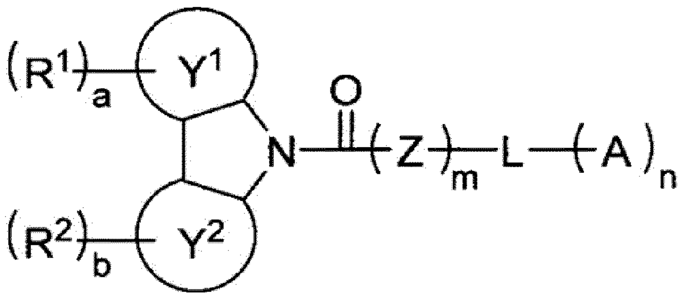

(2)

In the above formula (2), A, n, L, Z, m, $Y^1$, $Y^2$, $R^1$, $R^2$, a, and b have the same meanings as in the above formula (1), and the preferred ones are also the same.

1-12. Molecular weight

From the viewpoint of reducing the viscosity to a low level and maintaining good processability, the molecular weight of the compound (1) is preferably 2000 or less, more preferably 1500 or less, and even more preferably 1200 or less. From the viewpoint of reducing the shrinkage ratio during polymerization, the molecular weight of the compound of formula (1) is preferably 400 or more, more preferably 450 or more, and still more preferably 500 or more.

1-13. Relation between molecular structure and physical properties

The compound (1) has the effect of lowering the HOMO of an aromatic amine compound by bonding a flexible linking group containing a polymerizable group via a carbonyl bond to the nitrogen atom of a highly planar aromatic amine compound such as a carbazole compound, a benzocarbazole compound, a dibenzocarbazole compound, a phenothiazine compound, a phenoxazine compound, a dihydroacridine compound, an iminostilbene compound, and the like. This makes it possible to simultaneously achieve improved compound stability against heating, light irradiation, oxidation, etc.,

ensure the solubility of the monomer in various media, and adjust the compatibility of the polymer with the medium after the polymerization reaction.

In general, the introduction of a carbonyl bond onto the aromatic ring of an aromatic amine compound can also be expected to have the effect of lowering the HOMO of the aromatic amine compound. However, in this case, the energy difference between the HOMO and LUMO tends to be smaller than that of the compound (1), and there is a concern that the compound may become colored. Therefore, for use in optical materials that are colorless and transparent in the visible range, it is preferable to introduce a carbonyl bond onto the nitrogen atom as in the formula (1).

Furthermore, when the compound (1) has $R^1$ and $R^2$, a molecular twist wherein the dihedral angle at the linking site between $R^1$ or $R^2$ and the aromatic amino structure is 0 degrees or more can be appropriately introduced into the monomer and polymer. This makes it possible to further improve the solubility in various media, and allowes the compound to be used as a polymerizable monomer having an ultra-high refractive index.

1-14. Exemplary compounds

Specific examples of the compound (1) are exemplified below. The compound (1) of the present invention is not limited to these compounds, and other compounds may be used so long as they do not depart from the scope of the invention.

[Chem. 4]

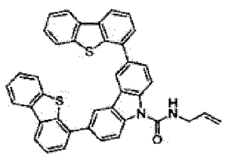
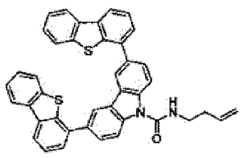
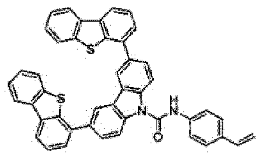
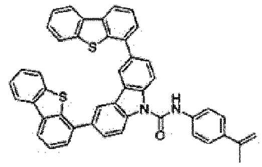
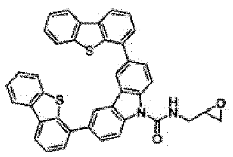
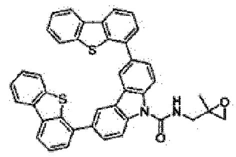
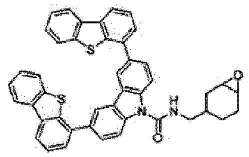
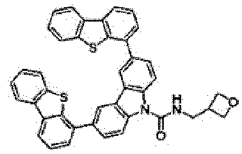
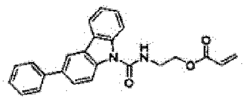
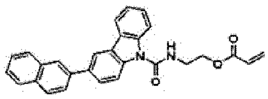
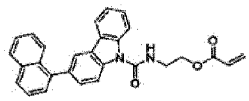
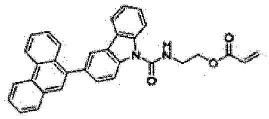
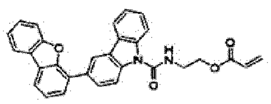
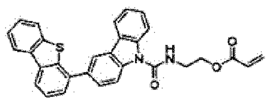
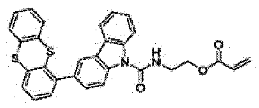
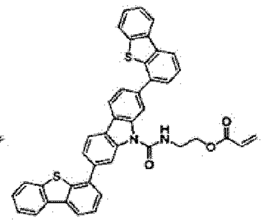

[Chem. 5]

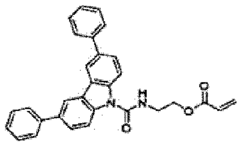
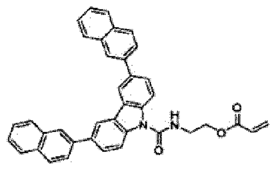
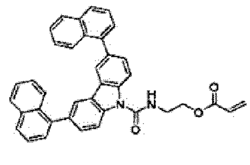
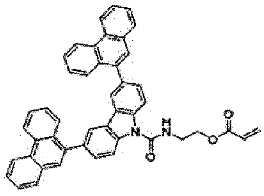
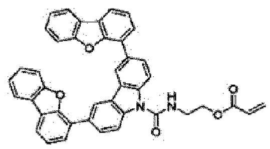
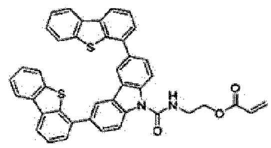
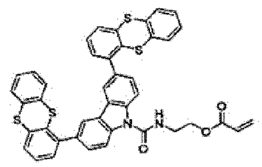
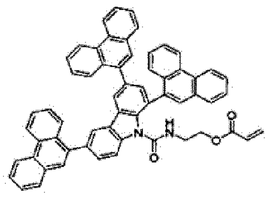
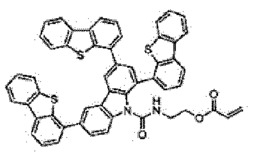
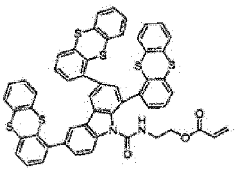
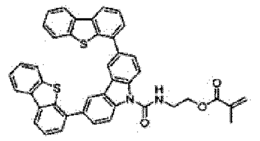
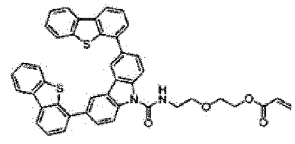
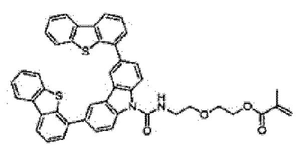
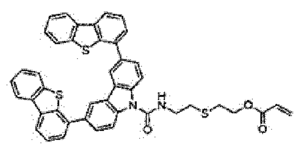
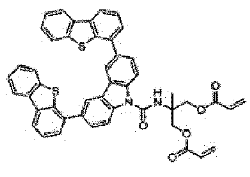
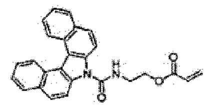

[Chem. 6]

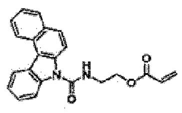
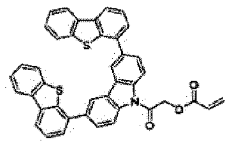
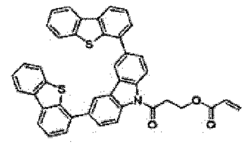
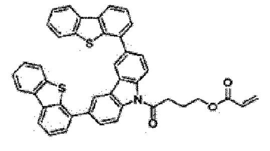
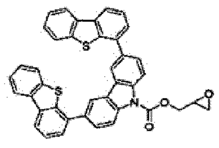
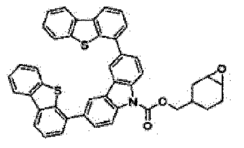
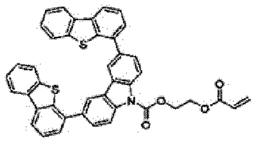
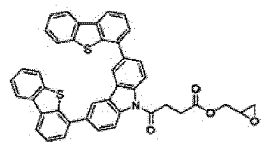
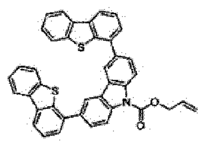
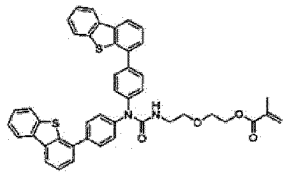
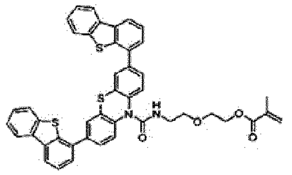
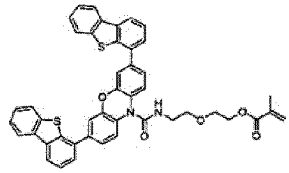
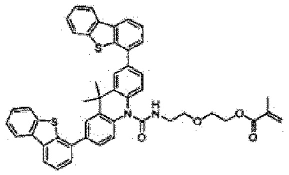
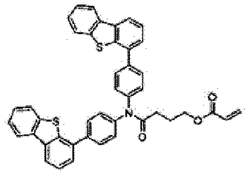
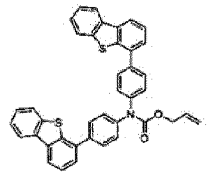
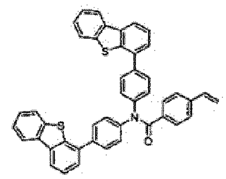
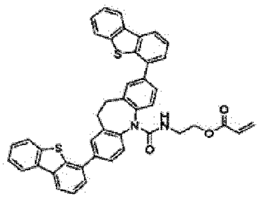
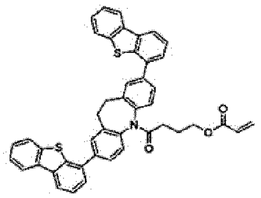
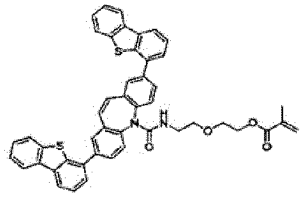
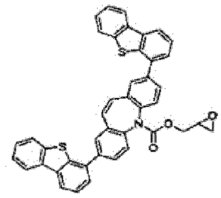

1-15. About synthesis method

The compound (1) can be synthesized using a combination of various known methods. For example, the compound of formula (1) can be synthesized through the reaction of a compound represented by the following formula (3) (hereinafter sometimes referred to as "compound (3)") and a carbonylation reagent such as isocyanate having a polymerizable group.

[Chem. 7]

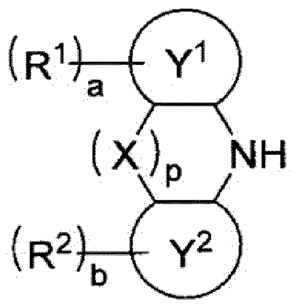

(3)

[In the formula, X, p, $Y^1$, $Y^2$, $R^1$, $R^2$, a, and b have the same meanings as in the formula (1).]
One synthesis example of the compound (1) will be described below.

[Chem. 8]

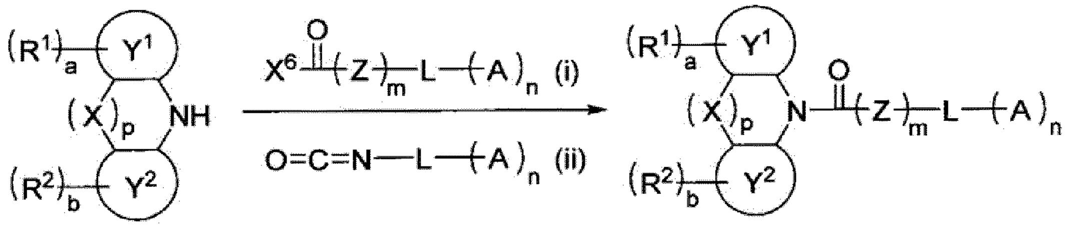


(3) (1)

[In the above reaction formula, Z, m, L, A, and n have the same meanings as in the formula (1). $X^6$ represents a leaving group such as a halogen atom, and the like. In the following reaction formulas, the same symbols as in the formula (1) represent the same things.]

For example, the compound (1) can be produced by reacting the amino group of the compound (3) with a carbonylation reagent compound having a polymerizable group represented by the formula (i) (hereinafter, sometimes referred to as carbonylation reagent (i)).

When Z is a nitrogen atom having no substituent, the compound (1) can also be produced using isocyanates represented by the above formula (ii) (hereinafter, sometimes referred to as "isocyanates (ii)").

Examples of the carbonylation reagent (i) include acid chlorides such as 4-vinylbenzoyl chloride, and the like, and chloroformates such as allyl chloroformate, and the like.

Examples of the isocyanates (ii) include 2-acryloyloxyethyl isocyanate, 2-methacryloyloxyethyl isocyanate, 2-(2-methacryloyloxyethyloxy)ethyl isocyanate, 1,1-(bisacryloyloxymethyl)ethyl isocyanate, and the like.

A known method can be applied to the reaction of the active hydrogen of the amino group in the formula (3) with the carbonylation reagent (i) or the isocyanates (ii). For example, the compound (1) can be obtained by reacting the compound (3) with the isocyanates (ii) in the presence of a basic compound.

The basic compound may be at least one of organic basic compounds (such as triethylamine, diisopropylethylamine, 1,1,3,3-tetramethylguanidine, diazabicycloundecene, diazabicyclononene, pyridine, imidazole, and the like) or at least one of inorganic basic compounds (such as sodium carbonate, potassium carbonate, sodium hydride, potassium hydride, potassium tert-butoxide, and the like), or a combination of at least one of the organic basic compounds and at least one of the inorganic basic compounds.

In the reaction of the compound (3) with the carbonylation reagent (i) or the isocyanates (ii), it is preferable to use an organic solvent. Examples of organic solvent include dichloromethane, tetrahydrofuran (THF), dimethoxyethane, toluene, N,N-dimethylformamide (DMF), and the like. One organic solvent may be used, or two or more organic solvents may be used in combination.

In the production of the compound (1), it is preferable to purify a reaction product (crude product) obtained by the synthetic reaction. By removing impurities by purification, low colorability can be achieved. A well-known purification method can be used. For example, an extraction method, column chromatography, a recrystallization method, a distillation method, etc. may be used for the purification. One of these purification methods may be performed, or these purification methods may be carried out in sequence in combination.

When the compound (1) is solid at room temperature, it is preferable to use a recrystallization method because coloring materials can be efficiently removed with ease.

Examples of the recrystallization solvent include: aliphatic hydrocarbons such as n-pentane, n-hexane, n-heptane, and the like; alicyclic hydrocarbons such as cyclopentane, cyclohexane, and the like; aromatic hydrocarbons such as toluene, ethylbenzene, xylene, mesitylene, and the like; halogenated hydrocarbons such as methylene chloride, chloroform, 1,2-dichloroethane, and the like; ethers such as diethyl ether, diisopropyl ether, tetrahydrofuran, t-butyl methyl ether, 1,4-dioxane, and the like; ketones such as acetone, methyl ethyl ketone, methyl isobutyl ketone, and the like; esters such as ethyl acetate, n-butyl acetate, propylene glycol monomethyl ether acetate, and the like; nitriles such as acetonitrile, propionitrile, and the like; alcohols such as methanol, ethanol, n-propanol, isopropanol, n-butanol, 2-butanol, t-butanol, 2-methoxyethanol, 2-butoxyethanol, propylene glycol monomethyl ether, and the like; glycols such as ethylene glycol, diethylene glycol, and the like; water, and the like. One of these solvents may be used alone, or a combination of two or more of them may be used.

[Chem. 9]

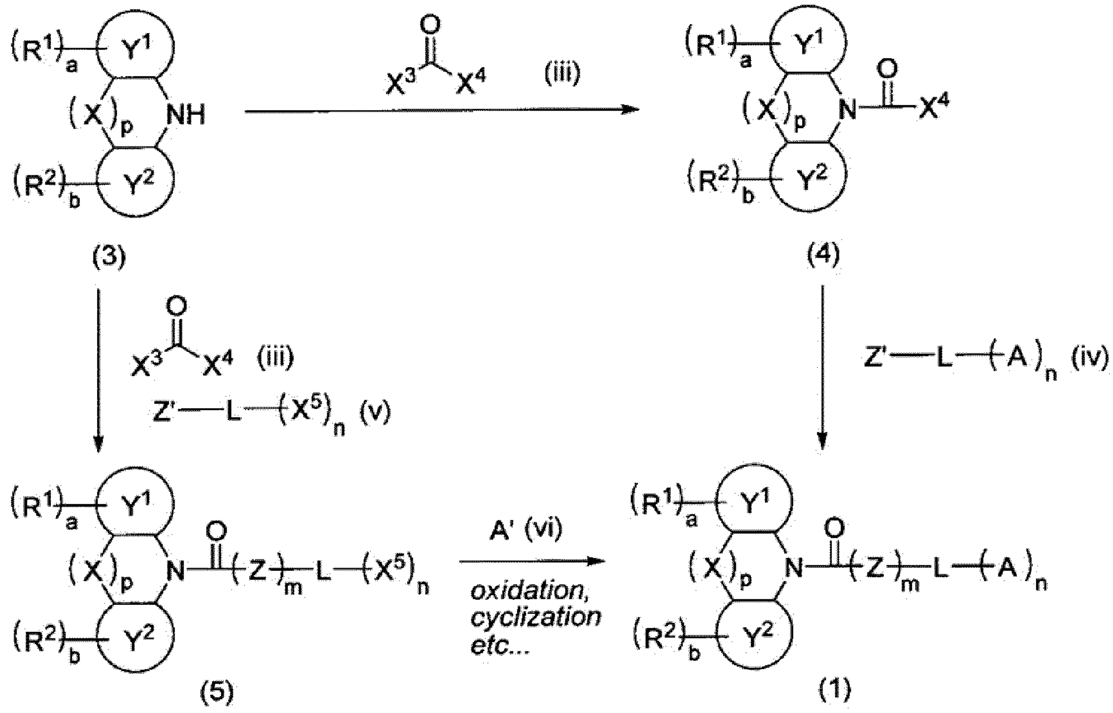


(3) (4) (5) (1)

[In the above reaction formula, $X^3$, $X^4$, and $X^5$ represent leaving groups such as halogen atoms, and the like. Z' and A' represent chemical species that are precursors of Z and A.]

The compound (1) can also be produced by further reacting a compound represented by the above formula (4) (hereinafter sometimes referred to as "intermediate (4)"), which is obtained by reacting the compound (3) with a carbonylation reagent represented by the above formula (iii) (hereinafter sometimes referred to as "carbonylation reagent (iii)"), such as phosgene, triphosgene, carbonyldiimidazole, or the like, as an intermediate with a reagent represented by the above formula (iv) having a polymerizable group (hereinafter sometimes referred to as "reagent (iv)").

Furthermore, the compound (1) can also be produced by allowing a polymerizable group precursor represented by the formula (vi) to act on a compound represented by the formula (5) (hereinafter sometimes referred to as "compound (5)") obtained by a reaction between the compound (3), the carbonylation reagent (iii), and a compound having a linking group represented by the formula (v) above, or by a polymerizable group-forming reaction such as an oxidation reaction or cyclization reaction of the compound (5).

The compound (3), which is the starting material for the compound (1), can be produced by combining various known methods.

[Chem. 10]

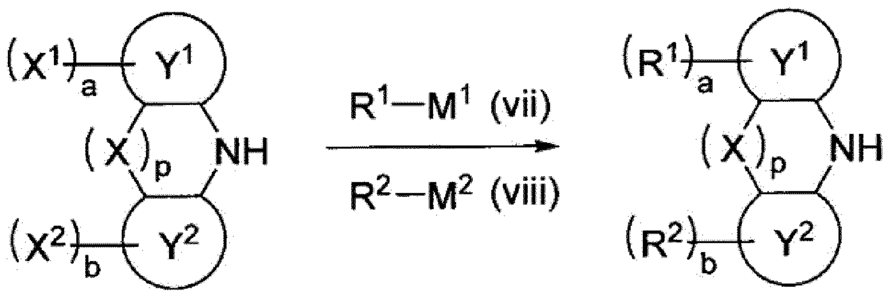


(6) (3)

For example, the compound (3) can be synthesized by simultaneously or sequentially linking aromatic groups $R^1$ and $R^2$ through a linking reaction between an aromatic halide represented by the formula (6) and organic reagents represented by the formulas (vii) and (viii).

2. About polymerizable composition of the present invention

The polymerizable composition of the present invention contains the compound (1) and a polymerization initiator.
The polymerization initiator initiates a polymerization reaction of the polymerizable functional group A in the compound (1), and the polymer of the present invention can thereby be obtained.

2-1. Polymerization initiator

There is no particular limitation on the type of polymerization initiator, and a suitable polymerization initiator may be selected from known polymerization initiators according to the polymerization method.
There is no limitation on the polymerization method, and the polymerization may be performed by any known method such as a bulk polymerization method, a solution polymerization method, a suspension polymerization method, an emulsion polymerization method, a partial polymerization method, or the like.
Examples of the polymerization initiator contained in the polymerizable composition of the present invention include radical polymerization initiators, redox-based polymerization initiators, anionic polymerization initiators, cationic polymerization initiators, and the like. Moreover, a cationic photopolymerization initiator that generates cations that are active species under irradiation with light can also be used.
Examples of the polymerization initiator described later include initiators referred usually to as polymerization catalysts.

2-1-1. Radical polymerization initiator

<Photopolymerization initiator>

Any known photo radical polymerization initiator can be used as the photopolymerization initiator that assists the polymerization of the polymerizable composition of the present invention. Examples of the photopolymerization initiator used include azo-based compounds, azide-based compounds, organic peroxides, organic borates, onium salts, bisimidazole derivatives, titanocene compounds, iodonium salts, organic thiol compounds, halogenated hydrocarbon derivatives, acetophenones, benzophenones, hydroxybenzenes, thioxanthones, anthraquinones, ketals, acylphosphine oxides, sulfone compounds, carbamic acid derivatives, sulfonamides, triarylmethanols, and oxime esters. In particular, the photopolymerization initiator is preferably benzophenones, acylphosphine oxide compounds, oxime ester compounds, etc. in terms of compatibility, availability, and the like.
Specific examples of the photopolymerization initiator include benzophenone, 2,4,6-trimethylbenzophenone, methylorthobenzoylbenzoate, 4-phenylbenzophenone, t-butylanthraquinone, 2-ethylanthraquinone, diethoxyacetophenone, 2-hydroxy-2-methyl-1-phenylpropan-1-one, oligo{2-hydroxy-2-methyl-1-[4-(1-methylvinyl)phenyl]propanone}, benzil dimethyl ketal, 1-hydroxycyclohexyl phenyl ketone, benzoin methyl ether, benzoin ethyl ether, benzoin isopropyl ether, benzoin isobutyl ether, 2-methyl-[4-(methylthio)phenyl]-2-morpholino-1-propanone, 2-benzyl-2-dimethylamino-1-(4-morpholinophenyl)-butanone-1, diethylthioxanthone, isopropylthioxanthone, 2,4,6-trimethylbenzoyldiphenylphosphine oxide, bis(2,6-dimethoxybenzoyl)-2,4,4-trimethylpentylphosphine oxide, bis(2,4,6-trimethylbenzoyl)-phenylphosphine oxide, 2-hydroxy-1-{4-[4-(2-hydroxy-2-methylpropionyl)benzyl]phenyl}-2-methylpropan-1-one, methylbenzoylformate, 1-[4-(phenylthio)-2-(O-benzoyloxime)]-1,2-octanedione, 1-[9-ethyl-6-(2-methylbenzoyl)-9H-carbazol-3-yl]-1-(O-acetyloxime)ethanone, and the like.
Any one of these photopolymerization initiators may be used alone, or any combination of two or more of them may be used at any ratio.
The content of the photopolymerization initiator in the polymerizable composition of the present invention is usually 0.01 parts by mass or more, preferably 0.02 parts by mass or more, and still more preferably 0.05 parts by mass or more based on 100 parts by mass of the total of all radical polymerizable compounds in the polymerizable composition. The upper limit of the content is usually 10 parts by mass or less, preferably 5 parts by mass or less, and still more preferably 3 parts by mass or less. When the content of the photopolymerization initiator added is excessively large, the polymerization proceeds abruptly. In this case, not only does the birefringence of the cured product increase, but also its hue may deteriorate. When the content is excessively small, the polymerizable composition may not be polymerized sufficiently.

<Thermal polymerization initiator>

Any known thermal radical polymerization initiator can be used as the thermal polymerization initiator that assists the polymerization of the polymerizable composition of the present invention. Examples of such a thermal radical polymerization initiator include organic peroxides and azo compounds. Among these, organic peroxides are preferred because air bubbles are unlikely to be formed in the polymer obtained through the polymerization reaction.

Specific examples of the organic peroxides include: ketone peroxides such as methyl ethyl ketone peroxide; peroxy ketals such as 1,1-di(t-hexylperoxy)-3,3,5-trimethylcyclohexane, 1,1-di(t-hexylperoxy)cyclohexane, and 1,1-di(t-butylperoxy)cyclohexane; hydroperoxides such as 1,1,3,3-tetramethylbutyl hydroperoxide, cumene hydroperoxide, and p-menthane hydroperoxide; dialkyl peroxides such as dicumyl peroxide and di-t-butyl peroxide; diacyl peroxides such as dilauroyl peroxide and dibenzoyl peroxide; peroxydicarbonates such as di(4-t-butylcyclohexyl)peroxydicarbonate and di(2-ethylhexyl)peroxydicarbonate; and, peroxyesters such as t-butylperoxy-2-ethylhexanoate, t-hexylperoxyisopropyl monocarbonate, t-butyl peroxybenzoate, and 1,1,3,3-tetramethylbutyl-2-ethylhexanoate.

Specific examples of the azo compounds include 2,2'-azobisisobutyronitrile, 2,2'-azobis(2-methylbutyronitrile), 2,2'-azobis(2,4-dimethylvaleronitrile), 1,1'-azobis-1-cyclohexanecarbonitrile, dimethyl-2,2'-azobisisobutyrate, 4,4'-azo-bis-4-cyanovaleric acid, and 2,2'-azobis-(2-amidinopropane)dihydrochloride.

Any one of these thermal polymerization initiators may be used alone, or any combination of two or more may be used at any ratio.

The content of the thermal polymerization initiator in the polymerizable composition of the present invention is usually 0.1 parts by mass or more, preferably 0.5 parts by mass or more, and more preferably 0.8 parts by mass or more based on 100 parts by mass of the total of all the radical polymerizable compounds in the polymerizable composition. The upper limit of the content is usually 10 parts by mass or less, preferably 5 parts by mass or less, and more preferably 2 parts by mass or less. When the content of the thermal polymerization initiator is excessively large, the polymerization proceeds abruptly. In this case, not only is the optical uniformity of the polymer to be obtained impaired, but also its hue may deteriorate. When the content is excessively small, the thermal polymerization may not proceed sufficiently.

When the photopolymerization initiator is used in combination with the thermal polymerization initiator, the mass ratio is usually "100:1" to "1:100" ("the photopolymerization initiator:the thermal polymerization initiator", the same applies to this paragraph) and preferably "10:1" to "1:10." When the amount of the thermal polymerization initiator is excessively small, the polymerization may be insufficient. When the amount is excessively large, coloration may occur.

2-1-2. Redox-based polymerization initiator

The redox-based polymerization initiator is a radical initiator that utilizes a redox reaction of a peroxide and a reducing agent used in combination, can generate radicals even at low temperature, and is usually used for emulsion polymerization etc.

Specific examples of the redox-based polymerization initiator include: combinations of dibenzoyl peroxide serving as a peroxide and an aromatic tertiary amine serving as a reducing agent such as N,N-dimethylaniline, N,N-dimethyl-p-toluidine, and N,N-bis(2-hydroxypropyl)-p-toluidine; combinations of a hydroperoxide serving as a peroxide and a metallic soap serving as a reducing agent; combinations of a hydroperoxide serving as a peroxide and thioureas serving as a reducing agent; and the like.

As for a water-soluble redox-based polymerization initiator, a peroxide such as a persulfate, hydrogen peroxide, or a hydroperoxide is used in combination with a water-soluble inorganic reducing agent (such as $Fe^{2+}$ or $NaHSO_3$) or a water-soluble organic reducing agent (such as an alcohol or a polyamine).

A preferred range of the content of the redox-based polymerization initiator in the polymerizable composition of the present invention is the same as that for the thermal polymerization initiator.

2-1-3. Anionic polymerization initiator

Examples of the anionic polymerization initiator used for the polymerizable composition of the present invention include alkali metals, n-butyllithium, sodium amide, sodium naphthalenide, a Grignard reagent, lithium alkoxides, and alkali metal benzophenone ketyls. Any of these may be used alone, or any combination of two or more of them may be used at any ratio.

2-1-4. Cationic polymerization initiator

Examples of the cationic polymerization initiator used for the polymerizable composition of the present invention include: Bronsted acids such as perchloric acid, sulfuric acid, and trichloroacetic acid; Lewis acids such as boron trifluoride, aluminum trichloride, aluminum tribromide, and tin tetrachloride; iodine; chlorotriphenylmethane; and the like.

Any one of these may be used alone, or any combination of two or more of them may be used at any ratio.

The content of the anionic polymerization initiator or the cationic polymerization initiator in the polymerizable composition of the present invention is usually 0.001 parts by mass or more, preferably 0.005 parts by mass or more, and still more preferably 0.01 parts by mass or more based on 100 parts by mass of the total of all anionically or cationically polymerizable compounds in the polymerizable composition. The upper limit of the amount is usually 5 parts by mass or less, preferably 1 part by mass or less, and more preferably 0.5 parts by mass or less. When the content of the anionic or cationic polymerization initiator is less than 0.001 parts by mass, the reaction is not sufficient. When the content exceeds 5 parts by mass, it is difficult to achieve a sufficient pot life and a sufficient polymerization rate simultaneously.

2-1-5. Cationic photopolymerization initiator

The cationic photopolymerization initiator in the present invention is an initiator that generates cationic species under light. There is no particular limitation on the cationic photopolymerization initiator so long as it is a compound that generates cationic species upon irradiation with light. Onium salts are usually well known and used. Examples of the onium salts include diazonium salts of Lewis acids, iodonium salts of Lewis acids, sulfonium salts of Lewis acids, and the like. Specific examples include a phenyldiazonium salt of boron tetrafluoride, a diphenyl iodonium salt of phosphorus hexafluoride, a diphenyl iodonium salt of antimony hexafluoride, a tri-4-methylphenylsulfonium salt of arsenic hexafluoride, a tri-4-methylphenylsulfonium salt of antimony tetrafluoride, and the like. Preferably, aromatic sulfonium salts are used.

Specific examples of the cationic photopolymerization initiator include S,S,S',S'-tetraphenyl-S,S'-(4,4'-thiodiphenyl)disulfonium bishexafluorophosphate, diphenyl-4-phenylthiophenyl sulfonium hexafluorophosphate, diphenyl-4-phenylthiophenyl sulfonium hexafluoroantimonate, and the like. Specific examples of the initiator product include product name: UVI-6992 manufactured by The Dow Chemical Company, product name: CPI-100P manufactured by San-Apro Ltd., product name: CPI-101A manufactured by San-Apro Ltd., product name: CPI-200K manufactured by San-Apro Ltd., product name: Omnicat 270 manufactured by IGM Resins Ltd, and the like.

Any one of these cationic photopolymerization initiators may be used alone, or any combination of two or more of them may be used at any ratio.

The content of the cationic photopolymerization initiator in the polymerizable composition of the present invention is preferably from 0.02 parts by mass to 20 parts by mass inclusive and more preferably from 0.1 parts by mass to 10 parts by mass inclusive based on 100 parts by mass of the total of all cationically photopolymerizable compounds in the polymerizable composition. When the content of the cationic photopolymerization initiator is lower than 0.02 parts by mass, the reaction is insufficient. When the content exceeds 20 parts by mass, it is difficult to achieve a sufficient pot life and a sufficient polymerization rate simultaneously.

The cationic photopolymerization initiator may be used in combination with the cationic polymerization initiator described above. In this case, the cationic polymerization initiator is used in an amount of usually 0.1 to 10 parts by mass and preferably 1 to 5 parts by mass based on 100 parts by mass of the cationically polymerizable compounds in the polymerizable composition. When the amount of the cationic polymerization initiator used is excessively small, a reduction in the polymerization rate occurs. When the amount is excessively large, the physical properties of the polymer to be obtained may deteriorate.

The cationic photopolymerization initiator may be used in combination with a cationic photopolymerization sensitizer. The cationic photopolymerization sensitizer is a formulation used when light emitted from a light source used for cationic photopolymerization does not match well the absorption wavelength of the cationic photopolymerization initiator. The cationic photopolymerization sensitizer is used in combination with the cationic photopolymerization initiator to transmit the energy of the irradiation light efficiently to the cationic photopolymerization initiator. Known examples of the cationic photopolymerization sensitizer include phenol-based compounds such as methoxyphenol (JP5-230189A), thioxanthone compounds (JP2000-204284A), and dialkoxyanthracene compounds (JP2000-119306A).

The cationic photopolymerization sensitizer is used in an amount of usually 0.2 to 5 parts by mass and preferably 0.5 to 1 part by mass based on 1 part by mass of the cationic photopolymerization initiator. When the amount of the cationic photopolymerization sensitizer is excessively small, the sensitizing effect may not be easily obtained. When the amount is excessively large, the physical properties of the polymer may deteriorate.

2-2. Polymerizable compounds

The polymerizable composition of the present invention may contain only one compound of the present invention represented by formula (1) as a polymerizable compound or any combination of two or more compounds represented by formula (1) as polymerizable compounds at any ratio.

The polymerizable composition of the present invention may contain an additional polymerizable compound other than the compound (1).

The content of the compound (1) in the polymerizable composition of the present invention is preferably 1% by mass or more and 99% by mass or less, and more preferably 5% by mass or more and 95% by mass or less, based on 100% by mass of the total solid content of the polymerizable composition of the present invention. When the content of the compound (1) is less than 1% by mass, the effect obtained by using the compound (1) is not fully exhibited. On the other hand, when the content exceeds 99% by mass, the curability tends to decrease.

Examples of the additional polymerizable compound other than the compound (1) include cationically polymerizable monomers, anionically polymerizable monomers, and radically polymerizable monomers. Any one of these polymerizable compounds may be used alone, or any combination of two or more of them may be used at any ratio. A polymerizable compound having two or more polymerizable functional groups per molecule (which may be referred to as a polyfunctional monomer) may also be used. When the polyfunctional monomer is used, a crosslinked structure is formed in the polymer, so that thermal stability, weather resistance, solvent resistance, and the like can be improved.

When the polymerizable composition of the present invention contains the additional polymerizable compound other than the compound (1), the content of the additional polymerizable compound is preferably 0.1% by mass or more and 10% by mass or less, and more preferably 0.3% by mass or more and 5% by mass or less, based on 100% by mass of the total solid content of the polymerizable composition of the present invention. When the content of the additional polymerizable compound is less than 0.1% by mass, the effect of imparting characteristics by the addition of the additional polymerizable compound is not sufficient. On the other hand, when the content exceeds 5% by mass, problems such as a reduction in optical characteristics and a reduction in strength tend to occur.

<Cationically polymerizable monomer>

Examples of the cationically polymerizable monomer include compounds having an oxirane ring, styrene and derivatives thereof, vinylnaphthalene and derivatives thereof, vinyl ethers, N-vinyl compounds, and compounds having an oxetane ring.

In particular, a compound having at least an oxirane ring is preferably used, and a combination of a compound having an oxetane ring and a compound having an oxirane ring is used more preferably.

Examples of the compound having an oxirane ring include polymerizable monomers having two or more oxirane rings per molecule.

Examples of such polymerizable monomers include alicyclic polyepoxies, polyglycidyl esters of polybasic acids, polyglycidyl ethers of polyhydric alcohols, polyglycidyl ethers of polyoxyalkylene glycols, polyglycidyl ethers of aromatic polyols, hydrogenated compounds of polyglycidyl ethers of aromatic polyols, urethane polyepoxy compounds, epoxidized polybutadienes, and the like.

Examples of the styrene and derivatives thereof include styrene, p-methylstyrene, p-methoxystyrene, β-methylstyrene, p-methyl-β-methylstyrene, α-methylstyrene, p-methoxy-β-methylstyrene, divinylbenzene, and the like.

Examples of the vinylnaphthalene and derivatives thereof include 1-vinylnaphthalene, α-methyl-1-vinylnaphthalene, β-methyl-1-vinylnaphthalene, 4-methyl-1-vinylnaphthalene, 4-methoxy-1-vinylnaphthalene, and the like.

Examples of the vinyl ethers include isobutyl ether, ethyl vinyl ether, phenyl vinyl ether, p-methylphenylvinyl ether, p-methoxyphenylvinyl ether, and the like.

Examples of the N-vinyl compounds include N-vinylcarbazole, N-vinylpyrrolidone, N-vinylindole, N-vinylpyrrole, N-vinylphenothiazine, and the like.

Examples of the compounds having an oxetane ring include various known oxetane compounds described in JP2001-220526A, JP2001-310937A, and the like.

Any one of these cationically polymerizable monomers may be used alone, or any combination of two or more of them may be used at any ratio.

<Anionically polymerizable monomer>

Examples of the anionically polymerizable monomer include hydrocarbon monomers, polar monomers, and the like.

Examples of the hydrocarbon monomer include styrene, α-methylstyrene, butadiene, isoprene, vinylpyridine, vinylanthracene, derivatives thereof, and the like.

Examples of the polar monomer include: methacrylic acid esters (such as methyl methacrylate, ethyl methacrylate, and isopropyl methacrylate); acrylic acid esters (such as methyl acrylate and ethyl acrylate); vinyl ketones (such as methyl vinyl ketone, isopropyl vinyl ketone, cyclohexyl vinyl ketone, and phenyl vinyl ketone); isopropenyl ketones (such as methyl isopropenyl ketone and phenyl isopropenyl ketone); and other polar monomers (such as acrylonitrile, acrylamide, nitroethylene, methylene malonate, cyanoacrylates, and vinylidene cyanide), and the like.

Any one of these anionically polymerizable monomers may be used alone, or any combination of two or more of them may be used at any ratio.

<Radically polymerizable monomer>

The radically polymerizable monomer is a compound having at least one ethylenically unsaturated double bond per molecule, and examples thereof include (meth) acrylic acid esters, (meth)acrylamides, vinyl esters, styrenes, and the like.

Examples of the (meth) acrylic acid esters include methyl (meth)acrylate, ethyl (meth)acrylate, (n- or i-)propyl (meth)acrylate, (n-, i-, sec-, or t-)butyl (meth)acrylate, amyl (meth)acrylate, adamantyl (meth)acrylate, chloroethyl (meth) acrylate, 2-hydroxyethyl (meth)acrylate, 2-hydroxypropyl (meth)acrylate, 2-hydroxypentyl (meth)acrylate, cyclohexyl (meth)acrylate, allyl (meth)acrylate, trimethylolpropane mono(meth)acrylate, pentaerythritol mono(meth)acrylate, benzyl (meth)acrylate, methoxybenzyl (meth)acrylate, chlorobenzyl (meth)acrylate, hydroxybenzyl (meth)acrylate, hydroxyphenethyl (meth)acrylate, dihydroxyphenethyl (meth)acrylate, furfuryl (meth)acrylate, tetrahydrofurfuryl (meth)acrylate, phenyl (meth)acrylate, hydroxyphenyl (meth)acrylate, chlorophenyl (meth)acrylate, sulfamoylphenyl (meth)acrylate, 2-phenoxyethyl (meth)acrylate, 2-(hydroxyphenylcarbonyloxy)ethyl (meth)acrylate, phenol EO-modified (meth)acrylate, phenylphenol EO-modified (meth)acrylate, paracumylphenol EO-modified (meth)acrylate, nonylphenol EO-modified (meth)acrylate, N-acryloyloxyethylhexahydrophthalimide, bisphenol F EO-modified diacrylate, bisphenol A EO-modified diacrylate, dibromophenyl (meth)acrylate, tribromophenyl (meth)acrylate, dicyclopentenyloxyethyl (meth)acrylate, dicyclopentanyl acrylate, tricyclodecanedimethylol di(meth)acrylate, bisphenoxyethanolfluorene di(meth)acrylate, trimethylolpropane tri(meth)acrylate, pentaerythritol tri(meth)acrylate, pentaerythritol tetra(meth)acrylate, dipentaerythritol penta(meth)acrylate, dipentaerythritol hexa(meth)acrylate, and the like. Here, "EO" means "ethylene oxide."

Examples of the (meth)acrylamides include (meth)acrylamide, N-methyl(meth)acrylamide, N-ethyl(meth)acrylamide, N-propyl(meth)acrylamide, N-butyl(meth)acrylamide, N-benzyl(meth)acrylamide, N-hydroxyethyl(meth)acrylamide, N-phenyl(meth)acrylamide, N-tolyl(meth)acrylamide, N-(hydroxyphenyl) (meth)acrylamide, N-(sulfamoylphenyl) (meth)acrylamide, N-(phenylsulfonyl) (meth)acrylamide, N-(tolylsulfonyl) (meth)acrylamide, N,N-dimethyl(meth)acrylamide, N-methyl-N-phenyl(meth)acrylamide, N-hydroxyethyl-N-methyl(meth)acrylamide, and the like.

Examples of the vinyl ester include vinyl acetate, vinyl butyrate, vinyl benzoate, benzoic acid vinyl ester, vinyl t-butyl benzoate, vinyl chlorobenzoate, vinyl 4-ethoxybenzoate, vinyl 4-ethylbenzoate, vinyl 4-methylbenzoate, vinyl 3-methylbenzoate, vinyl 2-methylbenzoate, vinyl 4-phenylbenzoate, vinyl pivalate, and the like.

Examples of the styrenes include styrene, p-acetylstyrene, p-benzoylstyrene, 2-butoxymethylstyrene, 4-butylstyrene, 4-sec-butylstyrene, 4-tert-butylstyrene, 2-chlorostyrene, 3-chlorostyrene, 4-chlorostyrene, dichlorostyrene, 2,4-diisopropylstyrene, dimethylstyrene, p-ethoxystyrene, 2-ethylstyrene, 2-methoxystyrene, 4-methoxystyrene, 2-methylstyrene, 3-methylstyrene, 4-methylstyrene, p-methylstyrene, p-phenoxystyrene, p-phenylstyrene, divinylbenzene, and the like.

Any one of these radically polymerizable monomers may be used alone, or any combination of two or more of them may be used at any ratio.

Any of the above-exemplified cationically polymerizable monomers, anionically polymerizable monomers, and radically polymerizable monomers may be used, or two or more of them may be used in combination.

For example, for high refractive index optical lenses and holographic holographic recording media, it is preferable to use a radically polymerizable monomer as the additional polymerizable compound used in combination with the compound (1) because a reaction for forming a resin matrix is unlikely to be inhibited.

2-3. Additional additive component

An additional component may be added to the polymerizable composition of the present invention so long as the effects of the present invention are not impaired.

Examples of the additional component include various additives such as a solvent, an antioxidant, a plasticizer, an ultraviolet absorber, a sensitizer, a chain transfer agent, an antifoaming agent, a polymerization inhibitor, any filler formed of an organic or inorganic material, a dispersing agent, a pigment, and a wavelength conversion material such as a phosphor.

The polymerizable composition of the present invention may contain a solvent for the purpose of controlling the viscosity.

The solvent is selected according to the physical properties of the polymerizable composition, and specific examples of the solvent include organic solvents such as: alcohols such as ethanol, propanol, isopropanol, ethylene glycol, and propylene glycol; aliphatic hydrocarbons such as hexane, pentane, and heptane; alicyclic hydrocarbon such as cyclopentane and cyclohexane; aromatic hydrocarbons such as toluene and xylene; halogenated hydrocarbons such as methylene chloride and chloroform; chain ethers such as dimethyl ether and diethyl ether; cyclic ethers such as dioxane and tetrahydrofuran; esters such as methyl acetate, ethyl acetate, butyl acetate, ethyl lactate, and ethyl butyrate; ketones such as acetone, ethyl methyl ketone, methyl isobutyl ketone, and cyclohexanone; cellosolves such as methyl cellosolve, ethyl cellosolve, and butyl cellosolve; carbitols such as methyl carbitol, ethyl carbitol, and butyl carbitol; propylene glycol monoalkyl ethers such as propylene glycol monomethyl ether, propylene glycol monoethyl ether, and propylene glycol

mono-n-butyl ether; glycol ether esters such as ethylene glycol monomethyl ether acetate and propylene glycol monomethyl ether acetate; amides such as N,N-dimethylformamide, and N,N-dimethylacetamide; sulfoxides such as dimethyl sulfoxide; nitriles such as acetonitrile and benzonitrile; and N-methylpyrrolidone.

Any of these solvents may be used alone, or a solvent mixture thereof may be used. Water may be used for some polymerization methods (such as emulsion polymerization and suspension polymerization).

There is no particular limitation on the amount of the solvent (or a dispersion medium) used. The solvent may be used such that the polymerizable composition has a viscosity suitable for the polymerization method, a processing method, or its intended application.

In the present invention, it is preferable that an antioxidant or light stabilizer used as an additive is added to the polymerizable composition in order for the polymer to be obtained to have good resistance to thermal yellowing or good weatherability.

Specific examples of the antioxidant include: phenol-based antioxidants such as 2,6-di-t-butylphenol, 2,6-di-t-butyl-p-cresol, n-octadecyl-3-(3',5'-di-t-butyl-4'-hydroxyphenyl)propionate, tetrakis-[methylene-3-(3',5'-dit-butyl-4'-hydroxyphenyl)propionate]methane, triethylene glycol bis[3-(3-t-butyl-5-methyl-4-hydroxyphenyl)propionate], 1,6-hexanediol bis[3-(3,5-di-t-butyl-4-hydroxyphenyl)propionate], and the like; and phosphorus-based antioxidants such as triphenyl phosphite, trisisodecyl phosphite, isodecyldiphenyl phosphite, 2-ethylhexyldiphenyl phosphite, tetra(C12-C15alkyl)-4,4'-isopropylidenediphenyl diphosphite, tris(nonylphenyl)phosphite, tristridecyl phosphite, 2,4,8,10-tetra-tert-butyl-6-[(2-ethylhexan-1-yl)oxy]-12H-dibenzo[d,g][ 1,3,2]dioxaphosphosine, 3,9-bis(2,6-di-tert-butyl-4-methylphenoxy)-2,4,8,10-tetraoxa-3,9-diphosphaspiro[5.5]undecane, 3,9-dioctadecan-1-yl-2,4,8,10-tetraoxa-3,9-diphosphaspiro[5.5]undecane, tris(2,4-di-t-butylphenyl)phosphite, and the like. One of these may be used alone, or a combination of two or more may be used.

Preferably, the antioxidant used is a combination of a phenol-based antioxidant and a phosphorus-based antioxidant. Preferred examples of the combination of the phenol-based antioxidant and the phosphorus-based antioxidant include a combination of tris(2,4-di-t-butylphenyl) phosphite used as the phosphorus-based antioxidant and at least one phenol-based antioxidant selected from tetrakis-[methylene-3-(3',5'-di-t-butyl-4'-hydroxyphenyl)propionate] methane and n-octadecyl-3-(3',5'-di-t-butyl-4'-hydroxyphenyl)propionate.

From the viewpoint of allowing the polymer to be obtained to have good resistance to thermal yellowing, the amount of the antioxidant added to the polymerizable composition of the present invention is preferably 0.01 to 5 parts by mass, more preferably 0.05 to 3 parts by mass, and still more preferably 0.1 to 2 parts by mass based on 100 parts by mass of the total amount of the polymerizable composition.

The light stabilizer used is preferably a hindered amine-based light stabilizer (HALS). Specific examples of the HALS include 2,2,6,6-tetramethyl-4-piperidinyl stearate, 2,2,6,6-tetramethyl-4-piperidyl methacrylate, 1,2,2,6,6-pentamethyl-4-piperidyl methacrylate, bis(2,2,6,6-tetramethyl-1-undecyloxypiperidin-4-yl)carbonate, sebacic acid bis(2,2,6,6-tetramethyl-4-piperidyl) ester, sebacic acid bis(1,2,2,6,6-pentamethyl-4-piperidyl) ester, ADEKA STAB LA-68 (manufactured by ADEKA Corporation), ADEKA STAB LA-63P (manufactured by ADEKA Corporation), butane-1,2,3,4-tetracarboxylic acid tetrakis(1,2,2,6,6-pentamethyl-4-piperidinyl) ester, 1,2,3,4-butanetetracarboxylic acid tetrakis(2,2,6,6-tetramethyl-4-piperidinyl) ester, and TINUVIN 111FDL, TINUVIN 123, TINUVIN 144, TINUVIN 152, TINUVIN 249, TINUVIN 292, TINUVIN 5100 (which are manufactured by BASF), and the like. One of them may be used alone, or two or more of them may be used in combination.

The mixing amount of the light stabilizer in the polymerizable composition of the present invention is preferably 0.01 to 5 parts by mass, more preferably 0.05 to 3 parts by mass, and still more preferably 0.1 to 2 parts by mass based on 100 parts by mass of the total mass of the polymerizable composition in order for a polymer to be obtained to have good resistance to thermal yellowing and good weather resistance.

One of the above antioxidants and one of the above light stabilizers may be used. A combination of two or more of the antioxidants may be used, and a combination of two or more of the light stabilizers may be used.

2-4. Method for producing polymerizable composition

The polymerizable composition of the present invention may be produced by mixing the components, or may be produced by mixing components other than the polymerization initiator in advance and adding the polymerization initiator immediately before the polymerization reaction.

3. Method for polymerizing polymerizable composition of invention

There is no particular limitation on the method for polymerizing the polymerizable composition of the present invention. Examples of the method include a polymerization method using active energy ray irradiation and a thermal polymerization method.

3-1. Polymerization initiation method (active energy rays)

When the polymerizable composition of the present invention is subjected to photo radical polymerization, the polymerizable composition is irradiated with active energy rays.

Preferably, the active energy rays used are an electron beam or light in the ultraviolet to infrared wavelength range. An extra-high pressure mercury light source or a metal halide light source can be used when the active energy rays are ultraviolet rays, and a metal halide light source or a halogen light source can be used when the active energy rays are visible light. A halogen light source can be used when the active energy rays are infrared light. In addition, light sources such as lasers and LEDs can also be used.

The dose of the active energy rays is appropriately set according to the type of light source, the thickness of a coating, etc. and is appropriately set such that the total reaction rate of the polymerizable groups in the compound (1) and other polymerizable compounds is preferably 80% or more and more preferably 90% or more. The reaction rate is computed from changes in the intensities of the absorption peaks attributed to the polymerizable groups in an infrared absorption spectrum before and after the reaction.

After the polymerization under irradiation with the active energy rays, heat treatment or annealing treatment may be optionally performed to allow the polymerization to further proceed. The heating temperature in this case is preferably in the range of 80 to 200°C. The heating time is preferably in the range of 10 to 60 minutes.

3-2. Polymerization initiation method (heating)

When the polymerizable composition of the present invention is subjected to heat treatment for polymerization, the heating temperature is preferably in the range of 80 to 200°C and more preferably in the range of 100 to 150°C. When the heating temperature is lower than 80°C, it is necessary to increase the heating time, and cost efficiency tends to decrease. When the heating temperature is higher than 200°C, the cost of energy is high, and the heating time and cooling time are long, so that cost efficiency tends to decrease.

4. Polymer

The polymer of the present invention produced by polymerizing the polymerizable composition of the present invention will be described.

4-1. Refractive index

Generally, the polymerization reaction causes the overall density to increase. Therefore, the refractive index of its precursor, the monomer before the polymerization. When a monomer having a high refractive index is used and its polymerization reaction is allowed to proceed sufficiently, the polymer obtained can have a high refractive index. It is therefore thought to be important to increase the refractive index of the polymer by appropriately designing the molecular structure of the monomer.

The value of the refractive index is high when it is evaluated using irradiation light with a short wavelength. A sample that exhibits a relatively high refractive index at a short wavelength also exhibits a relatively large refractive index at a long wavelength, and this relation is not reversed. Therefore, by evaluating the refractive indexes of materials at a certain wavelength for comparison, the magnitudes of the intrinsic refractive indexes of the materials can be compared. In the present invention, the value at an irradiation wavelength of 587 nm is used as a reference.

The refractive indexes of the compound of the present invention and the polymer of the present invention is preferably 1.55 or more, more preferably 1.60 or more, particularly preferably 1.63 or more, and most preferably 1.65 or more. There is no particular limitation on the upper limit of the refractive indexes of the compound of the present invention and the polymer of the present invention, but the refractive index is usually 2.0 or less.

The compound of the present invention and the polymer of the present invention can be used as an optical material for lenses and the like. In this case, when the refractive index of the compound or the polymer is lower than 1.55, central portions of the lenses and the like are thick. This is not preferred because the lightweight of the plastic, which is one of its features, is not utilized. To develop precision optical members such as lenses, it is also important to use a combination of optical materials having a plurality of refractive indexes to thereby obtain optical characteristics suitable for the members. From this point of view, it can be said that a monomer and a polymer having a refractive index of more than 1.63 are a material particularly useful for optical components.

When the compound of the present invention and the polymer of the present invention are used as recording layer materials of holographic recording media, the refractive indexes of the compound of the present invention and the polymer of the present invention are usually in the range of 1.65 or more and 1.78 or less, and preferably 1.77 or less. When the refractive index is less than 1.65, the diffraction efficiency is low, and multiplicity is insufficient. When the refractive

index is more than 1.78, the difference in the refractive index between the polymer and the matrix resin is excessively large. In this case, strong scattering occurs, and therefore transmittance decreases, so that larger energy is required for recording and reproduction.

4-2. Glass transition temperature

The glass transition temperature of the polymer of the present invention is preferably 90°C or higher, more preferably 100°C or higher, still more preferably 110°C or higher, and particularly preferably 120°C or higher and is preferably 250°C or lower, more preferably 220°C or lower, and still more preferably 200°C or lower. When the glass transition temperature is below the above range, the optical properties may deviate from the design values in the use environment, and the heat resistance may not satisfy that required for practical use. When the glass transition temperature is above the above range, the processability of the polymer is low. In this case, a molded product having good appearance and high dimensional accuracy may not be obtained, and the polymer becomes brittle. Therefore, the mechanical strength decreases, and the handleability of the molded product may deteriorate.

5. Optical material and optical component

The compound of the present invention, the polymerizable composition of the present invention, and the polymer of the present invention have properties such as a high refractive index, easy processability, and excellent chemical stability and can therefore be applied to various optical materials and optical components.

Examples of the optical material include overcoats for optical use, hard coat agents, adhesives for optical members, resins for optical fibers, acrylic-based resin reformers, and the like.

Examples of the optical component include lenses, filters, diffraction gratings, prisms, optical guides, glass covers for display devices, photosensors, photoswitches, LEDs, light-emitting elements, optical waveguides, optical splitters, optical fiber adhesives, substrates for display elements, substrates for color filters, substrates for touch panels, polarizing plates, display backlights, light guide plates, antireflective films, viewing angle widening films, optical recording, optical fabrication, optical relief printing, and the like.

Moreover, the polymer of the present invention can be used for a layer in any of the above components. Examples of such a layer include a display protective films, and the like.

In particular, the compound and the polymer of the present invention are preferably applicable to plastic lenses because of the high-refractive index characteristics of the polymer. Examples of the lenses include imaging lenses of cameras (vehicle-mounted cameras, digital cameras, PC cameras, mobile phone camaras, monitoring cameras, etc.), eyeglass lenses, light beam condensing lenses, and beam diverging lenses.

Lenses formed using the compound and the polymer of the present invention may be optionally subjected to physical or chemical treatment such as surface polishing, antistatic treatment, hard coating treatment, antireflective coating treatment, or staining treatment for the purpose of preventing reflection, imparting high hardness, improving wear resistance, imparting chemical resistance, imparting anti-fogging properties, imparting fashionability, etc.

6. Holographic recording medium

The polymerizable composition of the present invention can be preferably used for a recording layer of a holographic recording medium. In this case, the polymerizable composition of the present invention is preferably a photoreactive composition containing, in addition to the compound of the present invention, a matrix resin, a photopolymerization initiator, a radical scavenger and other additives. The details of these materials when they are used as materials for a holographic recording medium will be described below.

6-1. About matrix resin

Preferably, the polymerizable composition of the present invention contains a matrix resin. In particular, the matrix resin forming a recording layer of a holographic recording medium is an organic material that is not largely modified chemically and physically under irradiation with light and is formed mainly of a polymer of an organic compound.

The matrix resin forms the polymerizable composition of the present invention together with the above-described polymerizable compound and a photopolymerization initiator described later, etc. The matrix resin is therefore strongly required to have good compatibility with the polymerizable compound and the photopolymerization initiator, etc. When the compatibility between the matrix resin and the other components is low, interfaces are formed between the materials, and reflection and refraction of light occurs at the interfaces. This causes leakage of light to unintended portions. Therefore, the interference fringes are distorted or broken, and recording may be performed in unwanted portions, so that deterioration in information may occur. The compatibility between the matrix resin and the other components can be evaluated based on, for example, light scattering intensity obtained by a detector disposed in a direction different from the direction of light passing through a sample, as described in, for example, Japanese Patent No. 3737306.

The matrix resin in the polymerizable composition of the present invention may be a resin that includes a plurality of materials soluble in a solvent in the polymerizable composition and is to be three-dimensionally crosslinked after shaped into a usable form. Examples of such a resin include thermoplastic resins, thermosetting resins, and photocurable resins described below.

The three-dimensionally crosslinked resin is insoluble in a solvent and is a reaction cured product of a polymerizable compound that is liquid at room temperature and a compound having reaction activity with the polymerizable compound. The three-dimensionally crosslinked resin serves as physical obstacles and therefore reduces a volume change during recording. Specifically, in the recording layer after recording, bright portions are expanded, and dark portions are shrunk, so that irregularities tend to be formed on the surface of the holographic recording medium. To reduce the volume change, it is more preferable that a polymerizable composition containing a three-dimensionally crosslinked resin matrix is used for the recording layer.

In particular, from the viewpoint of adhesion to a support, the matrix resin is preferably a thermosetting resin. Resin materials that can be used as the matrix resin will be described in detail.

6-1-1. Thermoplastic resin

Specific examples of the thermoplastic resin material include chlorinated polyethylene, polymethyl methacrylate resins (PMMA), copolymers of methyl methacrylate with other alkyl acrylates, copolymers of vinyl chloride with acrylonitrile, polyvinyl acetate resins (PVAC), polyvinyl alcohol, polyvinyl formal, polyvinylpyrrolidone, cellulose resins such as ethylcellulose and nitrocellulose, polystyrene resins, and polycarbonate resins. One of these resins may be used alone, or a mixture of two or more may be used.

There is no particular limitation on the solvent for these thermoplastic resins so long as it can dissolve these resins. Examples of such a solvent include: ketones such as acetone and methyl ethyl ketone; esters such as butyl acetate and propylene glycol methyl ether acetate; aromatic hydrocarbons such as toluene and xylene; ethers such as tetrahydrofuran and 1,2-dimethoxyethane; and amides such as N,N-dimethylacetamide and N-methylpyrrolidone. Only one of these solvents may be used alone, or a combination of two or more may be used.

6-1-2. Thermosetting resin

When the matrix resin used is a thermosetting resin, its curing temperature varies largely depending on the type of crosslinking agent and the type of catalyst.

Representative examples of the combination of functional groups that allows the matrix resin to cure at room temperature include a combination of an epoxy and an amine, a combination of an epoxy and a thiol, and a combination of an isocyanate and an amine. Representative example of the combination when a catalyst is used include a combination of an epoxy and a phenol, a combination of an epoxy and an acid anhydride, and a combination of an isocyanate and a polyol.

The former is simple because the reaction starts immediately after mixing. However, when the matrix resin is formed into, for example, a holographic recording medium, the matrix resin is cured while shaped into the holographic recording medium, and it is difficult to control the shape because there is only a limited time available for the formation of the holographic recording medium. In the latter case, the curing temperature and the curing time can be freely selected by appropriately selecting the type of catalyst and the amount of the catalyst used, and this is suitable for the case in which the thermosetting resin is cured while shaped into, for example, a holographic recording medium. Various resin raw materials including low molecular weight to large molecular weight materials are commercially available. Therefore, a suitable raw material can be selected such that the compatibility with a polymerizable reactive compound and a photo initiator and the adhesion to a substrate are maintained.

Each of the raw materials will next be described. For each raw material, one type may be used alone, or two or more types may be used in combination.

<Epoxy>

Examples of the epoxy include: polyglycidyl ether compounds of polyols such as (poly)ethylene glycol, (poly)propylene glycol, (poly)tetramethylene glycol, trimethylolpropane, and glycerin; alicyclic epoxy compounds having a 4 to 7-membered cyclic aliphatic group such as 3,4-epoxycyclohexylmethyl-3,4-epoxycyclohexanecarboxylate and 3,4-epoxy-1-methylcyclohexyl-3,4-epoxy-1-methylhexanecarboxylate; bisphenol A-type epoxy compounds; hydrogenated bisphenol A-type epoxy compounds; bisphenol F-type epoxy compounds; and, phenol and cresol novolac-type epoxy compounds.

Preferably, the epoxy has two or more epoxy groups per molecule, but no particular limitation is imposed on the type of epoxy. When the number of epoxy groups is small, hardness necessary for the matrix may not be obtained. There is no particular limitation on the upper limit of the number of epoxy groups per molecule. The number of epoxy groups is

usually 8 or less and preferably 4 or less. When the number of epoxy groups is excessively large, it takes a long time to consume the epoxy groups, and an excessively long time is necessary to form the matrix resin.

<Amine>

The amine used may contain a primary amino group or a secondary amino group. Examples of such an amine include: aliphatic polyamines such as ethylenediamine and diethylenetriamine and derivatives thereof; alicyclic polyamines such as isophoronediamine, menthanediamine, and N-aminoethylpiperazine and derivatives thereof; aromatic polyamines such as m-xylylenediamine and diaminodiphenylmethane and derivatives thereof; polyamides such as a condensation product of a dicarboxylic acid such as dimer acid and any of the above polyamines; imidazole compounds such as 2-methylimidazole and derivatives thereof; dicyandiamide; and adipic acid dihydrazide.

<Thiol>

Examples of the thiol include: dithiols such as 1,3-butanedithiol, 1,4-butanedithiol, 2,3-butanedithiol, 1,2-benzenedithiol, 1,3-benzenedithiol, 1,4-benzenedithiol, 1,10-decanedithiol, 1,2-ethanedithiol, 1,6-hexanedithiol, and 1,9-nonanedithiol; and thiol compounds including polythiols etc. such as THIOKOL (manufactured by Toray Fine Chemicals Co., Ltd.) and jERCURE QX40 (manufactured by Mitsubishi Chemical Corporation). Among these, commercial fast curable polythiols such as jERCURE QX40 are used preferably.

<Phenol>

Examples of the phenol include phenolic resins such as bisphenol A and novolac type phenolic resins, resol type phenolic resins, and the like.

<Acid anhydride>

Examples of the acid anhydride include: monofunctional acid anhydrides such as phthalic anhydride and tetrahydrophthalic anhydride and derivatives thereof; and bifunctional acid anhydrides such as pyromellitic anhydride and benzophenonetetracarboxylic anhydride and derivatives thereof.

<Amounts of amine, thiol, phenol, and acid anhydride used>

The ratios of the amounts of the amine, thiol, phenol, and acid anhydride used to the number of moles of the epoxy groups are usually 0.1 equivalents or more and preferably 0.7 equivalents or more and is usually 2.0 equivalents or less and preferably 1.5 equivalents or less. When the amounts of the amine, thiol, phenol, and acid anhydride used are excessively small or are excessively large, the number of unreacted functional groups is large, and the storage stability may be impaired.

<Polymerization initiator for thermosetting resin>

An anionic polymerization initiator or a cationic polymerization initiator selected according to the curing temperature and the curing time may be used as a catalyst for curing the thermosetting resin.

The anionic polymerization initiator generates anions under the application of heat or irradiation with light, and examples thereof include amines. Examples of the amines include: amino group-containing compounds such as dimethylbenzylamine, dimethylaminomethylphenol, and 1,8-diazabicyclo[5.4.0]undecene-7 and derivatives thereof; and, imidazole compounds such as imidazole, 2-methylimidazole, and 2-ethyl-4-methylimidazole and derivatives thereof. One or a plurality of them may be used according to the curing temperature and the curing time.

The cationic polymerization initiator generates cations under the application of heat or irradiation with light, and examples thereof include aromatic onium salts. Specific examples include compounds containing an anionic component such as $SbF_6^-$, $BF_4^-$, $AsF_6^-$, $PF_6^-$, $CF_3SO_3^-$, or $B(C_6F_5)_4^-$ and an aromatic cationic component containing an iodine atom, a sulfur atom, a nitrogen atom, a phosphorus atom, etc. In particular, diaryliodonium salts, triarylsulfonium salts, etc. are preferred. One or a plurality of them may be used according to the curing temperature and the curing time.

The amount used of the polymerization initiator for the thermosetting resin relative to the amount of the matrix resin is usually 0.001% mass or more and preferably 0.01% mass or more and is usually 50% by mass or less and preferably 10% by mass or less. When the amount used of the polymerization initiator for the thermosetting resin is excessively small, the concentration of the polymerization initiator for the thermosetting resin is excessively small, so that the polymerization may take an excessively long time. When the amount used of the polymerization initiator for the

thermosetting resin is excessively large, a continuous ring-opening reaction, which is a polymerization reaction, may not occur.

<Isocyanate>

Preferably, the isocyanate includes two or more isocyanate groups per molecule, but no particular limitation is imposed on the type of isocyanate. When the number of isocyanate groups per molecule is small, hardness necessary for the matrix resin may not be obtained. There is no particular limitation on the upper limit of the number of isocyanate groups per molecule, but the number of isocyanate groups is usually 8 or less and preferably 4 or less. When the number of isocyanate groups per molecule is excessively large, it takes a long time to consume the isocyanate groups, and an excessively long time may be necessary to form the matrix resin. There is no particular limitation on the upper limit of the number of isocyanate groups per molecule, but the number of isocyanate groups is usually about 20 or less.
Examples of the isocyanate include: aliphatic isocyanates such as hexamethylene diisocyanate, lysine methyl ester diisocyanate, and 2,4,4-trimethylhexamethylene diisocyanate; alicyclic isocyanates such as isophorone diisocyanate and 4,4'-methylenebis(cyclohexyl isocyanate); aromatic isocyanates such as tolylene diisocyanate, 4,4'-diphenylmethane diisocyanate, xylylene diisocyanate, and naphthalene-1,5'-diisocyanate; and multimers thereof. In particular, trimers to heptamers thereof are preferred.
Other examples include: reaction products of any of the above isocyanates with water and polyhydric alcohols such as trimethylolethane and trimethylolpropane; and multimers of hexamethylene diisocyanate and derivatives thereof.
As for the molecular weight of the isocyanate, its number average molecular weight is preferably from 100 to 50000 inclusive, more preferably from 150 to 10000 inclusive, and still more preferably from 150 to 5000 inclusive. When the number average molecular weight is excessively small, the crosslinking density increases. In this case, the hardness of the matrix resin is excessively high, and the recording speed may decrease. When the number average molecular weight is excessively large, the compatibility with other components decreases, and the crosslinking density decreases. In this case, the hardness of the matrix resin is excessively low, and recorded contents may be lost.

<Polyol>

Examples of the polyol include polypropylene polyols, polycaprolactone polyols, polyester polyols, and polycarbonate polyols.

(Polypropylene polyol)

The polypropylene polyol is obtained by a reaction of propylene oxide with a diol or a polyhydric alcohol. Examples of the diol and the polyhydric alcohol include ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, polyethylene glycol, and polytetramethylene glycol. Commercial examples of the polypropylene polyol include: SANNIX GP-400 and GP-1000 (product names, products of Sanyo Chemical Industries, Ltd.); and ADEKA POLYETHER G400, G700, and G1500 (product names, products of ADEKA CORPORATION).

(Polycaprolactone polyol)

The polycaprolactone polyol is obtained by a reaction of a lactone with a diol or a polyhydric alcohol. Examples of the lactone include α-caprolactone, β-caprolactone, γ-caprolactone, ε-caprolactone, α-methyl-ε-caprolactone, and β-methyl-ε-caprolactone.
Examples of the diol and the polyhydric alcohol include ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, polyethylene glycol, and polytetramethylene glycol.
Commercial examples of the polycaprolactone polyol obtained by a reaction of ε-caprolactone include PLACCEL 205, PLACCEL 205H, PLACCEL 205U, PLACCEL 205UT, PLACCEL 210, PLACCEL 210N, PLACCEL 210CP, PLACCEL 220, PLACCEL 230, PLACCEL 230N, PLACCEL 240, PLACCEL 220EB, PLACCEL 220EC, PLACCEL 303, PLACCEL 305, PLACCEL 308, PLACCEL 309, PLACCEL 312, PLACCEL 320, PLACCEL 401, PLACCEL L205AL, PLACCEL L212AL, PLACCEL L220AL, PLACCEL L320AL, PLACCEL T2103, PLACCEL T2205, and PLACCEL P3403 (product names, products of Daicel Corporation).

(Polyester polyol)

The polyester polyol is obtained, for example, by polycondensation of a dicarboxylic acid or an anhydride thereof

with a polyol.

Examples of the dicarboxylic acid include succinic acid, adipic acid, sebacic acid, azelaic acid, dimer acid, maleic anhydride, isophthalic acid, terephthalic acid, and trimellitic acid.

Examples of the polyol include ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, polyethylene glycol, and polytetramethylene glycol.

Examples of the polyester polyol include polyethylene adipate, polybutylene adipate, and polyhexamethylene adipate. Commercial examples of the polyester polyol include: ADEKA NEWACE F series, ADEKA NEWACE Y series, and ADEKA NEWACE NS series (product names, products of ADEKA CORPORATION); and Kuraray Polyol N-2010, P-4011, and P-1020 (produce names, products of KURARAY Co., Ltd.).

(Polycarbonate polyol)

Examples of the polycarbonate polyol include; polyols obtained by a dealcoholization condensation reaction of glycols with dialkyl carbonates (such as dimethyl carbonate and diethyl carbonate); polyols obtained by a dephenolization condensation reaction of glycols with diphenyl carbonates; and polyols obtained by a deglycolization condensation reaction of glycols with carbonates (such as ethylene carbonate and diethyl carbonate).

Examples of the glycols include: aliphatic diols such as 1,6-hexanediol, diethylene glycol, propylene glycol, 1,4-butanediol, 3-methyl-1,5 pentanediol, and neopentyl glycol; and alicyclic diols such as 1,4-cyclohexanediol and 1,4-cyclohexanedimethanol.

Examples of the polycarbonate polyol include: poly(hexamethylene carbonate)polyol obtained by a condensation reaction of 1,6-hexanediol with diethyl carbonate; poly(pentylene carbonate) obtained by a condensation reaction of pentanediol with diethyl carbonate; and poly(butylene carbonate) obtained by a condensation reaction of 1,4-butanediol with diethyl carbonate.

Commercial examples of the polycarbonate polyol include: PLACCEL CD CD205, PLACCEL CD CD210, and PLACCEL CD CD220 (product names, products of Daicel Corporation); and, DURANOL T5651, DURANOL T5652, and DURANOL T5650J (product names, products of Asahi Kasei Corporation).

(Molecular weight of polyol)

As for the molecular weight of the polyol described above, its number average molecular weight is preferably from 100 to 50000 inclusive, more preferably from 150 to 10000 inclusive, and still more preferably from 150 to 5000 inclusive. When the number average molecular weight is excessively small, the crosslinking density increases. In this case, the hardness of the matrix resin is excessively high, and the recording speed may decrease. When the number average molecular weight is excessively large, the compatibility with other components decreases, and the crosslinking density decreases. In this case, the hardness of the matrix resin is excessively low, and recorded contents may be lost.

<Additional components>

The matrix resin in the present embodiment may contain, in addition to the components described above, additional components so long as the gist of the present invention is retained.

Examples of the additional components include compounds having a hydroxyl group such as ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, trimethylolpropane, polyethylene glycol, and polytetramethylene glycol. These compounds are used for the purpose of changing the physical properties of the matrix resin.

<Urethane polymerization catalyst>

To facilitate the reaction of the isocyanate with the polyol, an appropriate urethane polymerization catalyst may be contained.

Examples of the urethane polymerization catalyst include: onium salts such as bis(4-t-butylphenyl)iodonium perfluoro-1-butanesulfonate, bis(4-t-butylphenyl)iodonium p-toluenesulfonate, bis(4-t-butylphenyl)iodonium trifluoromethanesulfonate, (4-bromophenyl)diphenylsulfonium triflate, (4-t-butylphenyl)diphenylsulfonium trifluoromethanesulfonate, diphenyliodonium perfluoro-1-butanesulfonate, (4-fluorophenyl)diphenylsulfonium trifluoromethanesulfonate, diphenyl-4-methylphenylsulfonium trifluoromethanesulfonate, triphenylsulfonium trifluoromethanesulfonate, and bis(alkylphenyl)iodonium hexafluorophosphonate; catalysts prepared using Lewis acids as main components such as zinc chloride, tin chloride, iron chloride, aluminum chloride, and $BF_3$; proton acids such as hydrochloric acid and phosphoric

acid; amines such as trimethylamine, triethylamine, triethylenediamine, dimethylbenzylamine, and diazabicycloundecene; imidazoles such as 2-methylimidazole, 2-ethyl-4-methylimidazole, and 1-cyanoethyl-2-undecylimidazolinium trimellitate; bases such as sodium hydroxide, potassium hydroxide, and potassium carbonate; tin catalysts such as dibutyltin laurate, dioctyltin laurate, and dibutyltin octoate; bismuth catalysts such as bismuth tris(2-ethylhexanoate) and tribenzoyloxy bismuth; and zirconium catalysts such as zirconium tetrakis(ethylacetoacetate), 1,1'-isopropylidenezirconocene dichloride, and zirconium tetrakis(2,4-pentanedionato).

Of these, bismuth catalysts and zirconium catalysts are preferred in order to improve storage stability.

There is no particular limitation on the bismuth-based catalyst so long as it is a catalyst containing elemental bismuth and is a compound that facilitates the reaction of the isocyanate with the polyol.

Examples of the bismuth-based catalyst include bismuth tris(2-ethylhexanoate), tribenzoyloxy bismuth, bismuth triacetate, bismuth tris(dimethyldithiocarbamate), bismuth hydroxide, triphenylbismuth(V) bis(trichloroacetate), tris(4-methylphenyl)oxobismuth(V), and triphenylbis(3-chlorobenzoyloxy)bismuth(V).

In particular, a trivalent bismuth compound is preferred in terms of catalytic activity, and bismuth carboxylate, i.e., a compound represented by general formula $Bi(OCOR)_3$ ((R is a linear or branched alkyl group, a cycloalkyl group, or a substituted or unsubstituted aromatic group), is more preferred. Any one of these bismuth-based catalysts may be used alone, or any combination of two or more of them may be used at any ratio.

There is no particular limitation on the zirconium-based catalyst so long as it is a catalyst containing elemental zirconium and is a compound that facilitates the reaction of the isocyanate with the polyol.

Examples of the zirconium-based catalyst include cyclopentadienylzirconium trichloride, decamethylzirconocene dichloride, 1,1'-dibutylzirconocene dichloride, 1,1'-isopropylidenezirconocene dichloride, tetrakis(2,4-pentanedionato)zirconium, tetrakis(trifluoro-2,4-pentanedionato)zirconium, tetrakis(hexafluoro-2,4-pentanedionato)zirconium, zirconium butoxide, zirconium-t-butoxide, zirconium propoxide, zirconium isopropoxide, zirconium ethoxide, bis(ethylacetoacetate)dibutoxy zirconium, tetrakis(ethylacetoacetate)zirconium, zirconium oxide, barium zirconium oxide, calcium zirconium oxide, zirconium bromide, zirconium chloride, zirconium fluoride, (indenyl) zirconium dichloride, and zirconium carbonate.

In particular, compounds having an organic ligand are preferred in terms of compatibility with other components, and compounds having an alkoxide structure or an acetylacetonate (2,4-pentanedionato) structure are more preferred. Any one of the above zirconium compounds may be used alone, or any combination of two or more of them may be used at any ratio.

One of the bismuth-based catalysts and the zirconium-based catalysts may be used alone, or any mixture of them may be used.

The ratio of the amount of the urethane polymerization catalyst used to the amount of the matrix resin is usually 0.0001% by mass or more and preferably 0.001% by mass or more, and is usually 10% by mass or less and preferably 5% by mass or less. When the amount of the urethane polymerization catalyst used is excessively small, an excessively long time may be necessary for curing. When the amount used is excessively large, it may be difficult to control the curing reaction.

The use of the urethane polymerization catalyst allows curing at room temperature. However, the curing may be performed at increased temperature. The temperature in this case is preferably between 40°C to 90°C.

6-1-3. Photocurable resin

When the matrix resin used is a photocurable resin, it is necessary to cure the matrix resin using a photo-initiator for the matrix resin suitable for the wavelength used. During curing of the matrix resin under irradiation with light, defective forming or poor bonding may occur. It is therefore desirable that the curing reaction is stable at around room temperature, which is main working temperature. In consideration of this, catalytic curing using the photo-initiator for the matrix resin is a desirable choice.

An active species, i.e., cations or anions, is usually generated from the photo-initiator for the matrix resin under irradiation with light. It is therefore preferable that a photocurable resin that is cured by such an active species is selected as the matrix resin.

Examples of the functional group reactive with cations such as protons include an epoxy group and an oxetanyl group. Specific examples of a compound having an epoxy group include: polyglycidyl ether compounds of polyols such as (poly)ethylene glycol, (poly)propylene glycol, (poly)tetramethylene glycol, trimethylolpropane, and glycerin; alicyclic epoxy compounds having a 4- to 7-membered cyclic aliphatic group such as 3,4-epoxycyclohexylmethyl-3,4-epoxycyclohexanecarboxylate and 3,4-epoxy-1-methylcyclohexyl-3,4-epoxy-1-methylhexanecarboxylate; bisphenol A-type epoxy compounds; hydrogenated bisphenol A-type epoxy compounds; bisphenol F-type epoxy compounds; and phenol and cresol novolac-type epoxy compounds. Examples of a compound having an oxetanyl group include; 2-ethyl-2-oxetanyl ether of bisphenol A; and 1,6-bis(2-ethyl-2-oxetanyloxy)hexane. (Note that the term "(poly)ethylene glycol," for example, means both "ethylene glycol" and "polyethylene glycol" which is a polymer of ethylene glycol.)

Examples of the functional group reactive with anions include an epoxy group and an episulfide group. Specific examples of a compound having an episulfide group include phenyl episulfide and diepisulfide methyl ether of bisphenol A.

The ratio of the amount of the photo-initiator for the matrix resin that is used to photo-cure the matrix resin to the amount of the polymerizable compound is usually 0.01% by mass or more and preferably 0.1% by mass or more, and is usually 1% by mass or less and preferably 0.5% by mass or less. When the amount used of the photo-initiator for the matrix resin is excessively small, an excessively long time may be necessary for curing. When the amount used is excessively large, it may be difficult to control the curing reaction.

In particular, when the polymerizable composition is used as a holographic recording material, the polymerizable composition is irradiated with light also during recording. It is therefore important that the wavelength for curing be different from the wavelength for recording, and the difference in wavelength is at least 10 nm and preferably 30 nm. The selection of the photo-initiator for the matrix resin can be roughly estimated from the absorption wavelength of the initiator.

6-2. Photopolymerization initiator

Any known photo radical polymerization initiator can be used as the photopolymerization initiator that assists the polymerization of the compound of the present invention. Examples include azo-based compounds, azide-based compounds, organic peroxides, organic borates, onium salts, bisimidazole derivatives, titanocene compounds, iodonium salts, organic thiol compounds, halogenated hydrocarbon derivatives, acetophenones, benzophenones, hydroxybenzenes, thioxanthones, anthraquinones, ketals, acylphosphine oxides, sulfone compounds, carbamic acid derivatives, sulfonamides, triarylmethanols, oxime esters, and the like. In particular, the photopolymerization initiator is preferably a titanocene compound, an acylphosphine oxide compound, an oxime ester compound, and the like, because the polymerization reaction proceeds under light in the visible range.

6-2-1. Titanocene compound

When a titanocene compound is used as the photopolymerization initiator, no particular limitation is imposed on the type of titanocene compound. For example, one selected from various titanocene compounds described in JP59-152396A, JP61-151197A, and the like, can be appropriately used.

Specific examples of the titanocene compound include di-cyclopentadienyl-Ti-di-chloride, di-cyclopentadienyl-Ti-bis-phenyl, di-cyclopentadienyl-Ti-bis-2,3,4,5,6-pentafluorophen-1-yl, dicyclopentadienyl-Ti-bis-2,3,5,6-tetrafluorophen-1-yl, di-cyclopentadienyl-Ti-bis-2,4,6-trifluorophen-1-yl, di-cyclopentadienyl-Ti-bis-2,6-di-fluorophen-1-yl, di-cyclopentadienyl-Ti-bis-2,4-di-fluorophen-1-yl, di-methylcyclopentadienyl-Ti-bis-2,3,4,5,6-pentafluorophen-1-yl, di-methylcyclopentadienyl-Ti-bis-2,3,5,6-tetrafluorophen-1-yl, di-methylcyclopentadienyl-Ti-bis-2,6-difluorophen-1-yl, di-cyclopentadienyl-Ti-bis-2,6-difluoro-3-(pyrr-1-yl)-phen-1-yl, and the like.

6-2-2. Acylphosphine oxide compound

Specific examples of the acylphosphine oxide compound include monofunctional initiators having only one photo-cleavage point per molecule and bifunctional initiators having two photo-cleavage points per molecule.

Examples of the monofunctional initiator include triphenylphosphine oxide, diphenyl(2,4,6-trimethylbenzoyl) phosphine oxide, 2,6-dichlorobenzoyldiphenylphosphine oxide, and the like.

Examples of the bifunctional initiator include bis(2,4,6-trimethylbenzoyl)-phenylphosphine oxide, bis(2,6-dimethoxybenzoyl)-2,4,4-trimethylpentylphosphine oxide, bis(2,6dichlorobenzoyl)-4-propylphenylphosphine oxide, bis(2,6dichlorobenzoyl)-2,5dimethylphenylphosphine oxide, and the like.

6-2-3. Oxime ester-based compound

Specific examples of the oxime ester-based compound include 1-[4-(phenylthio)-2-(O-benzoyloxime)]-1,2-octanedione, 1-[9-ethyl-6-(2-methylbenzoyl)-9H-carbazol-3-yl]-1-(O-acetyloxime)ethanone, 4-(acetoxyimino)-5-[9-ethyl-6-(2-methylbenzoyl)-9H-carbazol-3-yl]-5-oxopentanoic acid methyl ester, 1-(9-ethyl-6-cyclohexanoyl-9H-carbazol-3-yl)-1-(O-acetyloxime)glutaric acid methyl ester, 1-(9-ethyl-9H-carbazol-3-yl)-1-(O-acetyloxime)glutaric acid methyl ester, 1-(9-ethyl-9H-carbazol-3-yl)-1-(O-acetyloxime)-3-methylbutanoic acid, and the like.

6-2-4. Amount of photopolymerization initiator used

Any one of the above photopolymerization initiators may be used alone, or any combination of two or more of them may be used at any ratio.

As for the content of the photopolymerization initiator in the polymerizable composition of the present invention,

the molar amount of the photopolymerization initiator per unit weight of the polymerizable composition is preferably 0.5 μmol/g or more, and more preferably 1 μmol/g or more. As for the content of the photopolymerization initiator in the polymerizable composition of the present invention, the molar amount per unit weight of the polymerizable composition is preferably 100 μmol/g or less, and more preferably 50 μmol/g or less.

When the content of the photopolymerization initiator is excessively small, the amount of radicals generated is small. In this case, the rate of photopolymerization decreases, and the recording sensitivity of a holographic recording medium may decrease. On the other hand, when the content of the photopolymerization initiator is excessively large, radicals generated by irradiation with light recombine with each other or disproportionate. In this case, the contribution of the radicals to photopolymerization is reduced, and again the recording sensitivity of a holographic recording medium may decrease. When two or more photopolymerization initiators are used in combination, it is preferable that the total amount of the photopolymerization initiators is set so as to fall within the above range.

6-3. Radical scavenger

In holographic recording, a radical scavenger may be added in order to fix the intensity pattern of interference light accurately as a polymer distribution in a holographic recording medium. Preferably, the radical scavenger has both a functional group that scavenges radicals and a reactive group to be fixed to the matrix resin through a covalent bond. Examples of the functional group that scavenges radicals include a stable nitroxyl radical group.

6-3-1. Type of radical scavenger

Examples of the reactive group to be fixed to the matrix resin through a covalent bond include a hydroxy group, an amino group, an isocyanate group, and a thiol group. Examples of such a radical scavenger include 4-hydroxy-2,2,6,6-tetramethylpiperidine-1-oxyl free radicals (TEMPOL), 3-hydroxy-9-azabicyclo[3.3.1]nonane N-oxyl, 3-hydroxy-8-azabicyclo[3.2.1]octane N-oxyl, and 5-HO-AZADO: 5-hydroxy-2-azatricyclo[3.3.1.1$^{3,7}$]decane N-oxyl.

6-3-2. Content of radical scavenger

Any one of the above various radical scavengers may be used alone, or any combination of two or more of them may be used at any ratio.

As for the content of the radical scavenger in the polymerizable composition of the present invention, the molar amount of the radical scavenger per unit weight of the polymerizable composition is preferably 0.5 μmol/g or more and more preferably 1 μmol/g or more. The content of the radical scavenger in the polymerizable composition of the present invention is preferably 100 μmol/g or less and more preferably 50 μmol/g or less.

When the content of the radical scavenger is excessively small, the efficiency of scavenging radicals is low, and a polymer with a low degree of polymerization diffuses, so that the amount of components not contributing to signals tends to increase. On the other hand, when the content of the radical scavenger is excessively large, the efficiency of the polymerization of the polymer decreases, and signals tend not to be recorded. When two or more radical scavengers are used in combination, it is preferable that the total amount of the radical scavengers is set so as to fall within the above range.

6-4. Additional components

The polymerizable composition of the present invention may contain additional components in addition to the above components unless the present invention departs from the scope thereof.

Examples of the additional components include components for preparing the polymerizable composition such as a solvent, a plasticizer, a dispersant, a leveling agent, an antifoaming agent, an adhesion promoter, and the like. Examples of the additional components when the polymerizable composition is used for a holographic recording medium include components for controlling a recording reaction such as a chain transfer agent, a polymerization terminator, a compatibilizer, a reaction aid, a sensitizer, and the like. Examples of other additives necessary for improving properties include a preservative, a stabilizer, an antioxidant, an ultraviolet absorber, a light stabilizer, and the like. Any one of these components may be used alone, or any combination of two or more of them may be used at any ratio.

<Sensitizer>

A compound that controls excitation of the photopolymerization initiator may be added to the polymerizable composition of the present invention. Examples of such a compound include a sensitizer, a sensitization aid, and the like.

The sensitizer used may be selected from various known sensitizers. Generally, a colored compound such as a coloring agent is often used as the sensitizer in order to absorb visible and ultraviolet laser beams. When the sensitizer is

used for a holographic recording medium, a suitable sensitizer is selected according to the wavelength of a laser beam used for recording and the type of initiator used. Specific preferred examples of the sensitizer used for a system using a green laser include compounds described in JPH5-241338A, JPH2-69A, JPH2-55446B, and the like. Examples of the sensitizer used for a system using a blue laser include compounds described in JP2000-10277A, JP2004-198446A, and the like. Any one of these sensitizers may be used alone, or any combination of two or more of them may be used at any ratio.

When a holographic recording medium to be obtained is required to be colorless and transparent, it is preferable to use a cyanine-based coloring agent as the sensitizer. Generally, a cyanine-based coloring agent is easily decomposed by light. Therefore, when the holographic recording medium is subjected to postexposure, i.e., left to stand under exposure to indoor light or sunlight for several hours to several days, the cyanine-based coloring agent in the holographic recording medium is decomposed, and the holographic recording medium does not exhibit absorption in the visible range. The thus-obtained holographic recording medium is colorless and transparent.

It is necessary to increase or decrease the amount of the sensitizer according to the thickness of a recording layer to be formed. The ratio of the amount of the sensitizer to the amount of the photopolymerization initiator described above in 6-2 is usually 0.01% by mass or more and preferably 0.1% by mass or more, and is usually 10% by mass or less and preferably 5% by mass or less. When the amount of the sensitizer used is excessively small, the initiation efficiency is low, and recording may take a very large amount of time. On the other hand, when the amount of the sensitizer used is excessively large, absorption of light used for recording and reproduction increases, and the light may not easily penetrate deep into the recording layer. When two or more sensitizers are used in combination, the total amount of the sensitizers is set so as to fall within the above range.

<Plasticizer>

The polymerizable composition of the present invention may contain a plasticizer to improve the reaction efficiency and adjust the physical properties of the recording layer of a holographic recording medium.

Examples of the plasticizer include: phthalates such as dioctyl phthalate, diisononyl phthalate, diisodecyl phthalate, diundecyl phthalate, and the like; adipates such as bis(2-ethylhexyl) adipate, diisononyl adipate, di-n-butyl adipate, and the like; sebacates such as dioctyl sebacate, dibutyl sebacate, and the like; phosphates such as tricresyl phosphate, and the like; citrates such as acetyl tributyl citrate, and the like; trimellitates such as trioctyl trimellitate, and the like; epoxidized soybean oil; chlorinated paraffin; alkoxylated (poly)alkylene glycol esters such as acetoxymethoxypropane, and the like; and alkoxy-terminated polyalkylene glycols such as dimethoxypolyethylene glycol, and the like.

A plasticizer containing elemental fluorine described in Japanese Patent No. 6069294 may also be used. Examples of the plasticizer containing elemental fluorine include 2,2,2-trifluoroethyl butylcarbamate, bis(2,2,2-trifluoroethyl)-(2,2,4-trimethylhexane-1,6-diyl)biscarbamate, bis(2,2,2-trifluoroethyl)-[4-({[(2,2,2-trifluoroethoxy)carbonyl]amino}-methyl)octane-1,8-diyl]biscarbamate, 2,2,3,3,4,4,5,5,6,6,7,7,8,8,9,9-hexadecafluorononyl butylcarbamate, 2,2,2-trifluoroethyl phenylcarbamate, and the like.

These plasticizers are usually used in an amount of 0.01% by mass or more and 50% by mass or less, preferably 0.05% by mass or more and 20% by mass or less based on the total solid content of the polymerizable composition. When the content of the plasticizer is below the above range, the effects of improving the reaction efficiency and adjusting the physical properties are not obtained. When the content is above the above range, the transparency of the recording layer deteriorates, and bleeding of the plasticizer becomes significant.

<Leveling agent>

A leveling agent may be used for the polymerizable composition of the present invention. Examples of the leveling agent include sodium polycarboxylates, ammonium polycarboxylates, amine polycarboxylates, silicon-based leveling agents, acrylic-based leveling agents, ester compounds, ketone compounds, fluorine compounds, and the like. Any one of them may be used alone, or any combination of two or more of them may be used at any ratio.

<Chain transfer agent>

A chain transfer agent may be used for the polymerizable composition of the present invention. Examples of the chain transfer agent include: phosphinates such as sodium phosphite, sodium hypophosphite, and the like; mercaptans such as mercaptoacetic acid, mercaptopropionic acid, 2-propanethiol, 2-mercaptoethanol, thiophenol, and the like; aldehydes such as acetaldehyde, propionaldehyde, and the like; ketones such as acetone, methyl ethyl ketone, and the like; halogenated hydrocarbons such as trichloroethylene, perchloroethylene, and the like; terpenes such as terpinolene, $\alpha$-terpinene, $\beta$-terpinene, $\gamma$-terpinene, and the like; non-conjugated dienes such as 1,4-cyclohexadiene, 1,4-cycloheptadiene, 1,4-cyclooctadiene, 1, 4-heptadiene, 1,4-hexadiene, 2-methyl-1,4-pentadiene, 3,6-nonanedien-1-ol, 9,12-octadecadienol, and the like; linolenic acids such as linolenic acid, γ-linolenic acid, methyl linoleate, ethyl linoleate, isopropyl linoleate, linolenic anhydride, and the like; linoleic acids such as linoleic acid, methyl linoleate, ethyl linoleate, isopropyl linoleate, linoleic anhydride, and the like; eicosapentaenoic acids such as eicosapentaenoic acid, ethyl eicosapentaenoate, and the like; docosahexaenoic acids such as docosahexaenoic acid, ethyl docosahexaenoate, and the like.

The ratio of the amount of the additives to the total amount of solids in the polymerizable composition in the present embodiment is usually 0.001% by mass or more and preferably 0.01% by mass or more, and is usually 30% by mass or less, and preferably 10% by mass or less. When two or more additive are used in combination, the total amount of the additives is set so as to fall within the above range.

6-5. Compositional ratio of components in polymerizable composition

The contents of the components in the polymerizable composition of the present invention can be freely set so long as they do not deviate from the scope of the present invention. Preferably, the ratios of the components shown below in terms of their molar amounts per unit mass of the polymerizable composition fall within the following ranges.

The content of the polymerizable compounds including the compound of the present invention is preferably 5 μmol/g or more, more preferably 10 μmol/g or more, and still more preferably 100 μmol/g or more. The content of the polymerizable compounds is preferably 1000 μmol/g or less, more preferably 500 μmol/g or less, and still more preferably 300 μmol/g or less.

When the content of the polymerizable compounds is equal to or more than the above more limit, a holographic recording medium having sufficient diffraction efficiency is obtained. When the content is equal to or lower than the above upper limit, the compatibility with the resin matrix in the recording layer is maintained, and the degree of shrinkage of the recording layer due to recording tends to be small.

When the isocyanate and the polyol are used for the matrix resin in the polymerizable composition of the present invention, the total content of the isocyanate and the polyol is usually 0.1% by mass or more, preferably 10% by mass or more, and still more preferably 35% by mass or more, and is usually 99.9% by mass or less, and preferably 99% by mass or less. When the total content is equal to or more than the lower limit, the recording layer can be formed easily.

In this case, the ratio of the number of functional groups in the polyol that are reactive with the isocyanate to the number of isocyanate groups in the isocyanate is preferably 0.1 or more and more preferably 0.5 or more, and is usually 10.0 or less and preferably 2.0 or less. When this ratio is within the above range, the number of unreacted functional groups is small, and the storage stability is improved.

In the polymerizable composition, it is preferable to determine the content of the urethane polymerization catalyst in consideration of the reaction rate of the isocyanate and the polyol, and the content is preferably 5% by mass or less, more preferably 4% by mass or less, and still more preferably 1% by mass or less. The amount of the urethane polymerization catalyst used is preferably 0.003% by mass or more.

The total amount of additional components other than the above components may be 30% by mass or less and is preferably 15% by mass or less and more preferably 5% by mass.

6-6. Method for producing polymerizable composition

In the present invention, no particular limitation is imposed on the method for producing the polymerizable composition containing the polymerizable compound, the matrix resin, and the photopolymerization initiator. The order of mixing components can be appropriately determined. When the polymerizable composition contains a component other than the above components, any combination of these components may be mixed in any order.

When the isocyanate and the polyol are used for the matrix resin, the polymerizable composition can be obtained by the following method for example, but the present invention is not limited thereto.

The polymerizable compound, the photopolymerization initiator, and components other than the isocyanate and the urethane polymerization catalyst are mixed together to prepare a photoreactive composition (solution A). A mixture of the isocyanate and the urethane polymerization catalyst is prepared as solution B.

Alternatively, the polymerizable compound, the photopolymerization initiator, and components other than the isocyanate may be mixed to prepare a photoreactive composition (solution A).

Preferably, each solution is subjected to dewatering and degassing. When the dewatering and degassing are insufficient, air bubbles are generated during the production of a holographic recording medium, and therefore a uniform recording layer may not be obtained. The dewatering and degassing may be performed by heating under reduced pressure so long as the components are not damaged.

The preparation of the polymerizable composition by mixing the solution A and solution B is preferably carried out immediately before molding of the holographic recording medium. In this case, a mixing technique using a conventional method may be used. When the solution A and the solution B are mixed, degassing may be optionally performed in order to remove residual gas. Preferably, the solution A and the solution B are subjected to a filtration process separately or

simultaneously after mixing in order to remove foreign substances and impurities. It is more preferable to filter these solutions separately.

The isocyanate used for the matrix resin may be an isocyanate-functional prepolymer prepared by a reaction of an isocyanate having an excessive amount of isocyanate groups with the polyol. The polyol used for the matrix resin may be an isocyanate reactive prepolymer prepared by a reaction of a polyol containing an excessive amount of isocyanate reactive functional groups with the isocyanate.

6-7. About holographic recording medium of present invention

The holographic recording medium of the present invention that uses the polymerizable composition of the present invention includes the recording layer and optionally includes a support and additional layers. Generally, the holographic recording medium includes the support on which the recording layer and the additional layers are laminated to form the holographic recording medium. However, when the recording layer and the additional layers have the strength and durability required for the medium, the holographic recording medium may include no support. Examples of the additional layers include a protective layer, a reflective layer, an anti-reflective layer (anti- reflective film), and the like.

6-7-1. Recording layer

The recording layer of the holographic recording medium of the present invention is a layer formed from the polymerizable composition of the present invention, and is a layer in which information is recorded. The information is usually recorded as a hologram. As described later in detail in a recording method section, the polymerizable compound (hereinafter referred to as a polymerizable monomer) contained in the recording layer partially undergoes a chemical change such as polymerization and the like during holographic recording. Therefore, in the holographic recording medium after recording, part of the polymerizable monomer is consumed and present as a reacted compound such as a polymer and the like.

There is no particular limitation on the thickness of the recording layer, and the thickness may be appropriately determined in consideration of the recording method and the like. The thickness is preferably 1 μm or more and more preferably 10 μm or more, and is preferably 1 cm or less and more preferably 3 mm or less. When the thickness of the recording layer is equal to or more than the above lower limit, selectivity for holograms when multiple recording is performed on the holographic recording medium is high, and therefore the degree of multiple recording can tend to be increased. When the thickness of the recording layer is equal to or less than the above upper limit, it becomes possible to uniformly mold the entire recording layer, and multiple recording having uniform diffraction efficiency of each hologram and a high S/N ratio tends to be possible.

Preferably, the rate of shrinkage of the recording layer due to exposure to light during information recording or reproduction is 0.25% or less, from the viewpoint of recording reproducibility.

6-7-2. Support

There is no particular limitation on the details of the support so long as it has the strength and durability required for the holographic recording medium, and any support can be used.

Ther is no limitation on the shape of the support, but the support is usually formed into a flat plate or a film.

Ther is no limitation on the material forming the support, and the material may be transparent or may be opaque.

Examples of the transparent material for the support include: organic materials such as acrylic, polyethylene terephthalate, polyethylene naphthoate, polycarbonate, polyethylene, polypropylene, amorphous polyolefin, polystyrene, polycycloolefin, cellulose acetate, and the like; and inorganic materials such as glass, silicon, quartz, and the like. Among these, polycarbonate, acrylic, polyester, amorphous polyolefin, glass, and the like are preferred, and polycarbonate, acrylic, amorphous polyolefin, polycycloolefin, and glass are more preferred.

Examples of the opaque material for the support include: metals such as aluminum, and the like; and the above mentioned transparent support coated with a metal such as gold, silver, aluminum or the like or with a dielectric such as magnesium fluoride, zirconium oxide, or the like.

There is no particular limitation on the thickness of the support. Preferably, the thickness is in the range of 0.05 mm or more and 1 mm or less. When the thickness of the support is equal to or more than the above lower limit, the mechanical strength of the holographic recording medium can be ensured, and a warp of the substrate can be prevented. When the thickness of the support is equal to or less than the above upper limit, advantages such as an increase in the transmission amount of light, and reduction in the weight and cost of the holographic recording medium and the like can be obtained.

The surface of the support may be subjected to surface treatment. The surface treatment is usually performed in order to improve the adhesion between the support and the recording layer. Examples of the surface treatment include

corona discharge treatment performed on the support and the formation of an undercoat layer on the support in advance. Examples of the composition for the undercoat layer include halogenated phenols, partially hydrolyzed vinyl chloride-vinyl acetate copolymers, polyurethane resins, and the like.

The surface treatment on the support may be performed for a purpose other than the improvement in adhesion. Examples of such surface treatment include: reflecting coating treatment in which a reflecting coating layer is formed using a metal material such as gold, silver, aluminum, and the like; and dielectric coating treatment in which a dielectric layer formed of magnesium fluoride, zirconium oxide, and the like is formed. Such a layer may be formed as a single layer, or two or more layers may be formed.

The surface treatment may be performed for the purpose of controlling the gas and water permeability of the substrate. For example, the reliability of the holographic recording medium can be improved by providing the supports sandwiching the recording layer with a function of suppressing the permeability of gas and moisture.

The support may be laminated only on one of the upper and lower sides of the recording layer of the holographic recording medium of the present invention or may be laminated on both sides. When supports are laminated on both the upper and lower sides of the recording layer, at least one of the supports is made transparent so that it can transmit active energy rays (such as excitation light, reference light, and reproduction light, and the like).

When the holographic recording medium has the support on one side or both sides of the recording layer, a transmission hologram or a reflection hologram can be recorded. When a support having anti-reflection characteristics on one side of the recording layer is used, a reflection hologram can be recorded.

A pattern for data addressing may be provided on the support. In this case, there is no limitation on the patterning method. For example, irregularities may be formed on the support itself, or the pattern may be formed on the reflective layer described later. The pattern may be formed using a combination of these methods.

6-7-3. Protective layer

The protective layer is a layer for preventing deterioration of the recording-reproduction propertied of the recording layer. There is no limitation on the specific structure of the protective layer, and any known protective layer can be used. For example, a layer formed of a watersoluble polymer, an organic or inorganic material, and the like can be formed as the protective layer.

There is no particular limitation on the formation position of the protective layer. The protective layer may be formed, for example, on the surface of the recording layer or between the recording layer and the support or may be formed on the outer surface side of the support. The protective layer may be formed between the support and another layer.

6-7-4. Reflective layer

The reflective layer is formed when the holographic recording medium is configured to be reflection type. In the case of a reflection type holographic recording media, the reflective layer may be formed between the support and the recording layer, or may be formed on the outer surface of the support, but it is usually preferable that the reflective layer is formed between the support and the recording layer.

Any known reflective layer can be used, and a thin metal film can be used for example.

6-7-5. Anti-reflective film

In both the transmission type and reflection type holographic recording media, an anti-reflective film may be laminated on the sides where the information light, reference light and reproduction light are incident and exited, or between the recording layer and the support. The anti-reflective film serves to improve the efficiency of light utilization and to suppress the generation of noise.

Any known anti-reflective film may be used.

6-7-6. Method for producing holographic recording medium

There is no limitation on the method for producing the holographic recording medium of the present invention. For example, the holographic recording medium can be produced by coating the support with the polymerizable composition of the present invention without using a solvent to form the recording layer. Any known coating method can be used. Specific examples of the coating method include a spray method, a spin coating method, a wire bar method, a dipping method, an air knife coating method, a roll coating method, a blade coating method, a doctor roll coating method, and the like.

When a recording layer having a large thickness is formed, a method in which the polymerizable composition is molded using a die, a method in which the polymerizable composition is applied to a release film and punched with a die,

and the like may be used to form the recording layer. The holographic recording medium of the present invention may be produced by mixing the polymerizable composition of the present invention with a solvent or an additive to prepare a coating solution, coating the support with the coating solution, and then drying the coating solution to form the recording layer. In this case also, any coating method can be used. For example, any of the above described methods can be used.

There is no limitation on the solvent used for the coating solution. It is usually preferable to use a solvent that can dissolve the component used sufficiently, provides good coating properties, and does not damage the support such as a resin substrate. One solvent may be used alone, or any combination of two or more solvents may be used at any ratio. There is no limitation on the amount of the solvent used. However, from the viewpoint of coating efficiency and handleability, it is preferable to prepare a coating solution having a solid concentration of about 1 to 100% by mass.

Examples of the solvent include: ketone-based solvents such as acetone, methyl ethyl ketone, methyl isobutyl ketone, cyclohexanone, methyl amyl ketone, and the like; aromatic-based solvents such as toluene, xylene, and the like; alcohol-based solvents such as methanol, ethanol, propanol, n-butanol, heptanol, hexanol, diacetone alcohol, furfuryl alcohol, and the like; ketone alcohol-based solvents such as diacetone alcohol, 3-hydroxy-3-methyl-2-butanone, and the like; ether-based solvents such as tetrahydrofuran, dioxane, and the like; halogen-based solvents such as dichloromethane, dichloroethane, chloroform, and the like; cellosolve-based solvents such as methyl cellosolve, ethyl cellosolve, butyl cellosolve, methyl cellosolve acetate, ethyl cellosolve acetate, and the like; propylene glycol-based solvents such as propylene glycol monomethyl ether, propylene glycol monoethyl ether, propylene glycol monobutyl ether, propylene glycol monomethyl ether acetate, propylene glycol monoethyl ether acetate, propylene glycol monobutyl ether acetate, dipropylene glycol dimethyl ether, and the like; ester-based solvents such as ethyl acetate, butyl acetate, amyl acetate, butyl acetate, ethylene glycol diacetate, diethyl oxalate, ethyl pyruvate, ethyl-2-hydroxybutyrate ethylacetoacetate, methyl lactate, ethyl lactate, methyl 2-hydroxyisobutyrate, methyl 3-methoxypropionate, and the like; perfluoroalkyl alcohol-based solvents such as tetrafluoropropanol, octafluoropentanol, hexafluorobutanol, and the like; highly polar solvents such as dimethylformamide, dimethylacetamide, N-methylpyrrolidone, dimethyl sulfoxide, and the like; chain hydrocarbon-based solvents such as n-hexane, n-octane, and the like; cyclic hydrocarbon-based solvents such as cyclohexane, methylcyclohexane, ethylcyclohexane, dimethylcyclohexane, n-butylcyclohexane, tert-butylcyclohexane, cyclooctane, and the like; and mixtures of these solvents.

Examples of the holographic recording medium production method include: a production method in which the recording layer is formed by coating the support with the polymerizable composition fused by heat and cooling the polymerizable composition to solidify the composition; a production method in which the recording layer is formed by coating the support with the polymerizable composition in liquid form and subjecting the polymerizable composition to thermal polymerization to cure the composition; a production method in which the recording layer is formed by coating the support with the polymerizable composition in liquid form and subjecting the polymerizable composition to photopolymerization to cure the composition, and the like.

The thus-produced holographic recording medium can be in the form of a self-supporting slab or disk and can be used for three-dimensional image display devices, diffraction optical elements, large-capacity memories, and the like.

In particular, the holographic recording medium of the present invention that uses the polymerizable composition of the present invention has a high refractive index modulation and is useful also for light guide plates of AR glasses (AR glasses).

6-7-7. Applications of holographic recording medium

<Large-capacity memory applications>

Information is written (recorded) and read (reproduced) to the holographic recording medium of the present invention by irradiating the medium with light.

When recording information, light capable of causing a chemical change in the polymerizable monomer, and the like, its polymerization and a change in concentration, is used as the object light (referred to also as recording light).

For example, when information is recorded as a volume hologram, the object light together with the reference light is applied to the recording layer to allow the object light to interfere with the reference light in the recording layer. In this case, the interfering light causes polymerization of the polymerizable monomer and a change in its concentration within the recording layer. Therefore, the interference fringes cause refractive index differences within the recording layer, and the information is recorded as a hologram through the interference fringes recorded in the recording layer.

When reproducing the volume hologram recorded in the recording layer, prescribed reproduction light (usually the reference light) is applied to the recording layer. The applied reproduction light is diffracted by the interference fringes. Since the diffracted light contains the same information as that in the recording layer, the information recorded in the recording layer can be reproduced by reading the diffracted light using appropriate detection means.

The wavelength ranges of the object light, the reproduction light, and the reference light can be freely set according to their applications and may be in the visible range or the ultraviolet range. Preferred examples of such light

include light from lasers with good monochromaticity and directivity such as: solid lasers such as ruby, glass, Nd-YAG, Nd-$YVO_4$ lasers, and the like; diode lasers such as GaAs, InGaAs, GaN lasers, and the like; gas lasers such as helium-neon, argon, krypton, excimer, $CO_2$ lasers, and the like; and dye lasers including dyes, and the like.

There is no limitation on the amounts of irradiation of the object light, the reproduction light, and the reference light, and these amounts can be freely set so long as recording and reproduction are possible. When the amounts of irradiation are extremely small, the chemical change of the polymerizable monomer is too incomplete, and the heat resistance and mechanical properties of the recording layer may not be fully obtained. When the amounts of irradiation are extremely large, the components of the recording layer (the components of the polymerizable composition of the present invention) may deteriorate. Therefore, the object light, the reproduction light, and the reference light are usually irradiated in the range of 0.1 $J/cm^2$ or more and 20 $J/cm^2$ or less according to the composition of the polymerizable composition of the present invention used to form the recording layer, the type of photopolymerization initiator, the amount of the photopolymerization initiator mixed, and the like.

Examples of the holographic recording method include a polarized collinear holographic recording method, a reference light incidence angle multiplexing holographic recording method, and the like. When the holographic recording medium of the present invention is used as a recording medium, good recording quality can be provided using any of the recording methods.

<Applications of light guide plate for AR glass >

Volume holograms are recorded in the holographic recording medium of the present invention in the same manner as in the large-capacity memory applications described above.

The volume holograms recorded in the recording layer are irradiated with prescribed reproduction light through the recording layer. The irradiated reproduction light is diffracted according to the interference fringes. In this case, even when the wavelength of the reproduction light does not coincide with the wavelength of the recording light, diffraction occurs when the Bragg condition for the interference fringes is satisfied. Therefore, by recording interference fringes corresponding to the wavelengths and incident angles of reproduction light beams to be diffracted, the reproduction light beams in a wide wavelength range can be diffracted, and the color display range of AR glasses can be increased.

By recording interference fringes corresponding to the wavelength and diffraction angle of reproduction light, the reproduction light entering from the outside of the holographic recording medium can be guided to the inside of the holographic recording medium, and the reproduction light guided inside the holographic recording medium can be reflected, split, and expanded or reduced in size. Moreover, the reproduction light guided through the inside of the holographic recording medium can be emitted to the outside of the holographic recording medium. This allows the viewing angle of AR glasses to be increased.

The wavelength ranges of the object light and the reproduction light can be freely set according to their applications, and the object light and the reproduction light may be in the visible range or in the ultraviolet range. Preferred examples of the object light and the reproduction light include light from the above-described lasers, and the like. The reproduction light is not limited to light from a laser, and the like, and display devices such as liquid crystal displays (LCDs), organic electroluminescent displays (OLEDs), and the like can also be used preferably.

There is no limitation on the amounts of irradiation of the object light, the reproduction light, and the reference light, and these amounts can be freely set so long as recording and reproduction are possible. When the amounts of irradiation are extremely small, the chemical change of the polymerizable monomer is too incomplete, and the heat resistance and mechanical properties of the recording layer may not be fully obtained. When the amounts of irradiation are extremely large, the components of the recording layer (the components of the polymerizable composition of the present invention) may deteriorate. Therefore, the object light, the reproduction light, and the reference light are usually irradiated in the range of 0.1 $J/cm^2$ or more and 20 $J/cm^2$ or less according to the composition of the polymerizable composition of the present invention used to form the recording layer, the type of photopolymerization initiator, the amount of the photopolymerization initiator mixed, and the like.

6-8. About performance indicator of holographic recording medium

The total $\Delta n$ calculated using the sum of the diffraction efficiency over the entire multiple recording is used as the indicator of the performance of the holographic recording medium. In the case of a transmission hologram, the diffraction efficiency of the hologram is given as the ratio of the intensity of the diffracted light to the sum of the intensity of the transmitted light and the intensity of the diffracted light. From the obtained diffraction efficiency, $\Delta n$ is calculated using the following formula in Coupled Wave Theory (H. Kogelnik, The Bell System Technical Journal (1969), 48, 2909-2947), and the total over the entire multiple recording is used as the total $\Delta n$.

[Math. 1]

$$\eta = \sin^2\left(\frac{\pi \cdot T \cdot \Delta n}{\lambda \cdot \cos\theta}\right)$$

$$total\Delta n = \sum \Delta n$$

Here, $\eta$ is the diffraction efficiency, and T is the thickness of the medium. $\lambda$ is the wavelength of the reference light, and $\theta$ is the incident angle of the reference light.

In the case of large-capacity memories, a higher total $\Delta$n means that more information can be recorded per unit volume, which is preferable. Also, in the case of AR glasses, a higher total $\Delta$n means that the projected image can be delivered brighter to the eyes, power consumption can be reduced, and the viewing angle can be widened, which is preferable.

EXAMPLES

The present invention will be described in more detail by way of Examples and Synthesis Examples. However, the present invention is not limited to the Examples and Synthesis Examples so long as they do not depart from the scope of the invention.

[Raw materials used]

Raw materials of compositions used in the Examples and Comparative Examples are as follows.

<Isocyanate>

- DURANATE (registered trademark) TSS-100: hexamethylene diisocyanate-based polyisocyanate (NCO: 17.6%) (manufactured by Asahi Kasei Corporation)

<Polyol>

- PLACCEL PCL-205U: polycaprolactone diol (molecular weight: 530) (manufactured by Daicel Corporation)
- PLACCEL PCL-305: polycaprolactone triol (molecular weight: 550) (manufactured by Daicel Corporation)

<Photopolymerization initiator>

- HLI02: 1-(9-ethyl-6-cyclohexanoyl-9H-carbazol-3-yl)-1-(O-acetyloxime)glutaric acid methyl ester

<Radical scavenger>

- TEMPOL: 4-hydroxy-2,2,6,6-tetramethylpiperidine-1-oxyl free radicals (manufactured by TOKYO CHEMICAL INDUSTRY Co., Ltd.)

<Light stabilizer>

- ADEKA STAB LA-63P (manufactured by ADEKA Co., Ltd.)

<Urethane polymerization catalyst>

- Octylic acid solution of bismuth tris(2-ethylhexanoate) (amount of effective component: 56% by weight)

[Example 1]

<Production of compound M-1>

Compound M-1 was produced by the following synthesis method.

[Chem. 11]

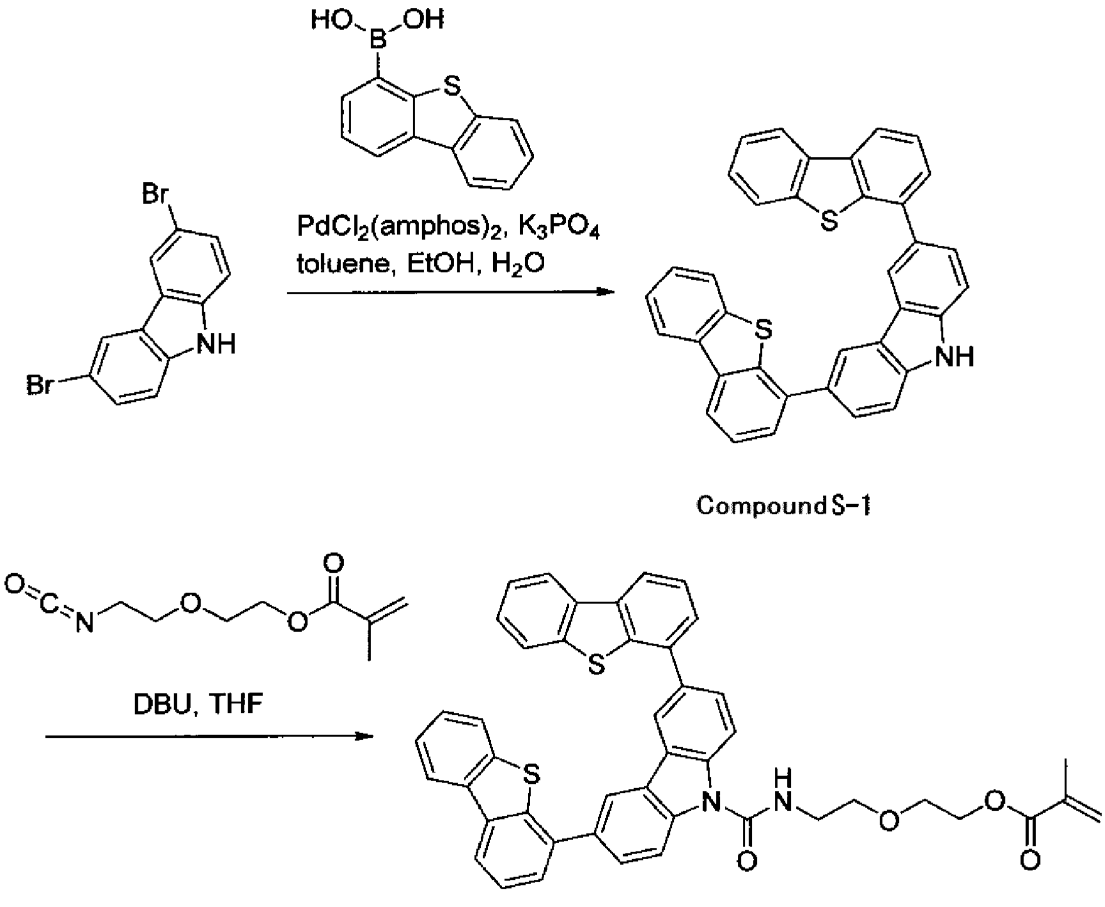


Compound S-1

Compound M-1

3,6-Dibromocarbazole (8.2 g), dibenzothiophene-4-boronic acid (12.1 g), and potassium phosphate (16.1 g) were suspended in 100 mL of toluene, 100 mL of ethanol, and 50 mL of water, and degassed by passing nitrogen gas through the solution. 2 mg of dichlorobis[di-t-butyl(p-dimethylaminophenyl)phosphino]palladium(II) was added to the reaction solution, and nitrogen gas was passed through the solution for additional 5 minutes. The reaction solution was heated under a nitrogen atmosphere and stirred under reflux for 3 hours. After cooling to room temperature, the resulting solid was filtered off and washed with toluene. The solid was dried in a vacuum dryer, and 12.8 g of compound S-1 was obtained.

The NMR measurement data of compound S-1 was as follows.

$^1H$ NMR(400MHz,$CDCl_3$,δ,ppm) 8.52-8.49(Ar,2H),8.29(brs,NH,1H),8.22-8.19(Ar,2H),8.18-8.15(Ar,2H),7.88-7.81(Ar,4H),7.64-7.56(Ar,6H),7.50-7.42(Ar,4H)

Compound S-1 (9.0 g), 2-(2-methacryloyloxyethyloxy)ethyl isocyanate (5.0 g), and tetrahydrofuran (THF) (90 mL) were mixed under a nitrogen atmosphere. Diazabicycloundecene (0.02 g) was added to this mixture and stirred for 3 hours.

After the reaction was completed, the reaction solution was poured into 50 mL of an aqueous solution of ammonium chloride, extracted with 200 mL of ethyl acetate, washed with 100 mL of saturated saline, then dried over anhydrous magnesium sulfate, filtered, and concentrated. The resulting solid was washed with a mixed solvent of hexane/ethyl acetate to obtain 10.0 g of compound M-1.

The NMR measurement data of compound M-1 was as follows.

$^1H$ NMR (400MHz,$CDCl_3$,δ,ppm) 8.47(brd,Ar,2H), 8.25-8.15(Ar,6H),7.90(dd,Ar,2H),7.83(Ar,2H),7.60(ar,4H),7.47(Ar,4H),6.32(brs,NH,1H),6.00(brs,C=$CH_2$,1H),5.40(dt,C=$CH_2$,1H),4. 37($CH_2$,2H),3.86($CH_2$,6H),1.81(brs,Me,3H)

<Production of holographic recording medium >

0.239 g of the compound M-1 used as the polymerizable monomer, 0.0096 g of a photopolymerization initiator HLI02, 3.30 mg of the radical scavenger TEMPOL, and 2.6 mg of light stabilizer LA-63P were dissolved in 2.53 g of DURANATE (registered trademark) TSS-100 to prepare solution A.

Separately, 1.73 g of PLACCEL PCL-205U and 0.74 g of PLACCEL PCL-305 (PLACCEL PCL-205U:PLACCEL PCL-305 = 70:30 (mass ratio)) were mixed, and 0.2 mg of an octylic acid solution of bismuth tris(2-ethylhexanoate) was dissolved in the mixture to thereby prepare solution B.

The solutions A and B were separately degassed at room temperature or 45°C under reduced pressure for 2 hours. Then 2.39 g of the solution A and 2.11 g of the solution B were mixed under stirring and further degassed in a vacuum for several minutes.

Then the vacuum degassed solution mixture was poured onto a microscope slide with 0.5 mm-thick spacer sheets placed on two opposite edges, and another microscope slide was placed thereon. Clips were used to fix the edges, and heating was performed at 80°C for 24 hours to produce a holographic recording medium as an evaluation sample. In this evaluation sample, a recording layer with a thickness of 0.5 mm was formed between the microscope slides used as covers.

In the holographic recording medium, the ratio of the number of isocyanate groups in the solution A to the number of isocyanate reactive groups in the solution B was 1.0. The holographic recording medium 2 contained 58.3 μmol/g of the polymerizable monomer, 3.05 μmol/g of the photopolymerization initiator, and 3.05 μmol/g of the radical scavenger.

<Holographic recording and evaluation >

Each of the holographic recording medium produced as the evaluation samples was used to perform holographic recording and evaluate the holographic recording performance of each holographic recording medium using procedures described below.

Holographic recording was performed using a semiconductor laser with a wavelength of 405 nm. An exposure device shown in Fig. 1 was used to perform two-beam plane-wave holographic recording at an exposure power density per beam of 10.2 $mW/cm^2$. The medium was rotated from -22.5° to 22.5°, and angular multiple recording was performed in the same position. The diffraction efficiency for each multiple recording operation was measured. The obtained diffraction efficiency was used to compute Δn, and the total over the entire multiple recording was used as the total Δn.

Details will next be described.

(Holographic recording)

Fig. 1 is a structural diagram showing the outline of the device used for holographic recording.

In Fig. 1, S represents a holographic recording medium sample, and M1 to M3 represent mirrors. PBS represents a polarizing beam splitter. L1 represents a recording laser light source emitting light with a wavelength of 405 nm (a single mode laser ("L1" in Fig. 1) manufactured by TOPTICA Photonics and capable of emitting light with a wavelength of about 405 nm). L2 represents a reproduction laser light source emitting light with a wavelength of 633 nm. PD1, PD2, and PD3 represent photodetectors. 1 represents an LED unit.

As shown in Fig. 1, a light beam with a wavelength of 405 nm was split using the polarizing beam splitter ("PBS" in the figure) into two beams intersecting on a recording surface such that the angle therebetween was 59.3°. In this case, the light beam was split such that a bisector of the angle between the two beams was perpendicular to the recording surface, and the two beams obtained by splitting the light beam were applied such that the vibration planes of the electric field

vectors of the two beams were perpendicular to a plane including the two intersecting beams.

After the holographic recording, a He-Ne laser capable of emitting light with a wavelength of 633 nm (V05-LHP151 manufactured by Melles Griot: "L2" in the figure) was used to apply the light to the holographic recording medium at an angle of 50.7°. The diffracted light was detected using a photo diode and a photosensor amplifier (S2281 and C9329: manufactured by Hamamatsu Photonics Co.,Ltd., "PD1" in the figure) to determine whether the holographic recording was correctly performed.

(Measurement of diffraction efficiency)

Multiple recording was performed while the sample was moved with respect to the optical axes such that the angle of the sample (the angle between the normal to the sample and a bisector of the interior angle of the two beams, i.e., incident light beams from the mirrors M1 and M2 in Fig. 1, at the intersection of the beams) was changed from -22.5° to 22.5°. Specifically, the multiple recording was performed 151 times while the angle of the sample was changed in steps of 0.3°.

After the multiple recording, the LED unit (1 in the figure, center wavelength: 405 nm) was turned on for a given period of time to completely consume the remaining initiator and the remaining monomer. This process is referred to as postexposure. The power of the LED was 100 $mW/cm^2$, and the irradiation was performed such that the integrated energy was 12 $J/cm^2$.

The diffraction efficiency of a hologram is given as the ratio of the intensity of the diffracted light to the sum of the intensity of the diffracted light and the intensity of the transmitted light. Light (wavelength: 405 nm) from the mirror M1 in Fig. 1 was directed to the sample, and the diffraction efficiency was measured at angles of -23° to 23°. $\Delta n$ was computed from the following formula in Coupled Wave Theory (H. Kogelnik, The Bell System Technical Journal (1969), 48, 2909-2947) using the obtained diffraction efficiency, and the total over the entire multiple recording was used as the total $\Delta n$.

[Math. 2]

$$\eta = \sin^2\left(\frac{\pi \cdot T \cdot \Delta n}{\lambda \cdot \cos\theta}\right)$$

$$total\Delta n = \sum \Delta n$$

Here, $\eta$ is the diffraction efficiency, and T is the thickness of the medium. $\lambda$ is the wavelength of the reference light, and $\theta$ is the incident angle of the reference light (29.65°).

A plurality of samples prepared were used. The evaluation was performed a plurality of times under different irradiation energy conditions, i.e., while the irradiation energy at the beginning of irradiation was increased or decreased and the total irradiation energy was increased or decreased. A search for conditions under which the polymerizable monomer was almost completely consumed (the total $\Delta n$ substantially reached equilibrium by the multiple recording) was performed in order to maximize the total $\Delta n$. The maximum value obtained was used as the total $\Delta n$ of the medium.

(Preparation of samples for measuring $\Delta E$ and absorption coefficient)

To measure $\Delta E$ and absorption coefficient, a holographic recording medium was prepared by exposing it to an energy of 36 $J/cm^2$ using an LED having a central wavelength of 405 nm manufactured by THORLABS.

(Measurement of $\Delta E$)

As a long-term performance stability test, the color index $\Delta E(00)$ of the fully exposed holographic recording medium was measured in a 10-degree visual field using a SE7700 spectrophotometer manufactured by Nippon Denshoku Industries Co., Ltd. before and after heating for 100 hours at 75°C in dry air in accordance with JIS: Z8722:2009.

As a reference, a sheet of EAGLE XG glass manufactured by Corning Com. was used.

These evaluation results are shown in Table 1 below.

[Example 2]

Compound M-2 was produced by the following synthesis method.

[Chem. 12]

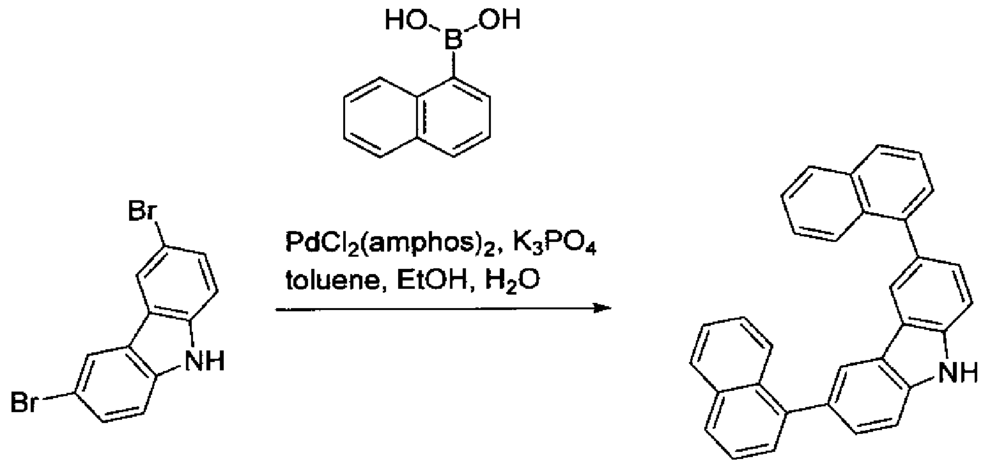


CompoundS-2

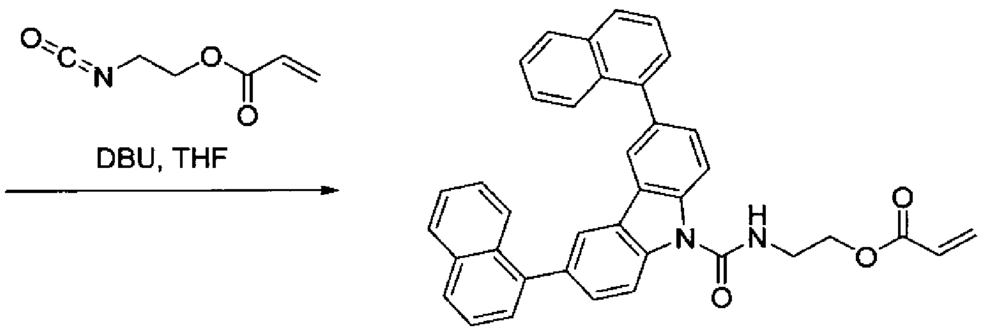


CompoundM-2

3,6-Dibromocarbazole (4.5 g), 1-naphthylboronic acid (5.2 g), and potassium phosphate (8.8 g) were suspended in 50 mL of toluene, 50 mL of ethanol, and 25 mL of water, and degassed by passing nitrogen gas through the solution. 10mg of dichlorobis[di-t-butyl (p-dimethylaminophenyl)phosphino]palladium(II) was added to the reaction solution, and nitrogen gas was passed through for additiomal 5 minutes. The reaction solution was heated under a nitrogen atmosphere and stirred under reflux for 3 hours. The resulting solid was filtered off, and the resulting solid was washed with water and ethanol. The solid was dried in a vacuum dryer, and 5.0 g of compound S-2 was obtained.

The NMR measurement data of compound S-2 were as follows.

$^1$H NMR(400MHz,$CDCl_3$,$\delta$,ppm) 8.25(brs,NH,1H),8.19(brs,Ar,2H), 8.00(brd,Ar,2H), 7.91(brd,Ar,2H),7.89-7.83(Ar,2H),7.59(brs,Ar,4H),7.57-7.51(Ar,4H),7.48(dd,Ar,2H),7.41(ddd,Ar)

Compound S-2 (1.0g), 2-isocyanatoethyl acrylate (0.4g), and THF (10mL) were mixed. Diazabicycloundecene (0.01g) was added to this mixture and stirred for 3 hours.

After the reaction was completed, the reaction solution was poured into 50 mL of an aqueous solution of ammonium chloride, extracted with 200 mL of ethyl acetate, washed with 100 mL of saturated saline, then dried over anhydrous magnesium sulfate, filtered, and concentrated. The resulting solid was washed with a mixed solvent of hexane/ethyl acetate to obtain 0.7 g of compound M-2.

The NMR measurement data of compound M-2 was as follows.

$^1$H NMR(400MHz,$CDCl_3$,$\delta$,ppm) 8.19-8.10(Ar,4H),7.97-7.82(Ar,6H),7.62(dd,Ar,2H),7.55-7.43(Ar,6H),7.39(Ar,2H),6.48(dd,CH=$CH_2$,1H),6.28(drt,NH,1H), 6.18(dd,CH=$CH_2$,1H),5.87(dd,CH=$CH_2$,1H),4.53(t,$CH_2$,2H),3.93(dt ,$CH_2$,2H)

A holographic recording medium was prepared and evaluated in the same manner as in Example 1, except that compound M-2 was used as the polymerizable monomer. The results are shown in Table 1 below.

[Example 3]

Compound M-3 was produced by the following synthesis method.

[Chem. 13]

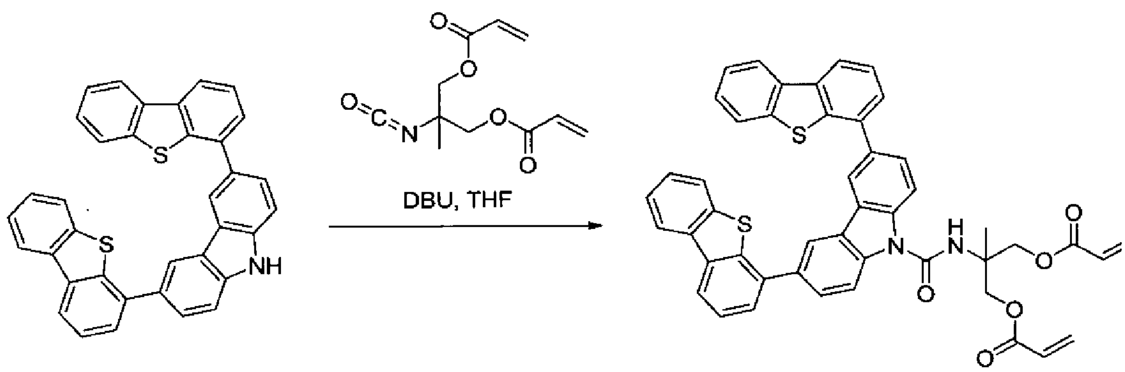


Compound S-1 Compound M-3

Under a nitrogen atmosphere, compound S-1 (4.7 g), 1,1-(bisacryloyloxymethyl)ethyl isocyanate (2.6 g), and THF (15 mL) were mixed. Diazabicycloundecene (0.04 g) was added to this mixture and stirred for 3 hours.

After the reaction was completed, the reaction solution was poured into 50 mL of an aqueous solution of ammonium chloride, extracted with 150 mL of ethyl acetate, washed with 20 mL of saturated saline, then dried over anhydrous magnesium sulfate, filtered, and concentrated. The resulting solid was purified by silica gel column chromatography to obtain 3.3 g of compound M-3.

The NMR measurement data of compound M-3 was as follows.

$^1H$ NMR(400MHz,$CDCl_3$,$\delta$,ppm) 8.38(brd,Ar,2H),8.17-8.09(Ar,6H),7.84(dd,Ar,2H),7.76(Ar,2H),7.57-7.49(Ar,4H),7.46-7.38(Ar,4H),6.48(dd,$CH=CH_2$,2H),6.35(s,NH,1H),6.19(dd,$CH=CH_2$ ,2H),5.89(dd,$CH=CH_2$,2H),4.69(d,$CH_2$,2H),4.63(d,$CH_2$,2H),1.76(s ,Me, 3H)

A holographic recording medium was prepared and evaluated in the same manner as in Example 1, except that compound M-3 was used as the polymerizable monomer. The results are shown in Table 1 below.

[Synthesis Example 1]

Compound M-4 was produced by the following synthesis method.

[Chem. 14]

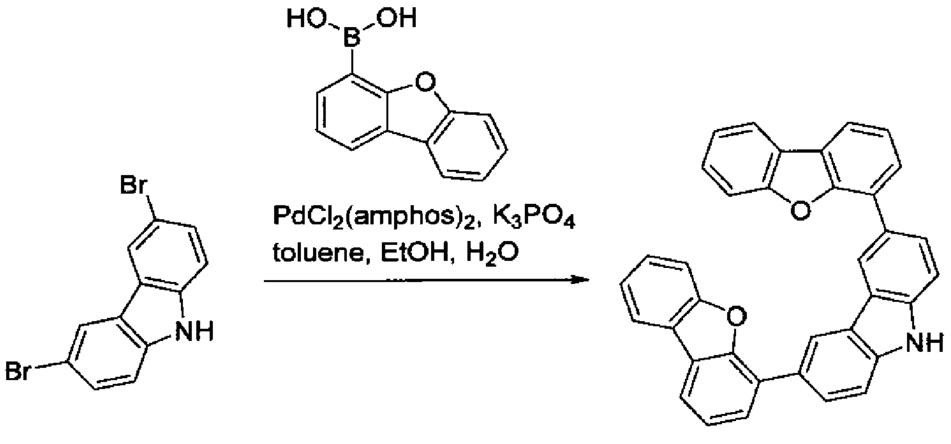


Compound S-3

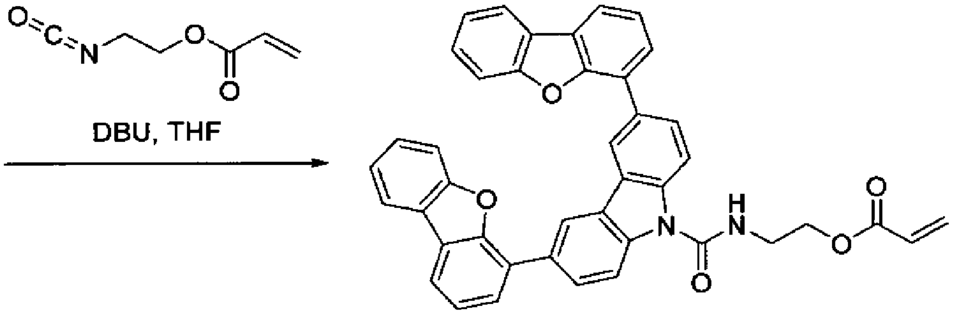


Compound M-4

3,6-Dibromocarbazole (3.5 g), 4-dibenzofuranboronic acid (5.0 g), and potassium phosphate (6.9 g) were suspended in 50 mL of toluene, 50 mL of ethanol, and 25 mL of water, and degassed by passing nitrogen gas through the suspension. 2 mg of dichlorobis[di-t-butyl(p-dimethylaminophenyl)phosphino]palladium(II) was added to the reaction solution, and nitrogen gas was passed through the solution for additional 5 minutes. The reaction solution was heated under a nitrogen atmosphere and stirred under reflux for 3 hours. The resulting solid was filtered off and washed with water and ethanol. The solid was dried in a vacuum dryer to obtain 5.0 g of compound S-3.

The NMR measurement data of compound S-3 was as follows.

$^1$H NMR(400MHz,$CDCl_3$,$\delta$,ppm)
8.69(brd,Ar,2H),8.26(brs,NH,1H),8.04(dd,Ar,2H),8.02(ddd,Ar, 2H),7.95(dd,Ar,2H),7.76-7.72(Ar,2H),7.66-7.61(Ar,4H),7.50 7.44(Ar,4H),7.37(ddd,Ar,2H)

Compound S-3 (2.0 g), 2-isocyanatoethyl acrylate (0.6 g), and THF (10 mL) were mixed under a nitrogen atmosphere. Diazabicycloundecene (0.02 g) was added to this mixture and stirred for 3 hours. After the reaction was completed, the reaction solution was poured into 50 mL of an aqueous solution of ammonium chloride, extracted with 200 mL of ethyl acetate, washed with 100 mL of saturated saline, then dried over anhydrous magnesium sulfate, filtered, and concentrated. The resulting solid was washed with a mixed solvent of hexane/ethyl acetate to obtain 1.2 g of compound M-4.

The NMR measurement data of compound M-4 was as follows.

$^1$H NMR(400MHz,$CDCl_3$,$\delta$,ppm)
8.65(brd,Ar,2H),8.22(d,Ar,2H),8.08(dd,Ar,2H),8.02(brd,Ar,2H ),7.97(dd,Ar,2H),7.73(dd,Ar,2H),7.63(d,Ar,2H),7.51-7.44 (Ar, 4H), 7.38 (ddd,Ar,2H),6.52(dd,CH=$CH_2$,1H),6.24(brt,NH, 1H),6.22(dd,CH=$CH_2$,1H),5.93(dd,CH=$CH_2$,1H),4.57(brt,$CH_2$,2H)3 .98(brq,$CH_2$,2H)

[Synthesis Example 2]

Compound M-5 was produced by the following synthesis method.

[Chem. 15]

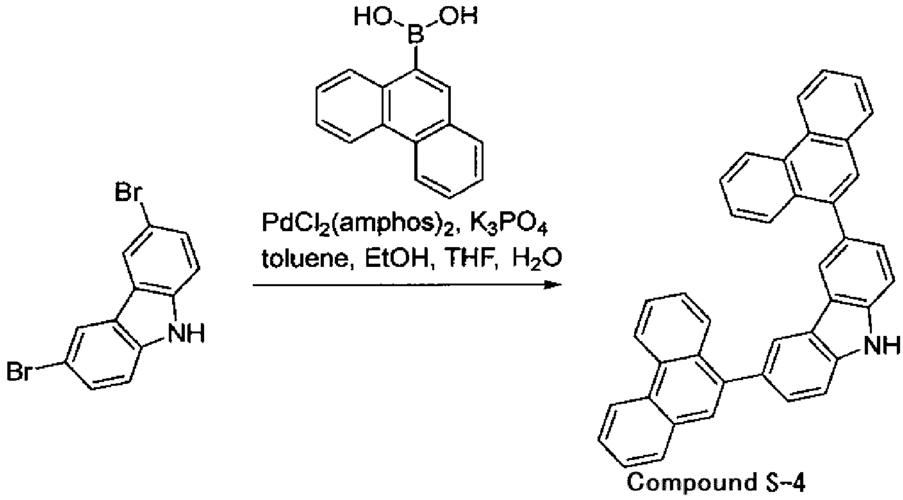


Compound S-4

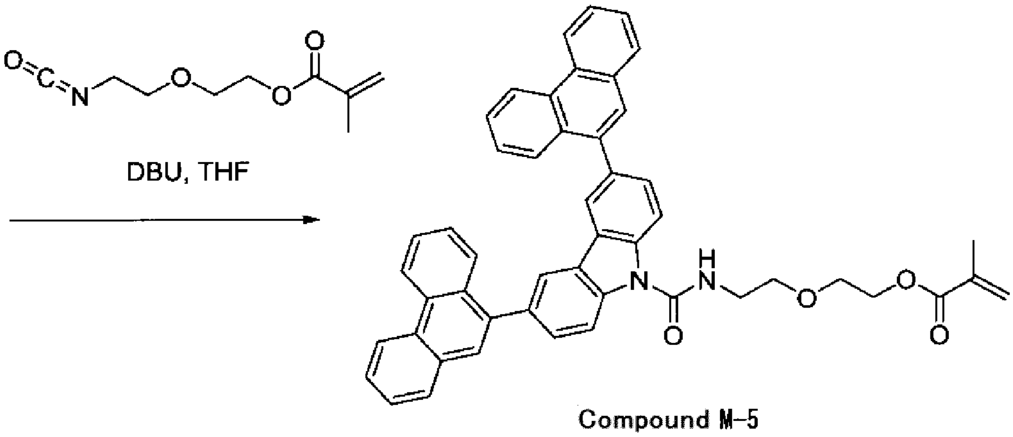


Compound M-5

3,6-Dibromocarbazole (3.5 g), 9-phenanthreneboronic acid (5.3 g), and potassium phosphate (6.9 g) were suspended in 50 mL of toluene, 25 mL of ethanol, 25 mL of tetrahydrofuran, and 25 mL of water, and degassed by passing nitrogen gas through the solution. 2 mg of dichlorobis[di-t-butyl (p-dimethylaminophenyl)phosphino]palladium(II) was added to the reaction solution, and nitrogen gas was passed through the solution for additional 5 minutes. The reaction solution was heated under a nitrogen atmosphere and stirred under reflux for 3 hours. After cooling to room temperature, 50 mL of 1N aqueous sodium hydroxide solution and 100 mL of ethyl acetate were added, and a separation operation was performed. The obtained organic layer was dried over magnesium sulfate, and the solvent was distilled off. The resulting solid was filtered off and washed with hexane. The solid was dried in a vacuum dryer, and 4.2 g of compound S-4 was obtained.

The NMR measurement data of compound S-4 was as follows.

$^1$H NMR(400MHz,CDCl$_3$,$\delta$,ppm)
8.79(brd,Ar,2H),8.73(brd,Ar,2H),8.29(brs,NH,1H),8.27(brs,Ar ,2H),8.03(dd,Ar,2H),7.90(dd,Ar,2H),7.79(brs,Ar,2H),7.69-7.58(Ar,10H),7.53(Ar,2H)

Compound S-4 (1.0 g), 2-(2-methacryloyloxyethyloxy)ethyl isocyanate (0.75 g), and THF (10 mL) were mixed under a nitrogen atmosphere. Diazabicycloundecene (0.02 g) was added to this mixture and stirred for 3 hours.

After the reaction was completed, the reaction solution was poured into 50 mL of an aqueous solution of

ammonium chloride, extracted with 200 mL of ethyl acetate, washed with 100 mL of saturated saline, then dried over anhydrous magnesium sulfate, filtered and concentrated. The resulting mixture was purified by column chromatography to obtain 1.1 g of compound M-5.

The NMR measurement data of compound M-5 was as follows.

$^1$H NMR(400MHz,$CDCl_3$,δ,ppm)
8.75(d,Ar,2H),8.69(d,Ar,2H),8.22(d,Ar,2H),8.20(brs,Ar,2H),7 .97(d,Ar,2H),7.87(d,Ar,2H),7.75(s,Ar,2H),7.71-7.54(Ar,8H),7.51(dd,Ar,2H),6.38(drt,NH,1H),6.01(s,C=$CH_2$,1H) ,5.39(brs,C=$CH_2$,1H),4.34(dd,$CH_2$,2H),3.84(brs,$CH_2$,4H),3.81(dd ,C-$H_2$,2H),1.80(brs,Me,3H)

[Synthesis Example 3]

Compound M-6 was produced by the following synthesis method.

[Chem. 16]

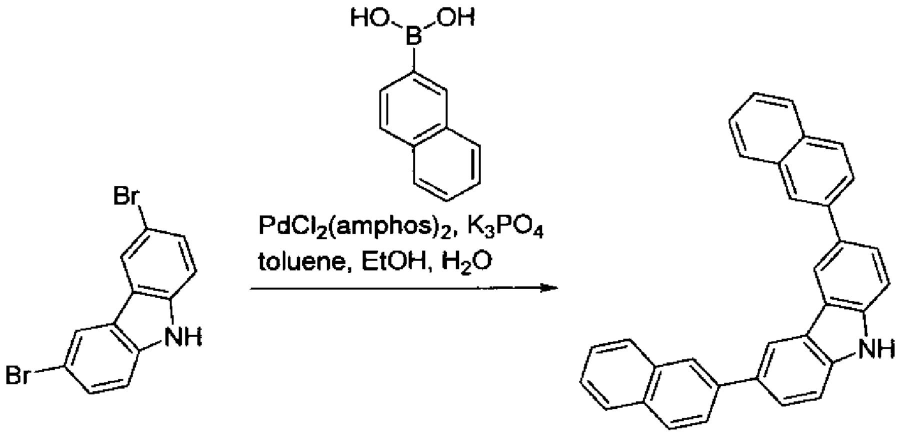


Compound S-5

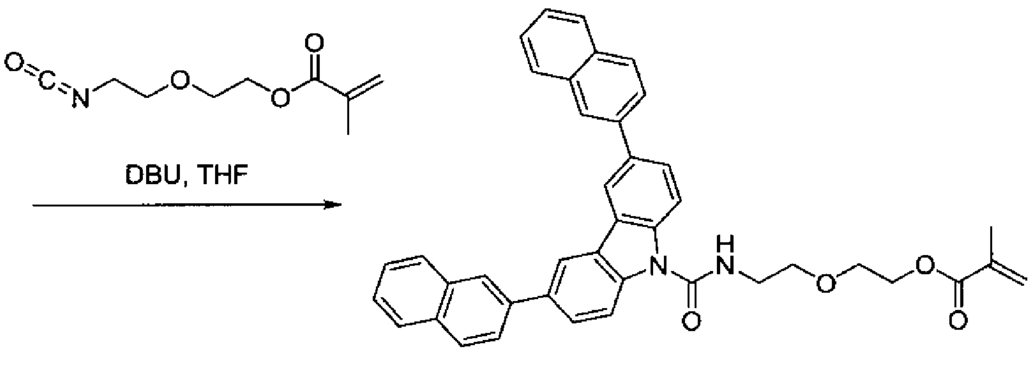


Compound M-6

3,6-Dibromocarbazole (4.5 g), 2-naphthylboronic acid (5.2 g), and potassium phosphate (8.8 g) were suspended in 50 mL of toluene, 50 mL of ethanol, and 25 mL of water, and degassed by passing nitrogen gas through the suspension. 10 mg of dichlorobis[di-t-butyl(p-dimethylaminophenyl)phosphino]palladium(II) was added to the reaction solution, and nitrogen gas was passed through for additional 5 minutes. The reaction solution was heated under a nitrogen atmosphere and stirred under reflux for 3 hours. The resulting solid was filtered off and washed with water and ethanol. The solid was dried in a vacuum dryer, and 5.8 g of compound S-5 was obtained.

The NMR measurement data of compound S-5 was as follows.

$^1H$ NMR(400MHz,$CDCl_3$,δ,ppm)
8.51 (brs,Ar,2H),8.21 (brs,NH, 1H), 8.16 (brs, Ar, 2H), 7.97-7.86(Ar,8H),7.83(dd,Ar,2H),7.56(brd,Ar,2H),7.54-7.46(Ar,4H)

Compound S-5 (1.0 g), 2-(2-methacryloyloxyethyloxy)ethyl isocyanate (0.7 g), and THF (5 mL) were mixed under a nitrogen atmosphere. Diazabicycloundecene (0.02 g) was added to this mixture and stirred for 3 hours.
After the reaction was completed, the reaction solution was poured into 50 mL of an aqueous solution of ammonium chloride, extracted with 200 mL of ethyl acetate, washed with 100 mL of saturated saline, then dried over anhydrous magnesium sulfate, filtered, and concentrated. The resulting solid was washed with a mixed solvent of hexane/ethyl acetate to obtain 1.2 g of compound M-6.
The NMR measurement data of compound M-6 was as follows.

$^1H$ NMR(400MHz,$CDCl_3$,δ,ppm)
8.23(brd,Ar,2H),8.05(brs,Ar,2H),7.99(d,Ar,2H),7.89-7.80(Ar,6H),7.78(dd,Ar,2H),7.72(dd,Ar,2H),7.50-7.42(Ar,4H),6.28(brs,NH,1H),5.99(brt,C=$CH_2$,1H),5.37(brt,C=C $H_2$,1H),4.33-4.29($CH_2$,2H),3.78-3.70($CH_2$,6H),1.79(brs,Me,3H)

[Synthesis Example 4]

Compound M-7 was produced by the following synthesis method.

[Chem. 17]

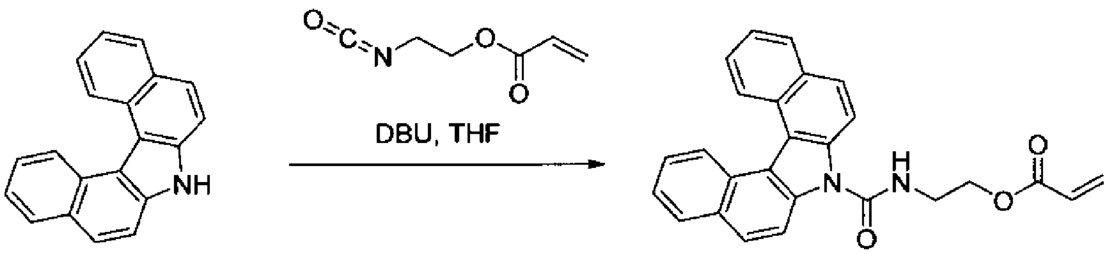


Compound M-7

1.3 g of 7H-dibenzo[c,g]carbazole, 2-isocyanatoethyl acrylate (1.0 g), and THF (10 mL) were mixed. Diazabicycloundecene (0.01 g) was added to this mixture and stirred for 3 hours.
After the reaction was completed, the reaction solution was poured into 50 mL of an aqueous solution of ammonium chloride, extracted with 200 mL of ethyl acetate, washed with 100 mL of saturated saline, then dried over anhydrous magnesium sulfate, filtered, and concentrated. The resulting solid was washed with a mixed solvent of hexane/ethyl acetate to obtain 1.3 g of compound M-7.
The NMR measurement data of compound M-7 was as follows.

$^1H$ NMR(400MHz,$CDCl_3$,δ,ppm)
9.09(d,Ar,2H),8.21(d,Ar,2H),8.04(brd,Ar,2H),7.93(d,Ar,2H),7 .68(ddd,Ar,2H),7.57(dd,Ar,2H),6.50(dd,CH=CH,1H),6.24(drt,N H,1H),6.19(dd,CH=$CH_2$,1H),5.92(dd,CH=$CH_2$,1H),4.55(t,$CH_2$,2H),3 .97 (dt,$CH_2$,2H)

[Synthesis Example 5]

Compound M-8 was produced by the following synthesis method.

[Chem. 18]

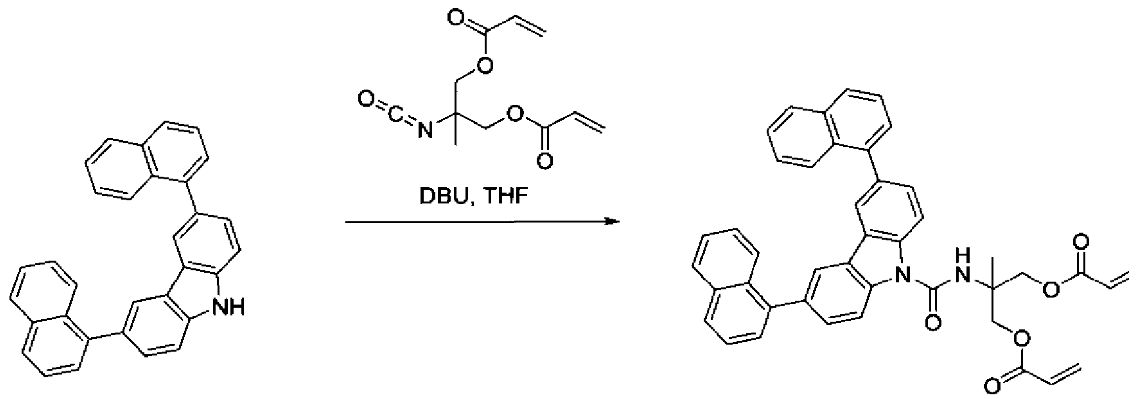


Compound S-2 Compound M-8

Compound S-2 (2.0 g), 1,1-(bisacryloyloxymethyl)ethyl isocyanate (1.4 g), and THF (5 mL) were mixed under a nitrogen atmosphere. Diazabicycloundecene (0.04 g) was added to this mixture and stirred for 3 hours.

After the reaction was completed, the reaction solution was poured into 50 mL of an aqueous solution of ammonium chloride, extracted with 100 mL of ethyl acetate, washed with 20 mL of saturated saline, then dried over anhydrous magnesium sulfate, filtered, and concentrated. The resulting solid was purified by silica gel column chromatography to obtain 1.2 g of compound M-8.

The NMR measurement data of compound M-8 was as follows.

$^1$H NMR(400MHz,$CDCl_3$,$\delta$,ppm) 8.17-8.13(Ar,4H),7.96-7.86(Ar,6H),7.63(dd,Ar,2H),7.58-7.47(Ar,6H),7.45-7.40(Ar,2H),6.49(dd,CH=$CH_2$,2H),6.25(s,NH,1H),6.20(dd,CH=$CH_2$ ,2H),5.91(dd,CH=-$CH_2$,2H),4.70(d,$CH_2$,2H),4.64(d,$CH_2$,2H),1.78(s ,Me,3H)

[Synthesis Example 6]

Compound M-9 was produced by the following synthesis method.

[Chem. 19]

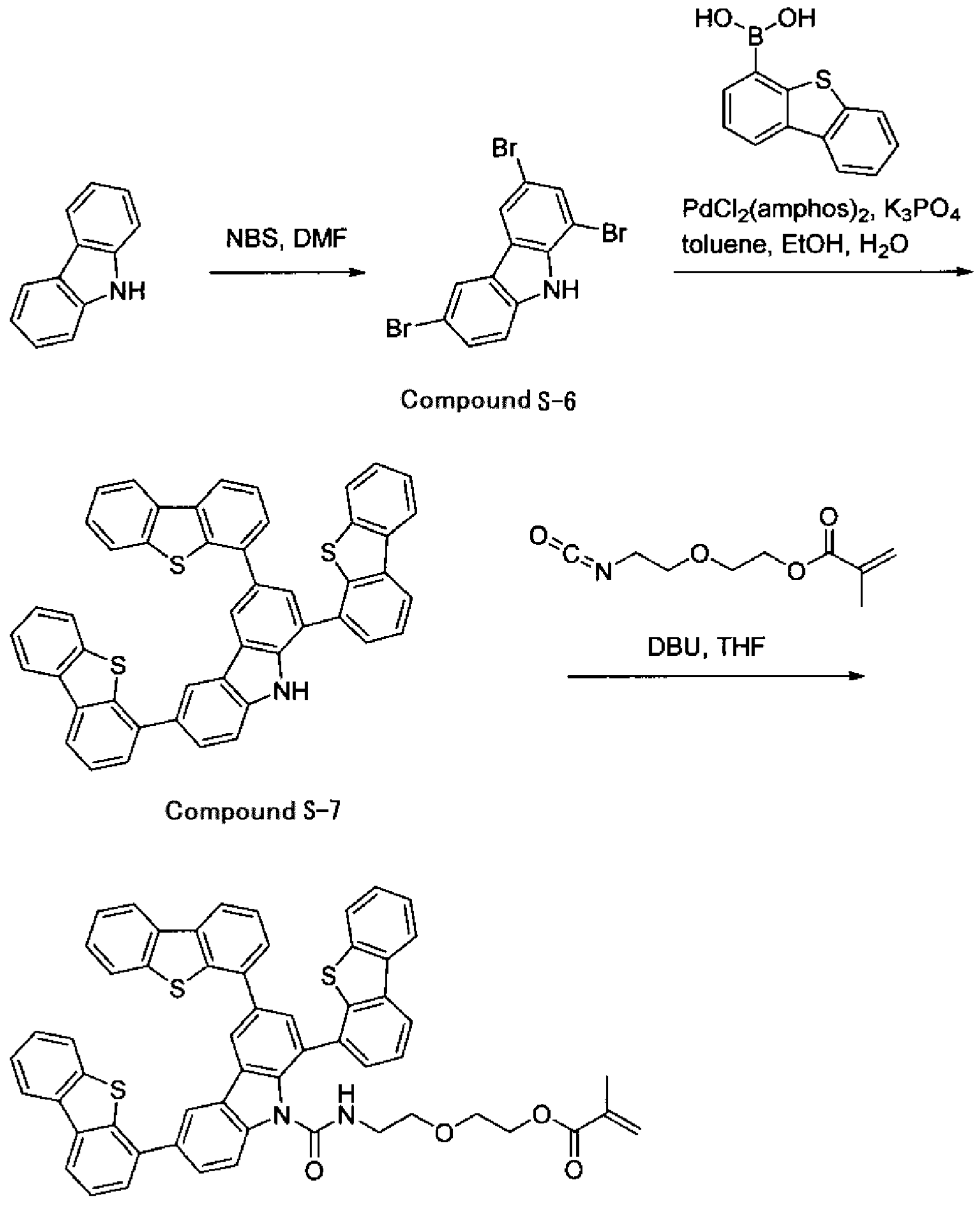


Compound S-6

Compound S-7

Compound M-9

5.0 g of carbazole was dissolved in 50 mL of dimethylformamide and cooled to 0 degrees. 17 g of N-bromosuccinimide was slowly added to this solution, and the mixture was stirred at 0 degrees for 30 minutes and then left to stand at room temperature overnight. The reaction mixture was poured into 200 mL of water, and the resulting solid was filtered off and washed with water. The resulting crude product was suspended and washed in dichloromethane, and the solid obtained by filtration was washed with dichloromethane to obtain 10 g of compound S-7.

The NMR measurement data of compound S-6 was as follows.

$^1$H NMR(400MHz,$CDCl_3$,δ,ppm)
8.28(brs,NH,1H),8.12(d,Ar,1H),8.08(d,Ar,1H),7.72(d,Ar,1H),7 .57(dd, Ar, 1H), 7.38 (d,Ar, 1H)

Compound S-6 (3g), dibenzothiophene-4-boronic acid (5.6g), and potassium phosphate (7.9g) were suspended in 25mL of toluene, 25mL of ethanol, and 15mL of water, and degassed by passing nitrogen gas through the solution. 53mg of dichlorobis[di-t-butyl (p-dimethylaminophenyl)phosphino]palladium(II) was added to the reaction solution, and nitrogen gas was passed through for additional 10 minutes. The reaction solution was heated under a nitrogen atmosphere and stirred under reflux for 6 hours. After cooling to room temperature, it was extracted with ethyl acetate and washed with water. The aqueous layer was extracted twice with ethyl acetate, and 0.4 g of activated carbon was added to the resulting organic layer and stirred for 30 minutes. After filtration through Celite, the organic layer was concentrated to obtain 4.6 g of

crude compound S-7.

The NMR measurement data of compound S-7 was as follows.

$^1$H NMR(400MHz,$CDCl_3$,δ,ppm)
8.62 (brd,Ar,1H),8.56 (brs,Ar, 1H),8.32-8.25 (Ar, 3H), 8.23-8.18(Ar,2H),8.18-8.14(Ar,2H),8.09(d,Ar,1H),7.87-7.77(Ar,NH,4H),7.72-7.67(Ar,2H),7.64-7.56(Ar,3-H),7.55-7.41(Ar,7H)

Compound S-7 (4.3 g), 2-(2-methacryloyloxyethyloxy)ethyl isocyanate (1.8 g), and THF (6 mL) were mixed under a nitrogen atmosphere. Diazabicycloundecene (0.04 g) was added to this mixture and stirred for 3 hours.

After the reaction was completed, the reaction solution was poured into 50 mL of an aqueous solution of ammonium chloride, extracted with 200 mL of ethyl acetate, washed with 100 mL of saturated saline, then dried over anhydrous magnesium sulfate, filtered and concentrated. The resulting solid was washed with a mixed solvent of hexane/ethyl acetate to obtain 3.7 g of compound M-9.

The NMR measurement data of compound M-9 was as follows.

$^1$H NMR(400MHz,$CDCl_3$,δ,ppm)
8.64(d,Ar,1H),8.54(d,Ar,1H),8.26-8.14(Ar,7H),8.11(d,Ar,1H),7.91(dd,Ar,1H),7.87-7.83(Ar,3H),7.72-7.58(Ar,6H),7.54-7.43 (Ar,6H), 5.94(brs,C=$CH_2$,1H),5.56(t,NH,1H),5.41(C=$CH_2$,1H) ,4.04-3.99($CH_2$,2H),3.24(brs,$CH_2$,1H),3.15-3.04($CH_2$,2-H),2.96(brs,$CH_2$,1H),2.67(brs,$CH_2$,1H),2.35(brs,$CH_2$, 1H),1.79(brt,Me,3H)

[Comparative Example 1]

Compound R-1 was produced by the following synthesis method.

[Chem. 20]

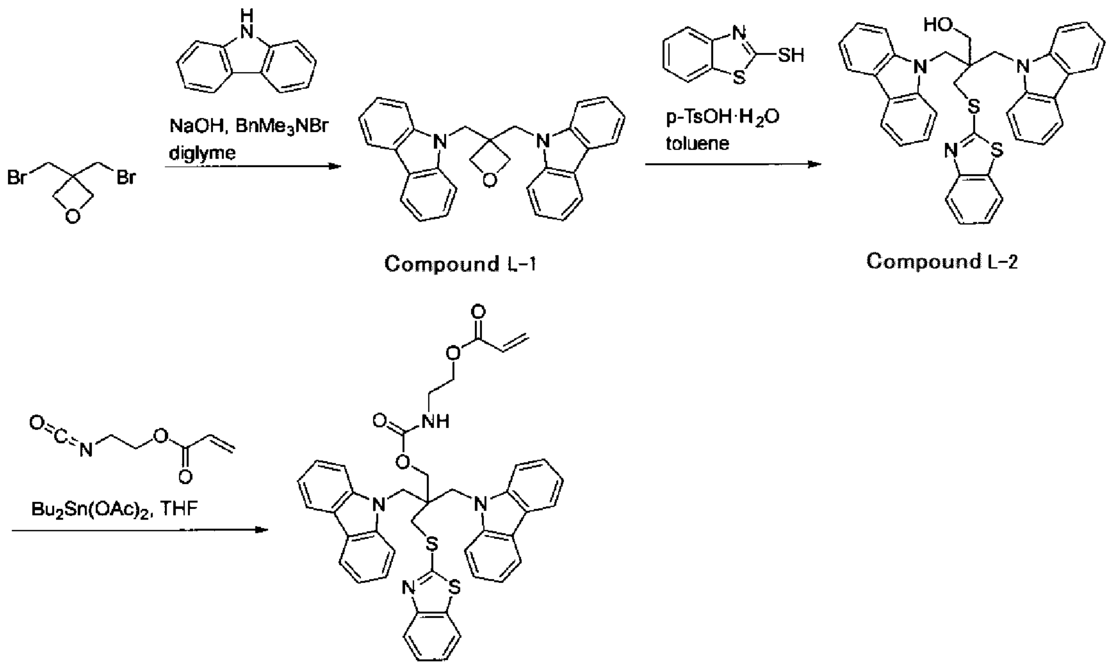


Compound L-1

Compound L-2

Compound R-1

9H-carbazole (1.4 g), bis(bromomethyl)oxetane (1.0 g), sodium hydroxide (0.66 g), and benzyltrimethylammonium bromide (20 mg) were suspended in 5 mL of diethylene glycol dimethyl ether (diglyme). The reaction mixture was heated to 120°C and stirred for 3 hours. After cooling to room temperature, the resulting white solid was filtered off, washed with methanol, and dried under reduced pressure to obtain 1.3 g of compound L-1.

The NMR measurement data of compound L-1 was as follows.

$^1$H NMR(400MHz,$CDCl_3$,δ,ppm)
8.13(brd,4H),7.43(Ar,4H),7.27(Ar,8H),4.69(s,4H),4.64(s,4H)

Compound L-1 (5.3g), 2-mercaptobenzothiazole (2.1g), and toluenesulfonic acid monohydrate (100mg) were suspended in 30mL of toluene. Under a nitrogen atmosphere, the reaction solution was heated to 120°C and stirred under reflux for 1 hour. After cooling the mixture to room temperature, it was extracted with ethyl acetate and washed with 1M aqueous sodium hydroxide solution. The aqueous layer was re-extracted with ethyl acetate, and the combined organic layers were concentrated. The obtained crude product was washed with a small amount of methanol to obtain 6.9g of compound L-2.

The NMR measurement data of compound L-2 was as follows.

$^1$H NMR(400MHz,$CDCl_3$,δ,ppm)
8.11 (brd, 4H), 7.82 (d,1H),7.61(d, 1H), 7.44 (Ar, 9H), 7.25 (Ar, 5H), 5.20(t,OH,1H),44.88(d,2H),4.50(d,2H),4.18(d,2H),3.89(s,2H)

Compound L-2 (3.8g) was dissolved in 30mL of tetrahydrofuran, and 28mg of dibutyltin diacetate was added. 2-isocyanatoethyl acrylate (1.0g) was added to this solution, and the reaction was carried out at room temperature. After 48 hours, a saturated aqueous solution of sodium bicarbonate was added to the reaction solution, and the solution was extracted with 50mL of chloroform. The organic layer obtained was dried over magnesium sulfate and concentrated at 30°C or less. The crude product obtained by the above concentration was purified using a silica gel column (hexane/ethyl acetate) to obtain 3.4 g of compound R-1.

The NMR measurement data of compound R-1 was as follows.

$^1$H NMR(400MHz,$CDCl_3$,δ,ppm)
8.08(d,4H),7.80(d,1H),7.72(d,1H),7.39(Ar,9H),7.29(Ar,1H),7.23(Ar,4H),6.42(d,1H),6.13(dd,1H),5.86(d,1H),4.73(d,2H),4.68 (d,2H),4.43(s,2H),4.40(t,OH,1H),4.06(t,2H),3.99(s,2H),3.75( m,2H),3.24(m,2H)

A holographic recording medium was prepared and evaluated in the same manner as in Example 1, except that compound R-1 was used as the polymerizable monomer. The results are shown in Table 1 below.

[Comparative Example 2]

Compound R-2 was produced by the following synthesis method.

[Chem. 21]

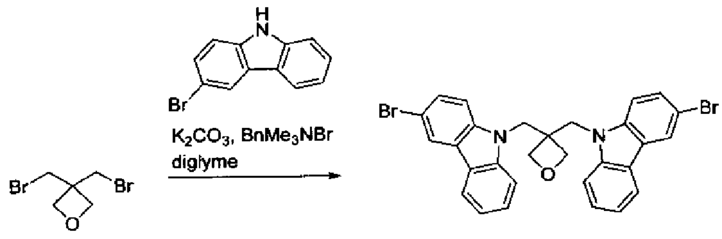


Compound L-3

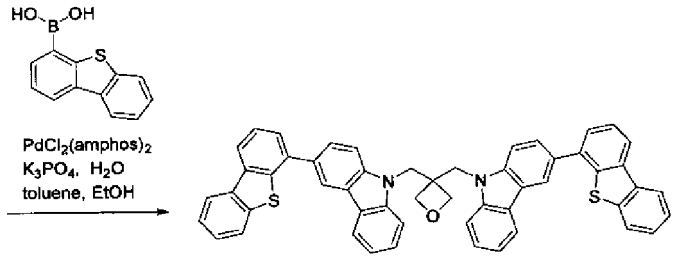


Compound L-4

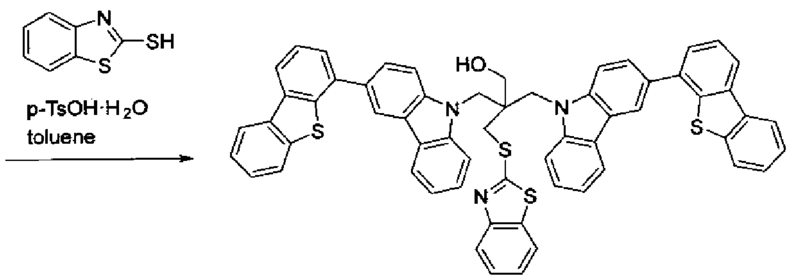


Compound L-5

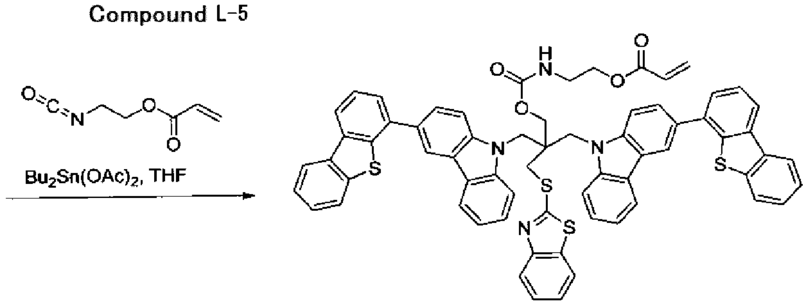


Compound R-2

3-Bromo-9H-carbazole (5.1 g), bis(bromomethyl)oxetane (2.5 g), potassium carbonate (2.9 g), and benzyltrimethylammonium bromide (0.1 g) were suspended in 5 mL of diethylene glycol dimethyl ether (diglyme). The reaction mixture was heated to 120°C and stirred for 3 hours. After cooling to room temperature, the resulting solid was filtered, washed with a mixed solvent of ethyl acetate/hexane, and dried under reduced pressure to obtain 5.2 g of compound L-3.

The NMR measurement data of compound L-3 was as follows.

$^1$H NMR(400MHz,$CDCl_3$,δ,ppm)
8.23(d,2H),8.07(d,2H),7.50(dd,2H),7.45(Ar,2H),7.29(d,2H),7. 26(d,2H),7.14(d,2H),4.63(s,4H),4.58(s,4H)

Compound L-3 (3.5g), dibenzothiophene-4-boronic acid (2.8g), and potassium phosphate (5.2g) were suspended in 60mL of toluene, 30mL of ethanol, and 30mL of water, and degassed by passing nitrogen gas through the solution. 31mg of dichlorobis[di-t-butyl (p-dimethylaminophenyl)phosphino]palladium(II) was added to the reaction solution, and nitrogen gas was passed through the solution for additional 10 minutes. The reaction solution was heated under a nitrogen atmosphere and stirred under reflux for 6 hours. After cooling to room temperature, it was extracted with

ethyl acetate and washed with water. The aqueous layer was extracted twice with ethyl acetate, and 0.4 g of activated carbon was added to the resulting organic layer and stirred for 30 minutes. After filtration through Celite, the crude product obtained after concentration was purified on a silica gel column (hexane/ethyl acetate) to obtain 4.3 g (90% yield) of compound L-4.

The NMR measurement data of compound L-4 was as follows.

$^1$H NMR(400MHz,$CDCl_3$,δ,ppm)
8.51(d,2H),8.19(Ar,6H),7.83(Ar,4H),7.59(d,4H),7.47(Ar,8H),7 .38(d,2H),7.32(t,2H),4.81(s,4H),4.77(s,4H)

Compound L-4 (4.8g), 2-mercaptobenzothiazole (1.1g), and toluenesulfonic acid monohydrate (106mg) were suspended in 30mL of toluene. Under a nitrogen atmosphere, the reaction solution was heated to 100°C and stirred for 5 minutes. After cooling to room temperature, the mixture was extracted with ethyl acetate and washed with 1M aqueous sodium hydroxide solution. The aqueous layer was reextracted with ethyl acetate, and the combined organic layers were concentrated. The resulting crude product was purified using a silica gel column (hexane/ethyl acetate) to obtain 3.8 g (65% yield) of compound L-5.

The NMR measurement data of compound L-5 was as follows:

$^1$H NMR(400MHz,$CDCl_3$,δ,ppm)
8.49(d,2H),8.19(Ar,6H),7.85(Ar,2H),7.80(Ar,2H),7.66(Ar,2H),
7.58(Ar,7H),7.46(Ar,9H),7.29(t,2H),4.98(d,2H),4.62(d,2H),4. 27(s,2H),3.99(s,2H)

Compound L-5 (3.8g) was dissolved in 30mL of tetrahydrofuran, and dibutyltin diacetate (60mg) was added. 2-Isocyanatoethyl acrylate (0.9g) was added to this solution and reacted at room temperature. After 48 hours had passed, a saturated aqueous solution of sodium bicarbonate was added to the reaction solution, and the solution was extracted with 50 mL of chloroform. The resulting organic layer was dried over magnesium sulfate and then concentrated at 30°C or lower. The crude product obtained by the concentration was purified using a silica gel column (hexane/ethyl acetate) to obtain 3.4 g (78% yield) of compound R-2.

The NMR measurement data of compound R-2 was as follows.

$^1$H NMR(400MHz,$CDCl_3$,δ,ppm)
8.47(d,2H),8.17(Ar,6H),7.82(Ar,4H),7.73(Ar,2H),7.58(Ar,6H), 7.44 (Ar, 10H),7.29(t,2H),6.36(d,1H),6.08(dd, 1H), 5.74 (d, 1H), 4 .82(s,4H),4.61(brt,NH,1H),4.55(s,2H),4.11(m,4H),3.32(m,2H)

A holographic recording medium was prepared and evaluated in the same manner as in Example 1, except that compound R-2 was used as the polymerizable monomer. The results are shown in Table 1 below.

[Table 1]

| | Polymerizable monomer | Number of times of multiple recording | totalΔn | ΔE(00) before heating | ΔE(00) after heating at 75°C for 100 hours |
|---|---|---|---|---|---|
| Example 1 | M-1 | 151 | 0.0206 | 1.1 | 1.4 |
| Example 2 | M-2 | 151 | 0.0160 | 1.5 | 1.4 |
| Example 3 | M-3 | 151 | 0.0166 | 1.7 | 2.2 |
| Comparative Example 1 | R-1 | 151 | 0.0157 | 1.2 | 3.8 |
| Comparative Example 2 | R-2 | 151 | 0.0209 | 1.5 | 4.1 |

To produce each of the holographic recording mediums, the molar concentrations of the polymerizable monomer, the photopolymerization initiator, and the additives were fixed to their specific values, and only the type of polymerizable monomer was changed to produce and evaluate the holographic recording medium (Table 1).

As shown in Table 1, in Comparative Examples 1 and 2, in which the polymerizable monomer used had an aromatic amino group not linked by a carbonyl bond, the color index ΔE after heating at 75°C for 100 hours was 3.8 or more, whereas in Examples 1 to 3, in which a polymerizable monomer in which a carbonyl group was bonded to a nitrogen atom of an aromatic amino compound was used, the color index ΔE after heating at 75°C for 100 hours was 3.0 or less.

In optical element applications such as light guide plates for AR glasses, the higher the total Δn of the holographic

recording medium, the brighter the projected image, and the wider the viewing angle. In memory applications, increasing the total Δn can increase the recording capacity. On the other hand, holographic recording media having high coloring, especially in AR glass waveguide plate applications, absorb the guided light, and this causes deterioration in the efficiency of light utilization and aesthetic quality. In addition, when the color tone of the waveguide plate changes over time, the absorption intensity of the guided light also changes, and this causes to a decrease in the performance stability of the waveguide plate. Therefore, by using the compound of the present invention which has a high total Δn, low coloring, and low color tone change, it is possible to create an AR glass waveguide plate having excellent light utilization efficiency and long-term performance stability.

As described above, the compounds of the invention used in the Examples are superior to the compounds in the Comparative Examples.

[Measurement of refractive index]

The refractive index of each of the polymerizable monomers produced in the Examples and Comparative Examples was measured using the following method. The results are shown in Table 2.

A test solution was prepared by dissolving a sample in a solution mixture of 3-phenoxybenzyl acrylate and trimethylolpropane trimethacrylate with a mass ratio of 4:1 such that a prescribed concentration was obtained. Specifically, two test solutions with sample concentrations of 10% by mass and 20% by mass were prepared.

The refractive index of each test solution was measured using a Kalnew precision refractometer (product name: KPR-2000 manufactured by Shimadzu Corporation). The temperature of the test solution was 23°C, and the measurement wavelength used was the d line (587.6 nm) of a helium lamp. A calibration curve representing the correlation between the sample concentration and the refractive index was produced based on the measurement results. Using the obtained calibration curve, the refractive index at a sample concentration of 100% by mass was determined and used as the refractive index of the sample.

[Table 2]

| | Polymerizable Monomer | Refractive index |
|---|---|---|
| Example 1 | M-1 | 1.703 |
| Example 2 | M-2 | 1.685 |
| Example 3 | M-3 | 1.688 |
| Comparative Example 1 | R-1 | 1.664 |
| Comparative Example 2 | R-2 | 1.701 |

As can be seen from Table 2, the value of the refractive index of each of the compounds in the Examples is 1.65 or more, and each of these compounds has a refractive index high enough for a high refractive index monomer. Since the compounds in the Examples have refractive indexes equal to or higher than those of the Comparative Examples, it can be seen that the compounds in the Examples have improved colorability when heated while maintaining a high refractive index, and are excellent high refractive index materials having high compound stability compared to the compounds in the Comparative Examples.

Although the present invention has been described in detail by way of the specific modes, it is apparent for those skilled in the art that various changes can be made without departing from the spirit and scope of the present invention.

The present application is based on Japanese Patent Application No. 2022-106252 filed on June 30, 2022, the entire contents of which are incorporated herein by reference.

Reference Signs List

S holographic recording medium

M1, M2, M3 mirror

L1 semiconductor laser light source for recording light

L2 laser light source for reproduction light

PD1, PD2, PD3 photodetector

PBS polarizing beam splitter

1 LED unit

## Claims

1. A compound represented by the following formula (1):

[Chem. 1]

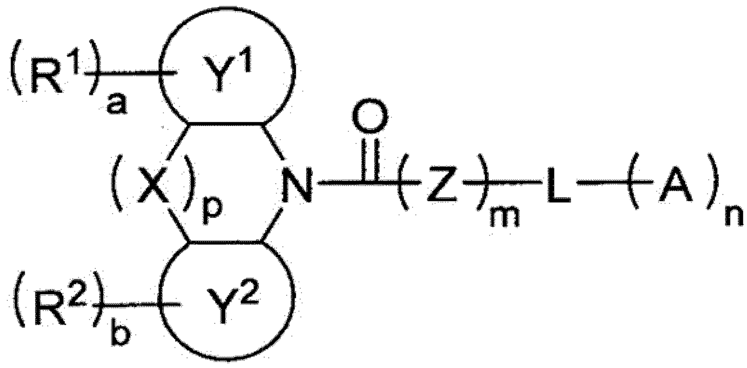

(1)

[In the formula, A represents a polymerizable group. n represents an integer of 1 to 3.

L represents an optionally branched (n+1)-valent linking group.
Z represents an oxygen atom, or a nitrogen atom optionally having a substituent. m represents an integer of 0 or 1.
X represents a single bond, an oxygen atom, a sulfur atom, a carbon atom optionally having a substituent, or a nitrogen atom optionally having a substituent. p represents an integer of 0 to 2. When p is 2, the two Xs may be the same or different.
$Y^1$ and $Y^2$ are each independently a benzene ring, a naphthalene ring, or a phenanthrene ring. When $Y^1$ or $Y^2$ is a benzene ring, a or b corresponding to $Y^1$ or $Y^2$ is an integer of 0 to 4. When $Y^1$ or $Y^2$ is a naphthalene ring, a or b corresponding to $Y^1$ or $Y^2$ is an integer of 0 to 6. When $Y^1$ or $Y^2$ is a phenanthrene ring, a or b corresponding to $Y^1$ or $Y^2$ is an integer from 0 to 8.
$R^1$ and $R^2$ each independently represent an aromatic ring group optionally having a substituent. When a and b are 2 or more, a plurality of $R^1$s and $R^2$s may be the same or different from each other.
In the formula, $Y^1$ and $Y^2$ are not both benzene rings and a and b are not both 0.]

2. The compound according to claim 1, wherein p is 0 or 1.

3. The compound according to claim 1 or 2, wherein A is an oxiranyl group, a vinyl group, an isopropenyl group, an allyl group, or a (meth)acryloyl group.

4. The compound according to claim 3, wherein A is a (meth)acryloyl group.

5. The compound according to claim 1 or 2, wherein Z is a nitrogen atom optionally having a substituent.

6. The compound according to claim 1 or 2, wherein $R^1$ and/or $R^2$ are a condensed aromatic ring group or a monocyclic aromatic group substituted with an aromatic ring group.

7. The compound according to claim 1 or 2, wherein $Y^1$ and/or $Y^2$ are a benzene ring or a naphthalene ring.

**8.** The compound according to claim 7, wherein $Y^1$ and $Y^2$ are benzene rings.

**9.** A polymerizable composition comprising: the compound according to claim 1 or 2; and a polymerization initiator.

**10.** A holographic recording medium comprising the polymerizable composition according to claim 9.

**11.** A polymer obtained by polymerizing the polymerizable composition according to claim 9.

**12.** An optical material comprising the polymer according to claim 11.

**13.** An optical component comprising the polymer according to claim 11.

**14.** A large-capacity memory comprising the holographic recording medium according to claim 10.

**15.** An optical element obtained by recording a hologram in the holographic recording medium according to claim 10.

**16.** An AR glass comprising the optical element according to claim 15.

Fig.1

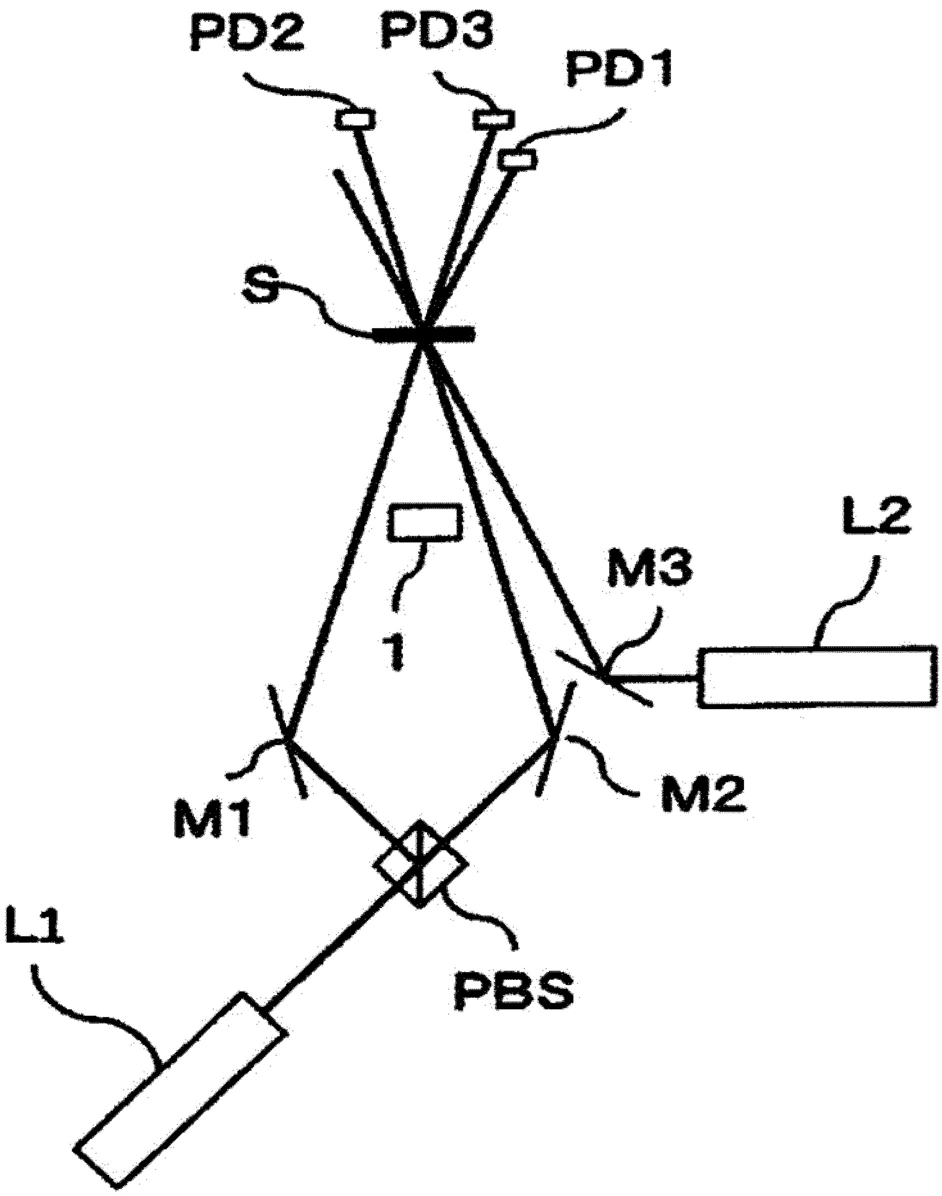

# INTERNATIONAL SEARCH REPORT

International application No.
**PCT/JP2023/024169**

**A. CLASSIFICATION OF SUBJECT MATTER**

***C07D 209/80***(2006.01)i; ***C07D 209/86***(2006.01)i; ***C07D 405/14***(2006.01)i; ***C07D 409/14***(2006.01)i; ***C08F 20/36***(2006.01)i; ***G02B 1/04***(2006.01)i; ***G02B 1/11***(2015.01)i; ***G02B 5/32***(2006.01)i; ***G03H 1/02***(2006.01)i; ***G11B 7/0065***(2006.01)i
FI: C07D209/80; C07D209/86; C07D405/14; C07D409/14 CSP; C08F20/36; G02B1/04; G02B1/11; G02B5/32; G03H1/02; G11B7/0065

According to International Patent Classification (IPC) or to both national classification and IPC

**B. FIELDS SEARCHED**

Minimum documentation searched (classification system followed by classification symbols)

C07D209/80; C07D209/86; C07D405/14; C07D409/14; C08F20/36; G02B1/04; G02B1/11; G02B5/32; G03H1/02; G11B7/0065

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2016-522904 A (DIC CORPORATION) 04 August 2016 (2016-08-04)<br>claims, paragraphs [0026]-[0029], [0077], example 1, tables 1-3 | 1-3, 5-16 |
| X | WO 2012/147809 A1 (SUMITOMO CHEMICAL COMPANY, LIMITED) 01 November 2012 (2012-11-01)<br>synthesis example 2, paragraph [0094] | 1-3, 5-16 |
| X | WO 2018/070079 A1 (NIPPON SODA COMPANY, LIMITED) 19 April 2018 (2018-04-19)<br>examples 8, 14, paragraph [0001] | 1-3, 5-7, 9-16 |
| X | US 3307940 A (AZOPLATE CORPORATION) 07 March 1967 (1967-03-07)<br>table I, no. 12, 14, 15, 28, formulas 12, 14, 15, 28, examples 7, 8 | 1-3, 5-7, 9-16 |
| X | OKAMOTO, Y. et al. Asymmetric Polymerization of N,N-Disubstituted Acrylamides. Polymer Journal. 1989, 21(7), pp. 543-549, DOI:10.1295/polymj.21.543<br>pp. 543, 544, 546, monomers P1-NAA, P-2-NZZ, tables I, VI, experimental | 1-3, 5-7, 9-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 September 2023** | **19 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**

International application No. **PCT/JP2023/024169**

**C. DOCUMENTS CONSIDERED TO BE RELEVANT**

| Category* | Citation of document, with indication, where appropriate, of the relevant passages | Relevant to claim No. |
|---|---|---|
| X | Database Registry [online]. retrieved from STN. entered STN: 07 June 2019, [retrieved on 05 September 2023], CAS registration no. 2325882-15-1<br>CAS registration no. 2325882-15-1 | 1-3, 5-7 |
| X | Database Registry [online]. retrieved from STN. entered STN: 06 June 2019, [retrieved on 05 September 2023], CAS registration no. 2325575-57-1<br>CAS registration no. 2325575-57-1 | 1-3, 5-7 |
| X | Database Registry [online]. retrieved from STN. entered STN: 20 November 2003, [retrieved on 05 September 2023], CAS registration no. 618879-24-6<br>CAS registration no. 618879-24-6 | 1-3, 5-7 |
| X | WO 2020/008957 A1 (FUJIFILM CORPORATION) 09 January 2020 (2020-01-09)<br>claims, paragraphs [0005], [0063], [0121]-[0131], examples | 1-16 |
| X | WO 2021/006011 A1 (SONY CORPORATION) 14 January 2021 (2021-01-14)<br>claims, examples, general formula (1), definition of R1 | 1-3, 5-16 |
| A | JP 2010-043079 A (BAYER MATERIALSCIENCE AKTIENGESELLSCHAFT) 25 February 2010 (2010-02-25)<br>entire text | 1-16 |
| A | VILKAUSKAITE, G. et al. Use of tosylated glycerol carbonate to access N-glycerylated aza-aromatic species. Tetrahedron. 2013, 69(18), pp. 3721-3727<br>table 1, Glycidyl carbamate 12b | 1-16 |

Form PCT/ISA/210 (second sheet) (January 2015)

**INTERNATIONAL SEARCH REPORT**
**Information on patent family members**

International application No.
**PCT/JP2023/024169**

| Patent document cited in search report | Publication date (day/month/year) | Patent family member(s) | Publication date (day/month/year) |
|---|---|---|---|
| JP 2016-522904 A | 04 August 2016 | US 2017/0073310 A1<br>claims, paragraphs [0027]-[0031], [0079], example 1, tables 1-3<br>WO 2015/165089 A1<br>EP 3137930 A1<br>TW 201540704 A<br>KR 10-2016-0143702 A<br>CN 106471398 A | |
| WO 2012/147809 A1 | 01 November 2012 | JP 2013-64101 A | |
| WO 2018/070079 A1 | 19 April 2018 | JP 2021-91719 A<br>US 2021/0284879 A1<br>examples 8, 14, paragraph [0001]<br>EP 3527642 A1<br>EP 4155361 A1<br>KR 10-2019-0041507 A<br>CN 109804033 A<br>KR 10-2021-0097841 A<br>TW 201814021 A<br>CN 113956175 A | |
| US 3307940 A | 07 March 1967 | GB 946107 A<br>DE 1131988 B<br>FR 1258127 A<br>CH 406849 A<br>AU 249377 A<br>LU 38672 A<br>NL 131776 C<br>NL 252125 A<br>DK 104038 C<br>AU 6091760 A | |
| WO 2020/008957 A1 | 09 January 2020 | KR 10-2021-0005669 A<br>TW 202005993 A | |
| WO 2021/006011 A1 | 14 January 2021 | US 2022/0289678 A1<br>claims, examples, general formula (1), definition of R1<br>EP 3995491 A1<br>TW 202110797 A<br>CN 114174262 A<br>KR 10-2022-0029590 A | |
| JP 2010-043079 A | 25 February 2010 | US 2010/0036013 A1<br>entire text<br>EP 2154128 A1<br>EP 2154129 A1<br>CA 2674826 A<br>SG 159455 A<br>KR 10-2010-0019388 A<br>BR PI0902816 A<br>IL 200025 A<br>CN 101704773 A<br>AT 493383 T | |

Form PCT/ISA/210 (patent family annex) (January 2015)

**INTERNATIONAL SEARCH REPORT**
**Information on patent family members**

International application No.
**PCT/JP2023/024169**

| Patent document cited in search report | Publication date (day/month/year) | Patent family member(s) | Publication date (day/month/year) |
|---|---|---|---|
| | | RU 2009130286 A | |
| | | CA 2674826 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008527413 A
- JP 2005133071 A
- WO 2021006011 A1
- WO 2021006012 A1
- JP 5230189 A
- JP 2000204284 A
- JP 2000119306 A
- JP 2001220526 A
- JP 2001310937 A
- JP 3737306 B
- JP 59152396 A
- JP 61151197 A
- JP H5241338 A
- JP H269 A
- JP H255446 B
- JP 2000010277 A
- JP 2004198446 A
- JP 6069294 B
- JP 2022106252 A


### Non-patent literature cited in the description


- **H. KOGELNIK**. *The Bell System Technical Journal*, 1969, vol. 48, 2909-2947